(19) Europäisches Patentamt
European Patent Office
Office européen des brevets

(11) **EP 3 728 581 B1**

(12) **EUROPEAN PATENT SPECIFICATION**

(45) Date of publication and mention
of the grant of the patent:
**09.10.2024 Bulletin 2024/41**

(21) Application number: **18890272.0**

(22) Date of filing: **20.12.2018**

(51) International Patent Classification (IPC):
*C12N 15/00* (2006.01)   *C12N 15/09* (2006.01)
*C12N 15/63* (2006.01)   *C12N 15/66* (2006.01)
*C12N 15/73* (2006.01)   *C12N 15/90* (2006.01)

(52) Cooperative Patent Classification (CPC):
**C12N 15/63; C12N 9/22; C12N 15/1079;
C12N 15/90**

(86) International application number:
**PCT/US2018/066908**

(87) International publication number:
**WO 2019/126562 (27.06.2019 Gazette 2019/26)**

(54) **CONTROLLING PHENOTYPE OF ORGANISMS WITH CRISPR/CAS GENE TARGETING**

STEUERUNG DES PHÄNOTYPS VON ORGANISMEN MIT CRISPR/CAS-GEN-TARGETING

CONTRÔLE DU PHÉNOTYPE D'ORGANISMES AVEC CIBLAGE DE GÈNE PAR CRISPR/CAS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.12.2017   US 201762610040 P**

(43) Date of publication of application:
**28.10.2020 Bulletin 2020/44**

(73) Proprietor: **Bio-Rad Laboratories, Inc.**
**Hercules, CA 94547 (US)**

(72) Inventors:
• **WARD, Alex, Michael**
**Hercules, CA 94547 (US)**
• **LEUNG, Molly, Mo-Yin**
**Hercules, CA 94547 (US)**
• **SPAGNOLO-SMITH, Jean, Frances**
**Hercules, CA 94547 (US)**
• **CHENAUX, George**
**Hercules, CA 94547 (US)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
WO-A1-2015/017866    WO-A1-2016/064999
WO-A1-2018/020248    WO-A2-2016/011080

• PYNE ET AL.: "Coupling the CRISPR/Cas9
System with Lambda Red Recombineering
Enables Simplified Chromosomal Gene
Replacement in Escherichia coli", APPL
ENVIRON MICROBIOL, vol. 81, no. 15, 22 May
2015 (2015-05-22), pages 5103 - 5114,
XP055336375, doi:10.1128/AEM.01248-15
• YAN ET AL.: "CRISPR-Cas12a-Assisted
Recombineering in Bacteria", APPL ENVIRON
MICROBIOL, vol. 83, no. 17, 1 September 2017
(2017-09-01), pages 1 - 13, XP055524927
• GLASER ET AL.: "GFP to BFP Conversion: A
Versatile Assay for the Quantification of
CRISPR/Cas9-mediated Genome Editing", MOL
THER NUCLEIC ACIDS, vol. 5, no. 7, 12 July 2016
(2016-07-12), pages 1 - 4, XP055515717
• CERTO ET AL.: "Tracking genome engineering
outcome at individual DNA breakpoints", NAT
METHODS, vol. 8, no. 8, 10 July 2011 (2011-07-10),
pages 671 - 676, XP002770350

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Genomic editing technologies can be used to introduce modifications in nucleic acid sequences in a sequence-specific manner. One such technology utilizes clustered regularly interspaced short palindromic repeats (CRISPR), which are segments of prokaryotic DNA that contain short repetitive nucleotide sequences, and CRISPR-associated (Cas) nucleases that induce a double-stranded break (DSB) in nucleic acid. The Cas nuclease can specifically induce a DSB at a target genomic locus through the use of a guide nucleic acid that targets the genomic locus. Following the induction of a DSB in the nucleic acid, repair of the DSB can occur via non-homologous end joining (NHEJ), alternative-end joining (A-EJ), and/or homology-directed repair (HDR). The CRISPR/Cas system can be used to introduce modifications that disrupt expression of a nucleic acid and/or to repair an existing mutation in a nucleic acid sequence in order to restore expression of the nucleic acid.

BRIEF SUMMARY OF THE INVENTION

The present invention provides engineered microbial organism cells and engineered microbial organisms, kits, methods and educational systems comprising such engineered microbial organism cells and engineered microbial organisms as recited in the attached independent claims 1, 15, 16, 18 and 19. In a first aspect of the invention, provided is an engineered microbial organism cell comprising:

(a) an endogenous gene or genomic region to be targeted that encodes a functional protein having a first detectable phenotype, and a heterologous polynucleotide sequence encoding a phenotype coding sequence operably linked to a promoter that (i) encodes a second functional protein having a second detectable phenotype, or (ii) comprises an engineered disruption in a sequence encoding the second protein that prevents expression of the second detectable phenotype for gene alteration, or

one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype; and
a second phenotype coding sequence operably linked to a promoter, wherein the second phenotype coding sequence either (i) encodes a second functional protein having a second detectable phenotype, or (ii) comprises an engineered disruption in a sequence encoding the second protein that prevents expression of the second detectable phenotype;
wherein each of the first and the second detectable phenotypes is a detectable color, fluorescence, scent, enzymatic activity, antibiotic resistance, morphology or lethality;

(b) a heterologous polynucleotide sequence comprising a promoter operably linked to a polynucleotide encoding a Cas nuclease and a heterologous polynucleotide sequence comprising a guide RNA (gRNA) that targets the endogenous gene, genomic region, or phenotype coding sequence, and a heterologous polynucleotide sequence comprising a promoter operably linked to a polynucleotide encoding lambda red.

In some embodiments, the engineered microbial organism cell further comprises a donor DNA sequence.
In some embodiments, an engineered microbial organism cell comprises an endogenous gene or genomic region to be targeted for gene alteration. In some embodiments, the endogenous gene or genomic region to be targeted for gene alteration is a functional gene or genomic region (e.g., produces a detectable phenotype) and the donor DNA sequence is a sequence that disrupts the functional gene or genomic region. In some embodiments, the endogenous gene or genomic region to be targeted for gene alteration is a functional gene or genomic region (e.g., produces a detectable phenotype) and the donor DNA sequence is a sequence that replaces the functional gene or genomic region with a different functional gene or different functional genomic region. In some embodiments, the endogenous gene or genomic region to be targeted is a disrupted gene or genomic region (e.g., a disruption that prevents expression of a detectable phenotype) and wherein the donor DNA sequence is a sequence that restores the function of the gene or genomic region.
In some embodiments, an engineered microbial organism cell comprises one or more heterologous polynucleotide sequences comprising a phenotype coding sequence operably linked to a promoter. In some embodiments, an engineered microbial organism cell comprises:
one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype.

In some embodiments, the engineered microbial organism cell comprises a phenotype coding sequence that encodes a functional protein having a detectable phenotype (e.g., a chromogenic coding sequence that encodes a functional chromogenic protein). In some embodiments, the engineered microbial organism cell comprises a phenotype coding sequence that comprises an engineered disruption in a sequence encoding a protein or promoter region that prevents expression of the detectable phenotype (e.g., a broken chromogenic gene that prevents expression of a chromogenic protein).

In some embodiments, an engineered microbial organism cell comprises:

one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype;

a heterologous polynucleotide sequence comprising a promoter operably linked to a polynucleotide encoding a Cas nuclease;

a heterologous polynucleotide sequence comprising a guide RNA (gRNA) that targets the phenotype coding sequence.

In some embodiments, the engineered microbial organism cell comprises:

a heterologous polynucleotide sequence comprising a phenotype coding sequence that either (i) encodes a functional protein having a detectable phenotype, or (ii) comprises an engineered disruption in a sequence encoding a protein that prevents expression of the detectable phenotype;

a heterologous polynucleotide sequence comprising a promoter operably linked to a polynucleotide encoding a Cas nuclease;

a heterologous polynucleotide sequence comprising a gRNA that targets the chromogenic coding sequence; and

a heterologous polynucleotide sequence comprising a homologous donor DNA sequence.

In some embodiments, the phenotype coding sequence encodes a functional protein having a detectable phenotype (e.g., a functional chromogenic or fluorescent protein, e.g., coral fluorescent protein). In some embodiments, the phenotype coding sequence comprises an engineered disruption in a sequence encoding a protein or promoter region that prevents expression of the detectable phenotype (e.g., a broken gene such as a broken chromogenic gene or broken fluorescent protein, e.g., a coral fluorescent protein). In some embodiments, the cell comprises a heterologous polynucleotide sequence comprising a homologous donor DNA sequence (e.g., for fixing a broken gene).

In some embodiments, an engineered microbial organism cell comprises:

a first heterologous polynucleotide sequence comprising a first phenotype coding sequence operably linked to a first promoter, wherein the first phenotype coding sequence either (i) encodes a functional first protein having a first detectable phenotype, or (ii) comprises an engineered disruption in a sequence encoding a first protein that prevents expression of the first detectable phenotype;

a second heterologous polynucleotide sequence comprising a second phenotype coding sequence operably linked to a second promoter, wherein the second phenotype coding sequence either (i) encodes a functional second protein having a second detectable phenotype, or (ii) comprises an engineered disruption in a sequence encoding a second protein that prevents expression of the second detectable phenotype;

a third heterologous polynucleotide sequence comprising a third promoter operably linked to a polynucleotide encoding a Cas nuclease (e.g., Cas9);

a fourth heterologous polynucleotide sequence comprising a first gRNA that targets the first phenotype coding sequence; and

a fifth heterologous polynucleotide sequence comprising a second gRNA that targets the second phenotype coding sequence.

In some embodiments, the detectable phenotype (e.g., each of the first detectable phenotype and the second detectable phenotype) is a detectable color, fluorescence, scent, enzymatic activity, or morphology. In some embodiments, the detectable phenotype (e.g., each of the first detectable phenotype and the second detectable phenotype) is lethality. In some embodiments, the detectable phenotype (e.g., each of the first detectable phenotype and the second detectable phenotype) is gain or loss of resistance to an antibiotic.

In some embodiments, the detectable phenotype is a detectable fluorescence, and wherein the phenotype coding sequence either (i) encodes a functional fluorescent protein, or (ii) comprises an engineered disruption in a sequence encoding a protein that prevents expression of the fluorescent protein. In some embodiments, the fluorescent protein is a coral fluorescent protein.

In some embodiments, the first detectable phenotype and the second detectable phenotype is a detectable color and/or fluorescence, and:

the first chromogenic coding sequence either (i) encodes a functional first chromogenic protein or (ii) comprises an engineered disruption in a sequence encoding a first chromogenic protein that prevents expression of the first chromogenic protein; and
the second chromogenic coding sequence either (i) encodes a functional second chromogenic protein or (ii) comprises an engineered disruption in a sequence encoding a second chromogenic protein that prevents expression of the second chromogenic protein.

In some embodiments, one or more polynucleotide sequences as disclosed herein (e.g., a polynucleotide sequence comprising a phenotype coding sequence, a polynucleotide sequence comprising a promoter operably linked to a polynucleotide encoding a Cas nuclease, or a polynucleotide sequence comprising a gRNA that targets the phenotype coding sequence) further comprises an auxotrophic, antibiotic, or other selectable marker. In some embodiments, one or more of the first polynucleotide sequence, second polynucleotide sequence, third polynucleotide sequence, fourth polynucleotide sequence, or fifth polynucleotide sequence further comprises an auxotrophic, antibiotic, or other selectable marker. In some embodiments, wherein a fourth heterologous polynucleotide sequence comprises a first gRNA that targets the first phenotype coding sequence and a fifth polynucleotide sequence comprises a second gRNA that targets the second phenotype coding sequence, the fourth polynucleotide sequence further comprises a first auxotrophic, antibiotic, or other selectable marker and the fifth polynucleotide sequence further comprises a second auxotrophic, antibiotic, or other selectable marker, wherein the first auxotrophic, antibiotic, or other selectable marker and the second auxotrophic, antibiotic, or other selectable marker are different markers. In some embodiments, the first auxotrophic, antibiotic, or other selectable marker and the second auxotrophic, antibiotic, or other selectable marker are the same marker.

In some embodiments, the microbial organism cell comprises a heterologous polynucleotide comprising a homologous donor polynucleotide sequence that is used as a template for repair of the Cas cleavage site. In some embodiments, the homologous donor polynucleotide sequence is double-stranded DNA (dsDNA) or single-stranded DNA (ssDNA). In some embodiments, the heterologous polynucleotide sequence comprising the homologous donor DNA sequence comprises a mutation that prevents expression of the chromogenic protein. In some embodiments, the heterologous polynucleotide sequence comprising the homologous donor DNA sequence comprises a mutation that modifies expression of the chromogenic protein from a first color to a second color. In some embodiments, the heterologous polynucleotide sequence comprising the homologous donor DNA sequence comprises a mutation that repairs a polynucleotide sequence comprising an engineered disruption (e.g., a broken gene) and restores expression of a functional chromogenic protein. In some embodiments, one or more polynucleotide sequences as disclosed herein (e.g., a polynucleotide sequence comprising a phenotype coding sequence, a polynucleotide sequence comprising a promoter operably linked to a polynucleotide encoding a Cas nuclease, or a polynucleotide sequence comprising a gRNA that targets the phenotype coding sequence) further comprises a homologous donor polynucleotide sequence, e.g., a double-stranded DNA (dsDNA) or single-stranded DNA (ssDNA) donor sequence. In some embodiments, the fourth polynucleotide sequence and/or the fifth polynucleotide sequence further comprises a homologous donor polynucleotide sequence. In some embodiments, wherein a fourth heterologous polynucleotide sequence comprises a first gRNA that targets the first phenotype coding sequence and a fifth polynucleotide sequence comprises a second gRNA that targets the second phenotype coding sequence, the fourth polynucleotide sequence and/or the fifth polynucleotide sequence further comprises a homologous donor polynucleotide sequence.

In some embodiments, a polynucleotide sequence comprising a gRNA that targets an endogenous gene, genomic region, or phenotypic coding sequence (e.g., a chromogenic coding sequence) is operably linked to the polynucleotide sequence comprising the promoter operably linked to the polynucleotide encoding a Cas nuclease. In some embodiments, a polynucleotide sequence comprising a gRNA that targets an endogenous gene, genomic region, or phenotypic coding sequence (e.g., a chromogenic coding sequence) and a polynucleotide sequence comprising a homologous donor DNA sequence are operably linked to the polynucleotide sequence comprising the promoter operably linked to the polynucleotide encoding a Cas nuclease.

In some embodiments, a promoter is a constitutively active promoter. In some embodiments, one or more of the first promoter, second promoter, and third promoter are constitutively active promoters. In some embodiments, both the first promoter and the second promoter are constitutively active promoters.

In some embodiments, a promoter is an inducible promoter. In some embodiments, one or more of the first promoter, second promoter, and third promoter are inducible promoters. In some embodiments, both the first promoter and the second promoter are inducible promoters. In some embodiments, the inducible promoter is a galactose-inducible promoter. In some embodiments, the inducible promoter is an arabinose-inducible promoter. In some embodiments, the inducible promoter is a rhamnose-inducible promoter.

In some embodiments, the Cas nuclease is a Cas9 nuclease. In some embodiments, the Cas nuclease is a Cas9 nuclease from *Streptococcus pyogenes* (SpCas9 nuclease). In some embodiments, the polynucleotide encoding the Cas nuclease is optimized for expression in the microbial organism cell (e.g., eukaryotic or prokaryotic cell). In some embodiments, the Cas nuclease is a Cas9 nuclease that is codon optimized for expression in a yeast cell. In some embodiments, the Cas nuclease is a Cas9 nuclease that is codon optimized for expression in an *E. coli* cell. In some embodiments, the Cas nuclease is a destabilized variant of Cas9.

In some embodiments, the microbial organism cell comprises a heterologous polynucleotide sequence comprising a chromogenic coding sequence that encodes a functional chromogenic protein (e.g., a GFP protein), and expression of the Cas nuclease and repair of the Cas cleavage results in the heterologous polynucleotide sequence having a mutation that prevents the expression of the chromogenic protein (e.g., prevents GFP expression). In some embodiments, the microbial organism cell comprises a heterologous polynucleotide sequence comprising a chromogenic coding sequence that encodes a functional chromogenic protein (e.g., a GFP protein), and expression of the Cas nuclease and repair of the Cas cleavage results in the heterologous polynucleotide sequence having one or more mutations that result in the expression of a second chromogenic protein (e.g., a YFP or BFP instead of GFP).

In some embodiments, the first chromogenic coding sequence encodes a functional first chromogenic protein and/or the second chromogenic coding sequence encodes a functional second chromogenic protein. In some embodiments, the first coding sequence comprises an engineered disruption in a sequence encoding a first chromogenic protein that prevents expression of the first chromogenic protein or its promoter, and/or the second coding sequence comprises an engineered disruption in a sequence encoding a second chromogenic protein that prevents expression of the second chromogenic protein or its promoter. In some embodiments, the first chromogenic coding sequence encodes a functional first chromogenic protein and the second coding sequence comprises an engineered disruption in a sequence encoding a second chromogenic protein that prevents expression of the second chromogenic protein or its promoter. In some embodiments, the first coding sequence comprises an engineered disruption in a sequence encoding a first chromogenic protein that prevents expression of the first chromogenic protein or its promoter, and the second chromogenic coding sequence encodes a functional second chromogenic protein.

In some embodiments, one or more of the polynucleotide sequences are in the same expression cassette or plasmid. In some embodiments, at least two polynucleotide sequences are in separate expression cassettes or plasmids. In some embodiments, the first polynucleotide sequence and the second polynucleotide are in the same expression cassette or plasmid. In some embodiments, the first polynucleotide sequence and the second polynucleotide are in separate expression cassettes or plasmids. In some embodiments, two or more (e.g., two, three, four, or five) of each the first polynucleotide sequence, the second polynucleotide sequence, the third polynucleotide sequence, the fourth polynucleotide sequence, and the fifth polynucleotide sequence are in the same expression cassette or plasmid. In some embodiments, each of the first polynucleotide sequence, the second polynucleotide sequence, the third polynucleotide sequence, the fourth polynucleotide sequence, and the fifth polynucleotide sequence is in a separate expression cassette or plasmid.

In some embodiments, the microbial organism cell is a prokaryotic cell. In some embodiments, the microbial organism cell is a prokaryotic cell selected from the group consisting of bacteria and archaebacteria. In some embodiments, the microbial organism cell is a protist cell. In some embodiments, the microbial organism cell is a eukaryotic cell. In some embodiments, the eukaryotic cell is a yeast cell. In some embodiments, the microbial organism cell is a bacterial cell, e.g., an *E coli* cell.

In some embodiments, the third polynucleotide sequence comprises a polynucleotide encoding a Cas9 nuclease that is from *S. pyogenes* or that is codon optimized for expression in the particular cell or organism in which it is expressed. In some embodiments, the cell is a yeast cell and the third polynucleotide sequence comprises a polynucleotide encoding a Cas9 nuclease that is codon optimized for expression in yeast.

In a second aspect of the invention, provided is an engineered microbial organisms comprising an engineered microbial organism cell of the first aspect of the invention as described herein. In some embodiments, the engineered organism is eukaryotic (e.g., an engineered yeast). In some embodiments, the engineered organism is prokaryotic (e.g., an engineered bacterium). In some embodiments, the engineered organism is in lyophilized form.

In a third aspect of the invention, provided are kits .comprising an engineered microbial organism cell of the first aspect of the invention as described herein or an engineered microbial organism of the second aspect of the invention as described herein; and further comprises one or more reagents comprising culture medium, selective medium, media supplements, solid plating medium, plates, tubes, loops, or other plasticware. In some embodiments, the kit further comprises one or more heterologous polynucleotide sequences comprising a homologous donor DNA (e.g., double stranded DNA or single stranded DNA). In some embodiments, the kit further comprises an inducer for inducing expression of an inducible promoter (e.g., galactose for a galactose-inducible promoter, arabinose for an arabinose-inducible promoter, or rhamnose for a rhamnose-inducible promoter). In some embodiments, the kit further comprises one or more reagents for detecting the genotype of the engineered microbial organism cell or engineered microbial organism, wherein the one or more reagents comprise DNA polymerases, primers, dNTPs, restriction enzymes, or buffers. In some embodiments, the kit further comprises one or more counterselection agents or selection agents for an auxotrophic, antibiotic, or other selectable marker as described herein.

In some embodiments, the kit comprises an engineered microbial organism cell or organism comprising one or more heterologous polynucleotide sequences comprising a phenotype coding sequence that may be operably linked to a promoter, wherein the phenotype coding sequence either (i) encodes a functional protein having a detectable phenotype, or (ii) comprises an engineered disruption in a sequence encoding a protein that prevents expression of the detectable phenotype; and further comprises one or more of a heterologous polynucleotide sequence comprising a promoter operably linked to a polynucleotide encoding a Cas nuclease, a heterologous polynucleotide sequence comprising a gRNA that targets the phenotype coding sequence, and a heterologous polynucleotide sequence comprising a homologous donor DNA sequence.

In a fourth aspect of the invention, provided are methods of altering gene expression in a cell, the method comprising:

culturing an engineered microbial organism cell of the first aspect of the invention as described herein or an engineered microbial organism of the second aspect of the invention as described herein to form a population of engineered microbial organism cells or engineered microbial organisms, wherein the culturing is performed under conditions that result in expression of the Cas nuclease in at least one engineered microbial organism cell, wherein the Cas nuclease cleaves an endogenous gene, genomic region, or phenotype coding sequence in the engineered microbial organism cell, thereby altering gene expression in at least one engineered microbial organism cell; or

(ii) the lambda red in at least one engineered microbial organism cell, wherein the lambda red catalyzes the homologous recombination of a donor DNA sequence at an endogenous gene, genomic region, or phenotype coding sequence in the engineered cell, thereby altering gene expression in at least one engineered cell.

In some embodiments, a promoter is operably linked to a polynucleotide encoding a Cas nuclease is an inducible promoter and the culturing is in the presence of an inducer to induce expression of the Cas nuclease in at least one engineered cell. In some embodiments, the promoter is a galactose-inducible promoter and the inducer is galactose. In some embodiments, the promoter is an arabinose-inducible promoter and the inducer is arabinose. In some embodiments, the promoter is a rhamnose-inducible promoter and the inducer is rhamnose.

In some embodiments, a method of altering gene expression comprises:

culturing an engineered microbial organism cell or engineered microbial organism as described herein to form a population of engineered microbial organism cells, wherein the culturing is performed under conditions that result in expression of the lambda red in at least one engineered microbial organism cell, wherein the lambda red catalyzes the homologous recombination of a donor DNA sequence at an endogenous gene, genomic region, or phenotype coding sequence in the engineered microbial organism cell;
thereby altering gene expression in at least one engineered cell.

In some embodiments, the lambda red is under the control of an inducible promoter, and wherein the culturing is in the presence of an inducer to induce expression of the lambda red in at least one engineered cell. In some embodiments, the promoter is a galactose-inducible promoter and the inducer is galactose. In some embodiments, the promoter is an arabinose-inducible promoter and the inducer is arabinose. In some embodiments, the promoter is a rhamnose-inducible promoter and the inducer is rhamnose.

In some embodiments, the method of altering gene expression comprises expressing both the Cas nuclease and the lambda red system in at least one engineered microbial organism cell. In some embodiments, both the Cas nuclease and the lambda red system are under the control of an inducible promoter. In some embodiments, expression of both the Cas nuclease and the lambda red system are induced by the same inducer. In some embodiments, expression of the Cas nuclease and the lambda red system are induced by different inducers.

In some embodiments, prior to the culturing step, the method comprises transforming or transfecting a cell with one or more heterologous polynucleotide sequences as disclosed herein to produce the engineered microbial organism cell. In some embodiments, the transforming comprises chemical transformation, transformation by electroporation, or transformation by silicon-carbide whiskers.

In some embodiments, the method further comprises screening the population of engineered microbial organism cells to identify at least one engineered microbial organism cell that exhibits a change in phenotype as compared to the phenotype of the engineered microbial organism cell prior to the culturing step.

In some embodiments, the engineered microbial organism cell or microbial organism comprises a heterologous polynucleotide sequence comprising a chromogenic coding sequence that encodes a functional chromogenic protein, a heterologous polynucleotide encoding a Cas nuclease, a heterologous polynucleotide sequence comprising a gRNA that targets the chromogenic coding sequence, and a heterologous polynucleotide sequence comprising a homologous donor DNA sequence that comprises a mutation that prevents expression of the chromogenic protein, and the method further comprises: culturing the population of engineered cells under conditions that result in expression of the gRNA in at least one engineered microbial organism cell; and screening the population of engineered cells to identify at least one engineered microbial organism cell that does not express the chromogenic protein.

In some embodiments, the engineered microbial organism cell or microbial organism comprises a heterologous polynucleotide sequence comprising a chromogenic coding sequence that encodes a functional chromogenic protein, a heterologous polynucleotide encoding a Cas nuclease, a heterologous polynucleotide sequence comprising a gRNA that targets the chromogenic coding sequence, and a heterologous polynucleotide sequence comprising a homologous donor DNA sequence that comprises a mutation that modifies expression of the chromogenic protein from a first color to a second color, and the method further comprises: culturing the population of engineered microbial organism cells under conditions that result in expression of the gRNA in at least one engineered microbial organism cell; and screening the population of engineered microbial organism cells to identify at least one engineered microbial organism cell that expresses the second color.

In some embodiments, the engineered microbial organism cell or microbial organism comprises a heterologous polynucleotide sequence comprising a chromogenic coding sequence that comprises an engineered disruption in a sequence encoding a chromogenic protein that prevents expression of the functional chromogenic protein, a heterologous polynucleotide encoding a Cas nuclease, a heterologous polynucleotide sequence comprising a gRNA that targets the chromogenic coding sequence, and a heterologous polynucleotide sequence comprising a homologous donor DNA sequence for repairing the engineered disruption, and the method further comprises: culturing the population of engineered microbial organism cells under conditions that result in expression of the gRNA in at least one engineered microbial organism cell; and screening the population of engineered microbial organism cells to identify at least one engineered microbial organism cell that expresses the functional chromogenic protein.

In some embodiments, the engineered microbial organism cell or microbial organism comprises a fourth polynucleotide sequence that further comprises a first auxotrophic, antibiotic, or other selectable marker and a fifth polynucleotide sequence that further comprises a second auxotrophic, antibiotic, or other selectable marker, wherein the first auxotrophic, antibiotic, or other selectable marker and the second auxotrophic, antibiotic, or other selectable marker are different markers, and the method further comprises:

    culturing the population of engineered microbial organism cells or engineered microbial organisms in the presence of a counterselection agent or selection agent for the first auxotrophic, antibiotic, or other selectable marker; and
    selecting for engineered microbial organism cells or engineered microbial organisms that do not express the fourth polynucleotide sequence;
    thereby preventing expression of the first gRNA and preventing alteration of the first phenotype coding sequence.

In some embodiments, the engineered microbial organism cell or microbial organism comprises a fourth polynucleotide sequence that further comprises a first auxotrophic, antibiotic, or other selectable marker and a fifth polynucleotide sequence that further comprises a second auxotrophic, antibiotic, or other selectable marker, wherein the first auxotrophic, antibiotic, or other selectable marker and the second auxotrophic, antibiotic, or other selectable marker are different markers, and the method further comprises:

    culturing the population of engineered microbial organism cells or engineered microbial organisms in the presence of a counterselection agent or selection agent for the second auxotrophic, antibiotic, or other selectable marker; and
    selecting for engineered microbial organism cells or engineered microbial organisms that do not express the fifth polynucleotide sequence;
    thereby preventing expression of the first gRNA and preventing alteration of the second phenotype coding sequence.

In some embodiments, (i) the first phenotype coding sequence encodes a functional first protein having a first detectable phenotype and the Cas9 nuclease cleaves the first phenotype coding sequence and disrupts expression of the first protein; and/or (ii) the second phenotype coding sequence encodes a functional second protein having a second detectable phenotype and the Cas9 nuclease cleaves the second phenotype coding sequence and disrupts expression of the second protein.

In some embodiments, (i) the first phenotype sequence comprises an engineered disruption in a sequence encoding a first protein that prevents expression of the first detectable phenotype, and the method comprises cleaving the first coding sequence at the engineered disruption with the Cas9 nuclease and repairing the first coding sequence to permit expression of the first protein; and/or (ii) the second phenotype sequence comprises an engineered disruption in a sequence encoding a second protein that prevents expression of the second detectable phenotype, and the method comprises cleaving the second coding sequence at the engineered disruption with the Cas9 nuclease and repairing the second coding sequence to permit expression of the second protein. In both cases, the engineered changes are defined by the sequence in the homologous repair donor sequence.

In some embodiments, (i) the first phenotype coding sequence encodes a functional first protein having a first detectable phenotype and the Cas9 nuclease cleaves the first phenotype coding sequence and disrupts expression of the first protein; and the second phenotype sequence comprises an engineered disruption in a sequence encoding a second protein that prevents expression of the second detectable phenotype, and the method comprises cleaving the second coding sequence at the engineered disruption with the Cas9 nuclease and repairing the second coding sequence to permit expression of the second protein; or (ii) the first phenotype sequence comprises an engineered disruption in a sequence encoding a first protein that prevents expression of the first detectable phenotype, and the method comprises cleaving the first coding sequence at the engineered disruption with the Cas9 nuclease and repairing the first coding sequence to permit expression of the first protein; and the second phenotype coding sequence encodes a functional second protein having a second detectable phenotype and the Cas9 nuclease cleaves the second phenotype coding sequence and disrupts expression of the second protein. In both cases, the engineered disruption is defined by the sequence in the homologous repair donor sequence.

In some embodiments, the engineered microbial organism cell is a eukaryotic cell, notably a yeast cell,. In some embodiments, the engineered cell is a yeast cell and the cell comprises a polynucleotide sequence that comprises a polynucleotide encoding a Cas9 nuclease that is codon optimized for expression in yeast.

In some embodiments, the engineered microbial organism cell is a prokaryotic cell, e.g., from a bacterium. In some embodiments, the engineered microbial organism cell is a bacterial cell (e.g., *E. coli*) and the cell comprises a polynucleotide sequence that comprises a polynucleotide encoding a Cas9 nuclease that is codon optimized for expression in bacteria. In some embodiments, the microbial organism cell comprises a polynucleotide sequence that comprises a polynucleotide encoding a *S. pyogenes* Cas9 nuclease.

In some embodiments, the engineered microbial organism is a bacterium. In some embodiments, the engineered microbial organism is eukaryotic, e.g., is a yeast.

In a fifth aspect of the invention, provided are modular systems and methods for training an individual in laboratory procedures and the use of laboratory equipment for gene expression and gene editing are provided, the modular system comprising:

(a) a module for expressing a detectable phenotype and/or for expressing a broken gene in a cell or organism, comprising an engineered microbial organism cell of the first aspect of the invention as described herein or an engineered microbial organism of the second aspect of the invention as described herein and further comprising one or more reagents for culturing, transfecting, and/or transforming the cell or organism;
(b) a module for altering expression of the detectable phenotype and/or for repairing the broken gene in the cell or organism, comprising one or more reagents for expressing a Cas nuclease, expressing a gRNA, or preventing expression of a gRNA in the cell or organism;
(c) a module for analyzing the cell or organism, comprising one or more reagents for detecting the alteration of gene expression and/or repair of the broken gene in the cell or organism; and
(d) an instruction manual, comprising instructions for use of each of the modules.

In some embodiments, the instruction manual comprises digital materials, video, electronic media, electronic storage media, and/or optical media.

In some embodiments, the modular system further comprises an assessment module, comprising materials for evaluating a user's performance or skills in utilizing the modules (a), (b), and (c) and the instruction manual (d).

In some embodiments, the modular system comprises:

(a) a module for altering expression of an endogenous gene or genomic region in a cell or organism (e.g., disrupting or restoring the function of an endogenous gene or genomic region in the genome of the cell or organism), comprising an engineered microbial organism cell or engineered microbial organism as disclosed herein, one or more reagents for expressing a Cas nuclease, and one or more reagents for targeting the endogenous gene or genomic region, and further comprising one or more reagents for culturing, transfecting, and/or transforming the cell or organism;
(b) a module for analyzing the cell or organism, comprising one or more reagents for detecting the alteration of gene expression (e.g., the disruption of function of the endogenous gene or genomic region or the restoration of function of the endogenous gene or genomic region); and
(c) an instruction manual, comprising instructions for use of each of the modules.

In some embodiments, the modular system further comprises an assessment module, comprising materials for evaluating a user's performance or skills in utilizing the modules (a) and (b) and the instruction manual (c).

BRIEF DESCRIPTION OF THE DRAWINGS

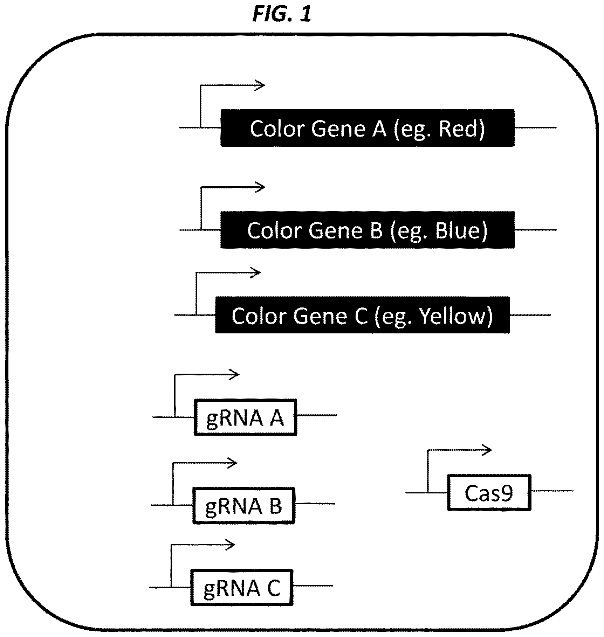

FIG. 1. Schematic depicting an organism containing multiple color genes (A, B, and C) and the genes necessary for targeted knock-out of each of the color genes (gRNA A, B, and C, targeting color genes A, B, and C, respectively, and Cas9 nuclease).

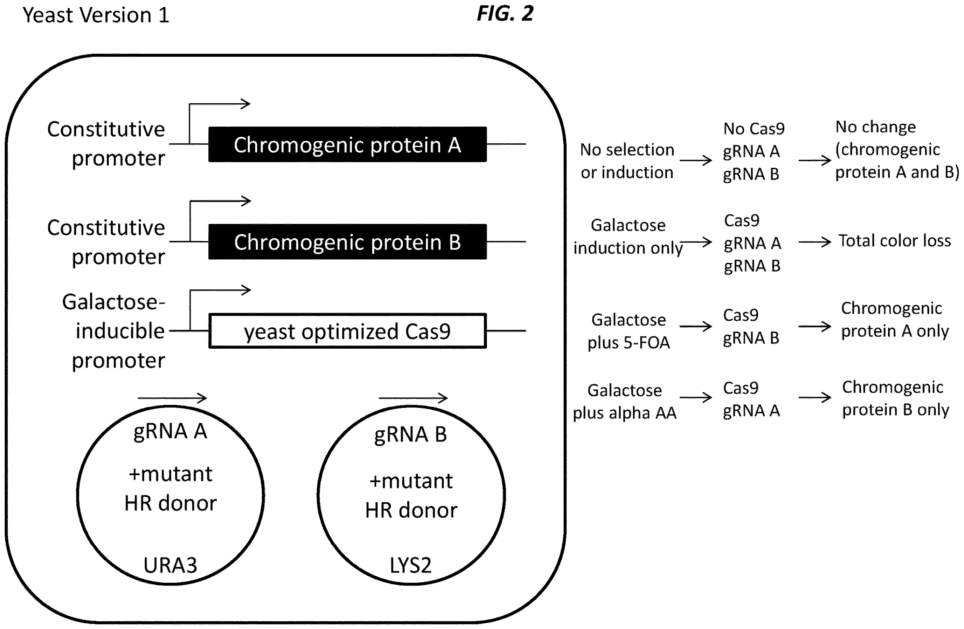

FIG. 2. Yeast Version 1. The yeast strain contains a gene encoding chromogenic protein A under the control of a constitutive promoter, a gene encoding chromogenic protein B under the control of a constitutive promoter, a yeast optimized gene encoding Cas9 under the control of a galactose-inducible promoter, a plasmid comprising gRNA A for targeting chromogenic protein A and the auxotrophic marker URA3, and a plasmid comprising gRNA B for targeting chromogenic protein B and the auxotrophic marker LYS2.

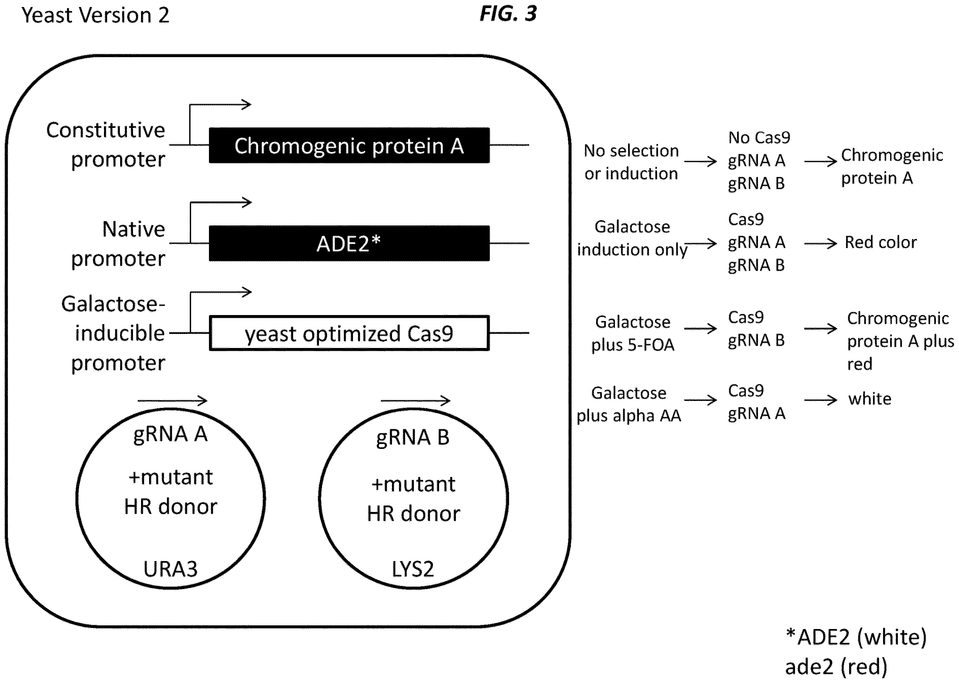

FIG. 3. Yeast Version 2. The yeast strain contains a gene encoding chromogenic protein A under the control of a constitutive promoter, a gene encoding the ADE2 gene under the control of a native promoter, a yeast optimized gene encoding Cas9 under the control of a galactose-inducible promoter, a plasmid comprising gRNA A for targeting chromogenic protein A and the auxotrophic marker URA3, and a plasmid comprising gRNA B for targeting ADE2 and the auxotrophic marker LYS2.

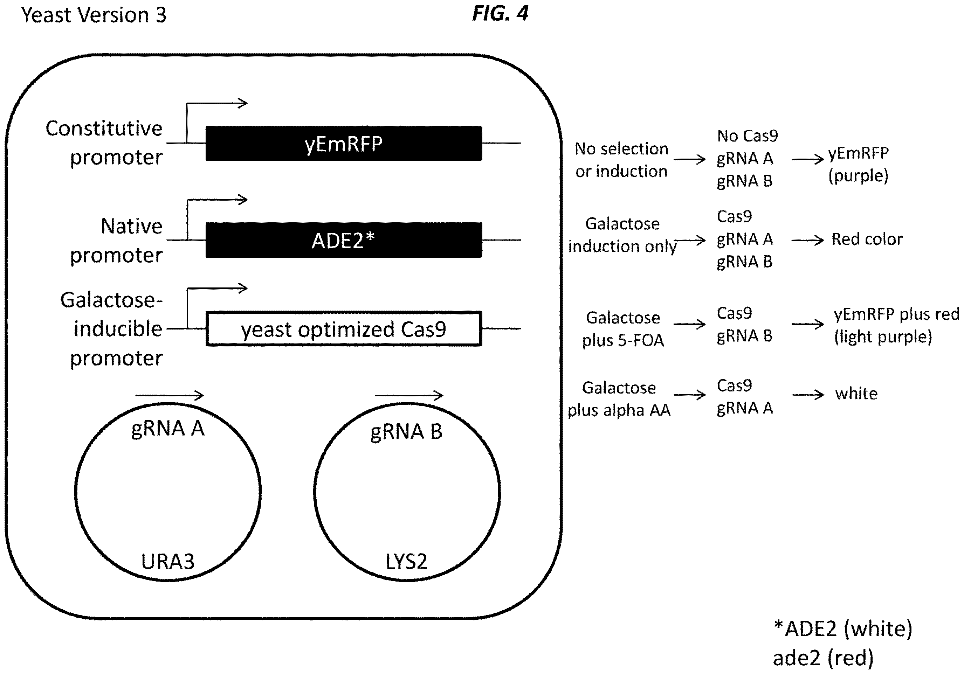

FIG. 4. Yeast Version 3. The yeast strain contains a gene encoding chromogenic protein yEmRFP under the control of a constitutive promoter, a gene encoding the ADE2 gene under the control of a native promoter, a yeast optimized gene encoding Cas9 under the control of a galactose-inducible promoter, a plasmid comprising gRNA A for targeting yEmRFP and the auxotrophic marker URA3, and a plasmid comprising gRNA B for targeting ADE2 and the auxotrophic marker LYS2.

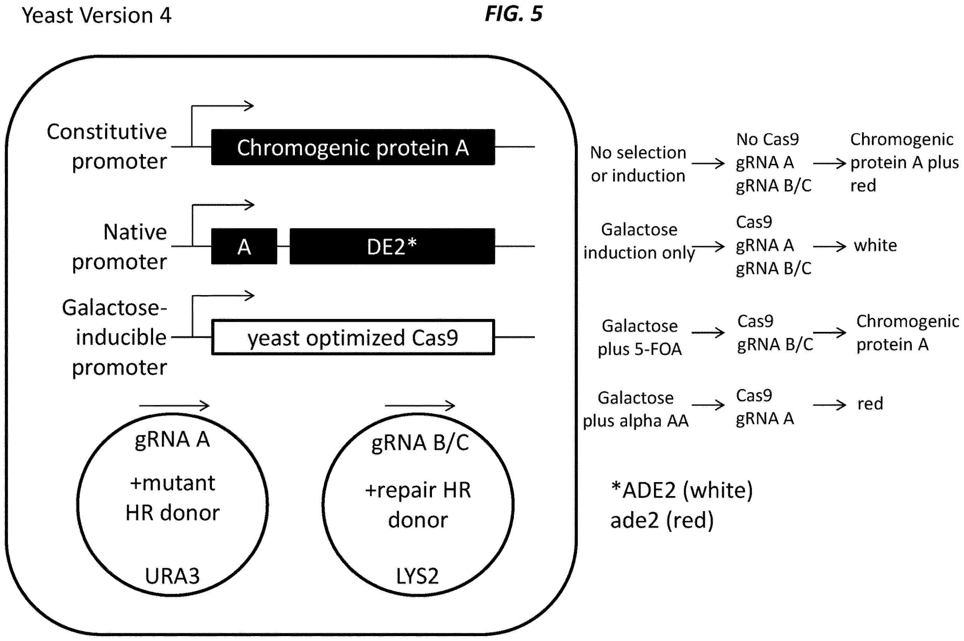

FIG. 5. Yeast Version 4. The yeast strain contains a gene encoding chromogenic protein yEmRFP under the control of a constitutive promoter, a gene encoding an ADE2 gene having an engineered disruption that causes a frameshift and prevents expression of ADE2 and under the control of a native promoter, a yeast optimized gene encoding Cas9 under the control of a galactose-inducible promoter, a plasmid comprising gRNA A for targeting yEmRFP and the auxotrophic marker URA3, and a plasmid comprising gRNA B for targeting the ADE2 having an engineered disruption and the auxotrophic marker LYS2.

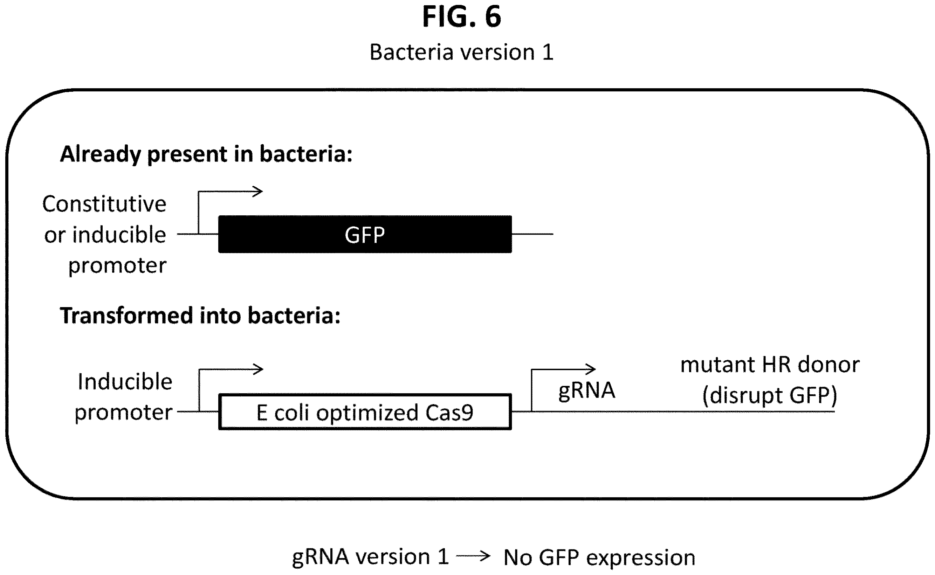

FIG. 6. Bacteria Version 1. The bacteria express a chromogenic or fluorescent protein (GFP) under the control of a constitutive or inducible promoter. An episomal plasmid that expresses a gRNA to GFP and expresses Cas9 under the control of an inducible promoter can be transformed into the bacteria. Note that if both GFP and Cas9 expression rely on inducible promoters, they may not use the same method of induction. In the absence of an inducer for the Cas9 promoter, no gene editing events take place and the bacteria are the color resulting from expression of chromogenic protein (GFP) (in the presence of inducer, if an inducible promoter is used for GFP). In the presence of the Cas9 promoter-specific inducer, Cas9 expression is induced and the gRNA will be incorporated into Cas9, leading to targeting of GFP. Repair of the Cas9 cleavage is directed by the HR donor DNA contained in the transformed plasmid, allowing incorporation of specific mutations that prevent GFP expression, such that the bacteria no longer express GFP and the bacterial colonies appear white in color.

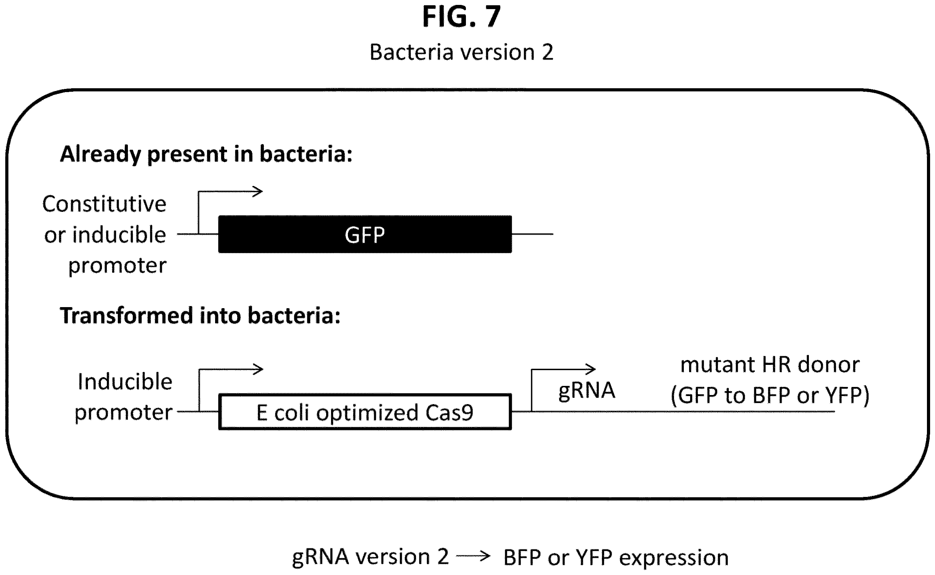

FIG. 7. Bacteria Version 2. The bacteria express a chromogenic or fluorescent protein (GFP) under the control of a constitutive or inducible promoter. An episomal plasmid that expresses a gRNA to GFP and expresses Cas9 under the control of an inducible promoter can be transformed into the bacteria. Note that if both GFP and Cas9 expression rely on inducible promoters, they may not use the same method of induction. In the absence of an inducer for the Cas9 promoter, no gene editing events take place and the bacteria are the color resulting from expression of chromogenic protein (GFP) (in the presence of an inducer, if an inducible promoter is used for GFP). In the presence of the Cas9 promoter-specific inducer, Cas9 expression is induced and the gRNA will be incorporated into Cas9, leading to targeting of GFP. Repair of the Cas9 cleavage is directed by the HR donor DNA contained in the transformed plasmid, allowing incorporation of specific mutations that convert GFP to BFP or YFP, such that the bacteria express BFP or YFP and the bacterial colonies appear blue or yellow in color, respectively.

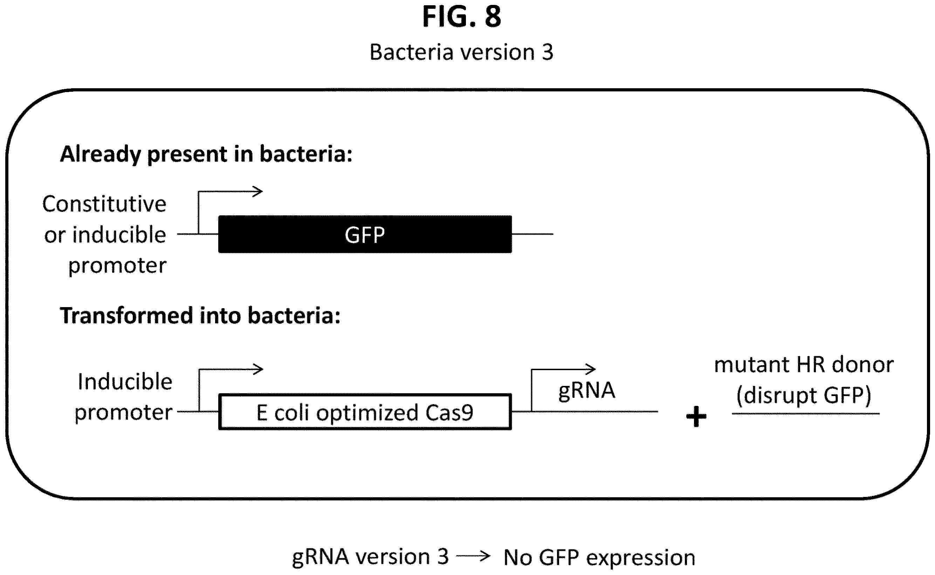

**FIG. 8.** Bacteria Version 3. The bacteria express a chromogenic or fluorescent protein (GFP) under the control of a constitutive or inducible promoter. An episomal plasmid that expresses a gRNA to GFP and expresses Cas9 under the control of an inducible promoter can be transformed into the bacteria. Note that if both GFP and Cas9 expression rely on inducible promoters, they may not use the same method of induction. In the absence of an inducer for the Cas9 promoter, no gene editing events take place and the bacteria are the color resulting from expression of chromogenic protein (GFP) (in the presence of inducer, if an inducible promoter is used for GFP). In the presence of the Cas9 promoter-specific inducer, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that prevent GFP expression, such that the bacteria no longer express GFP and the bacterial colonies appear white in color.

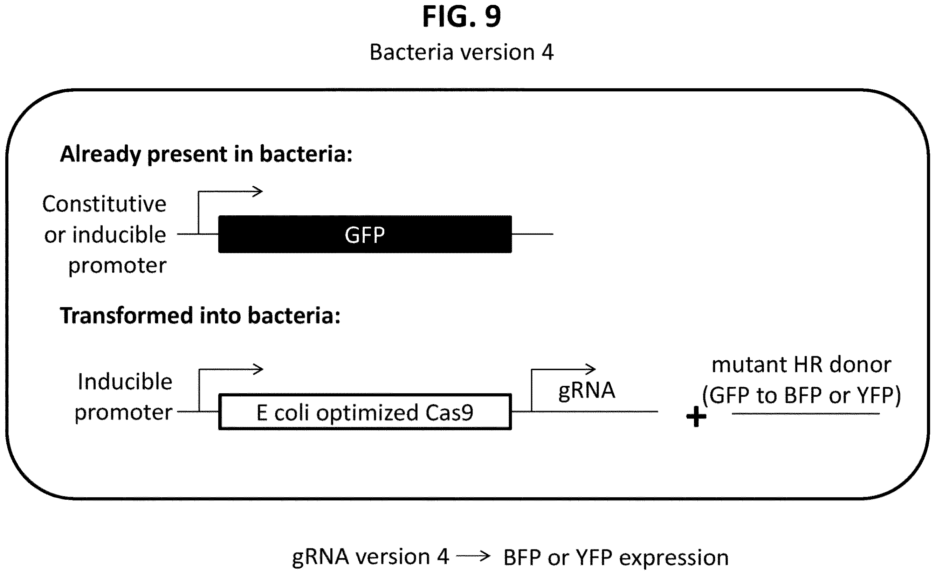

**FIG. 9.** Bacteria Version 4. The bacteria express a chromogenic or fluorescent protein (GFP) under the control of a constitutive or inducible promoter. An episomal plasmid that expresses a gRNA to GFP and expresses Cas9 under the control of an inducible promoter (such as an arabinose-inducible promoter or a rhamnose-inducible promoter) can be transformed into the bacteria. Note that if both GFP and Cas9 expression rely on inducible promoters, they may not use the same method of induction. In the absence of an inducer for the Cas9 promoter, no gene editing events take place and the bacteria are the color resulting from expression of chromogenic protein (GFP) (in the presence of inducer, if an inducible promoter is used for GFP). In the presence of the Cas9 promoter-specific inducer, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that convert GFP to BFP or YFP, such that the bacteria express BFP or YFP and the bacterial colonies appear blue or yellow in color, respectively.

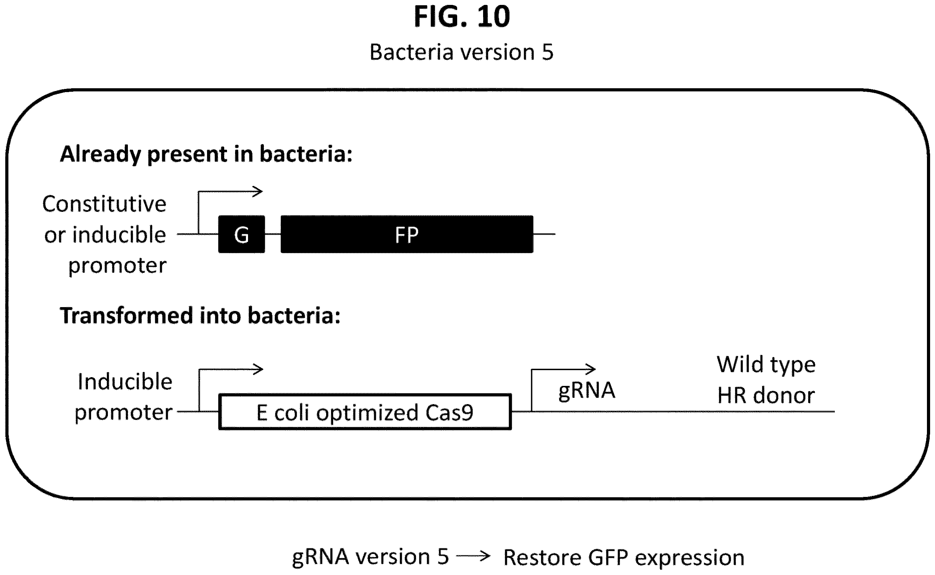

**FIG. 10.** Bacteria Version 5. The bacteria express a non-functional chromogenic or fluorescent protein (GFP) under the control of a constitutive or inducible promoter. An episomal plasmid that expresses a gRNA and expresses Cas9 under the control of an inducible promoter can be transformed into the bacteria. Note that if both GFP and Cas9 expression rely on inducible promoters, they may not use the same method of induction. In the absence of an inducer for the Cas9 promoter, no gene editing events take place and the bacteria are the color white, even in the presence of inducer, if an inducible promoter is used for GFP. In the presence of the Cas9 promoter-specific inducer, Cas9 expression is induced and the gRNA will be incorporated into Cas9, leading to targeting of GFP. Repair of the Cas9 cleavage is directed by the HR donor DNA contained in the transformed plasmid, allowing incorporation of specific mutations that restore GFP expression, such that the bacteria will express a functional GFP and the bacterial colonies appear green in color.

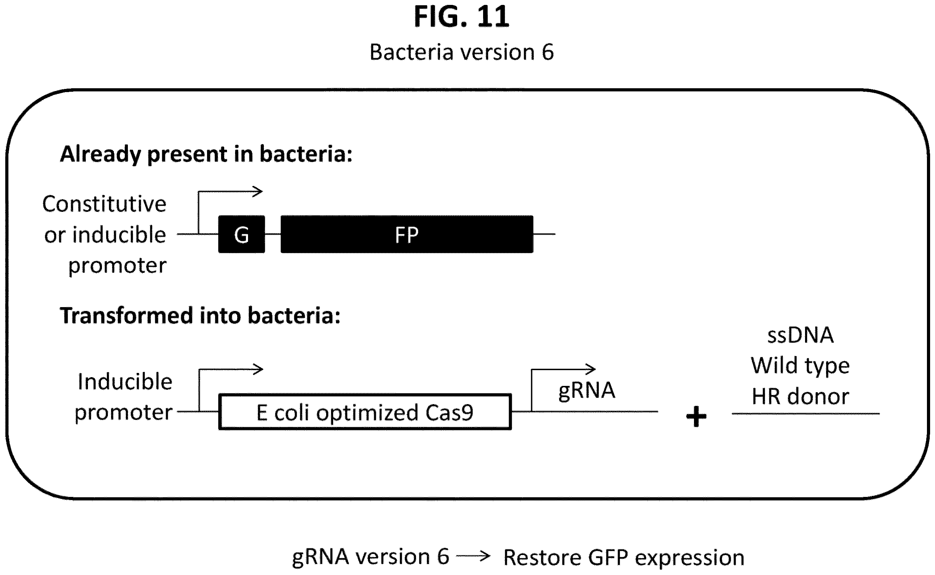

**FIG. 11.** Bacteria Version 6. The bacteria express a non-functional chromogenic or fluorescent protein (GFP) under the control of a constitutive or inducible promoter. An episomal plasmid that expresses a gRNA and expresses Cas9 under the control of an inducible promoter can be transformed into the bacteria. Note that if both GFP and Cas9 expression rely on inducible promoters, they may not use the same method of induction. In the absence of an inducer for the Cas9 promoter, no gene editing events take place and the bacteria are the color white, even in the presence of inducer, if an inducible promoter is used for GFP. In the presence of the Cas9 promoter-specific inducer, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that restore GFP expression, such that the bacteria will express a functional GFP and the bacterial colonies appear green in color.

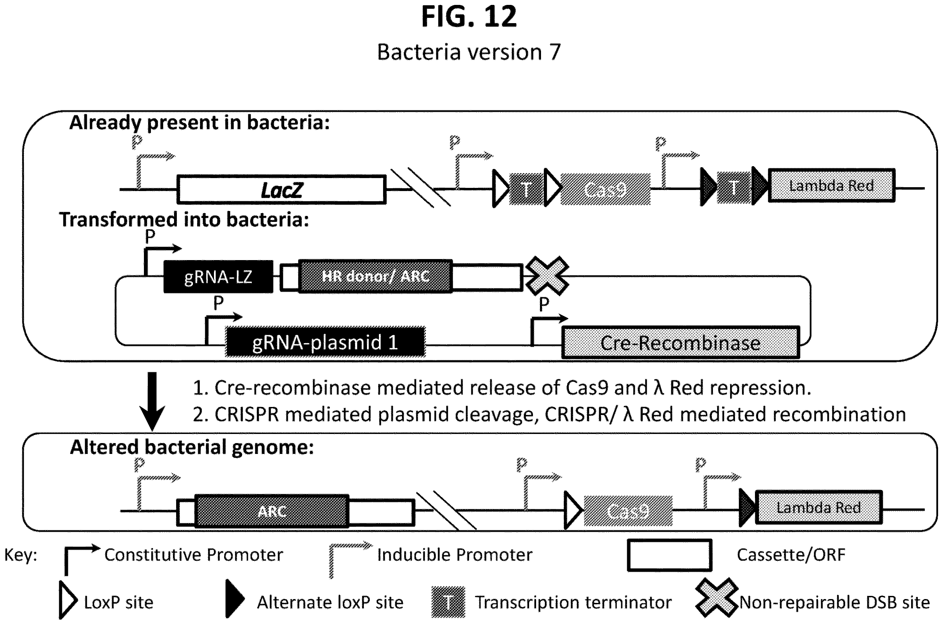

**FIG. 12.** Bacteria Version 7. The bacteria are genetically altered to have inducible Cas9 and inducible lambda red expression cassettes inserted within the bacterial genome. Both of these inducible promoters have loxP or loxP variant sequences that flank a transcription terminator sequence directly following the promoter, which block transcription and prevent expression of the induced proteins. The genetically altered bacteria are transformed with a single plasmid that contains an antibiotic resistance cassette (ARC) lacking its own promoter that is flanked on the 5' and 3' ends with short portions of an endogenous target gene (LacZ), an sgRNA that recognizes the targeted gene ("sgRNA-LZ"), a constitutively expressed Cre-recombinase cassette, and a "self-destruct" sgRNA that targets a self region within the plasmid, termed a non-repairable DSB site ("sgRNA-plasmid 1"). Upon expression of Cre recombinase, both of the transcription termination sequences will be excised, leaving a single loxP or loxP variant "scar" behind, and transcription and translation will become undeterred. A constitutively active Cas9 cassette will initiate Cas9 expression. The plasmid is destroyed; only bacteria that integrate the ARC will survive selection by that antibiotic. Upon induction of CRISPR-Cas and lambda red, recombination repair will allow the cell to express the antibiotic resistance protein and lose normal targeted gene expression (LacZ); these bacteria will survive in the presence of the antibiotic and will exhibit a loss of the normal targeted gene's function (e.g., loss of LacZ expression).

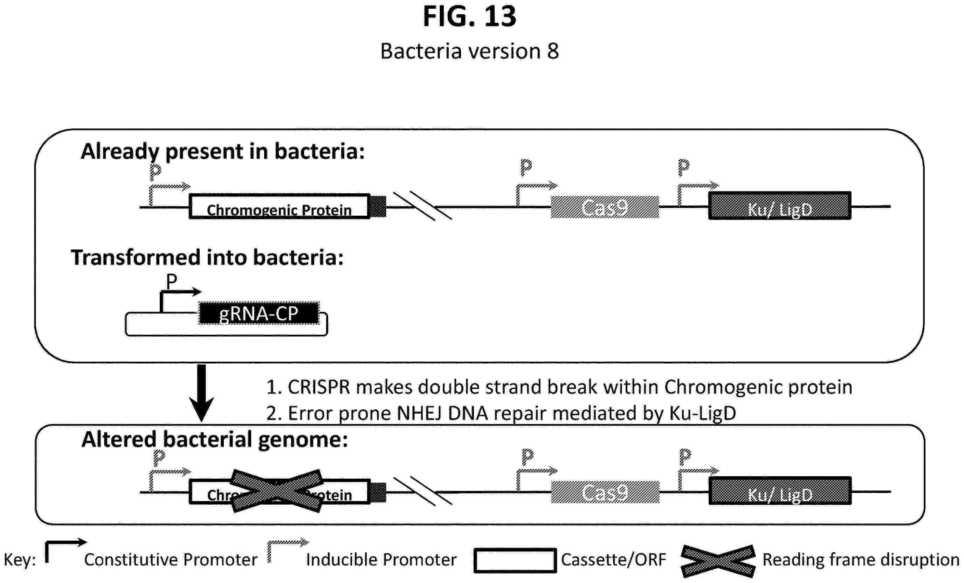

**FIG. 13.** Bacteria Version 8. Bacteria are genetically altered to have inducible Cas9 and Ku and LigD expression cassettes present. Additionally, an endogenous gene is replaced with an open reading frame (ORF) for a particular phenotype of interest (e.g., chromogenic protein ORF). These altered bacteria will express the chromogenic protein upon activation of the endogenous gene. The genetically altered bacteria are transformed with a single plasmid that has a cassette driving expression of an sgRNA that recognizes the chromogenic protein. Upon induction, a DSB occurs within the chromogenic protein. In contrast to unaltered *E coli,* the altered bacteria are able to perform non-homologous end joining (NHEJ)-based repair of the DSB break. Ku and LigD proteins will recognize the DSB and rejoin the two broken ends.

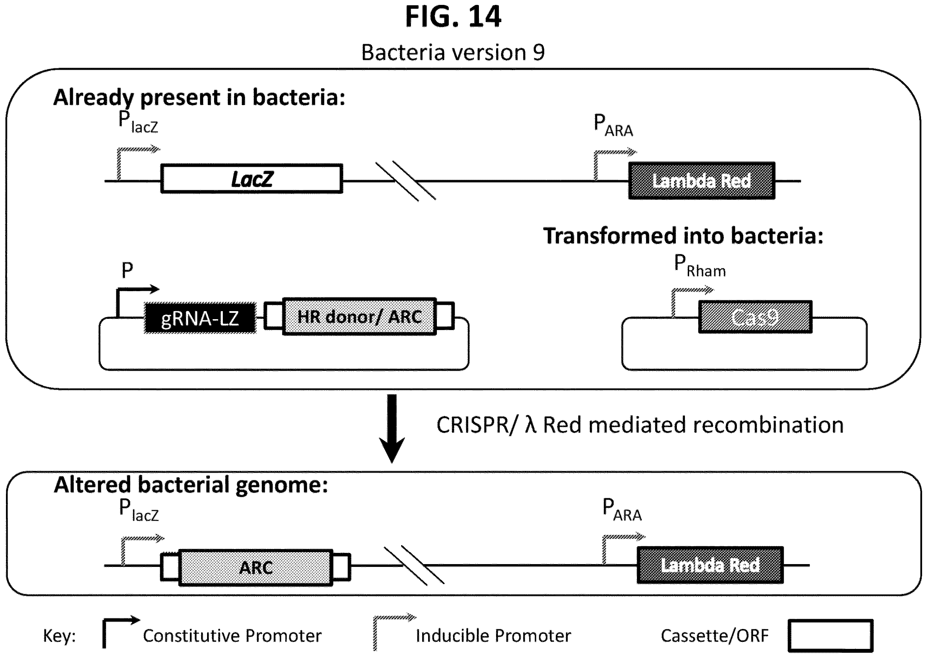

**FIG. 14.** Bacteria Version 9. Bacteria are genetically altered to have inducible CRISPR activity and inducible lambda red expression cassettes. Upon induction of CRISPR and lambda red recombination, an ORF (e.g., encoding an antibiotic resistance cassette (ARC)) lacking its own promoter that is flanked on the 5' 3' ends with homology arms derived from an endogenous target gene (LacZ) will replace portions of the endogenous gene. This results in a loss of function for the endogenous gene, and instead the native or altered endogenous promoter drives the expression of the novel ORF, giving the bacteria a gain of function simultaneous to loss of function of the endogenous gene. Surviving bacteria will be screened for and categorized for ratios of colonies exhibiting the loss and the gain of phenotypes.

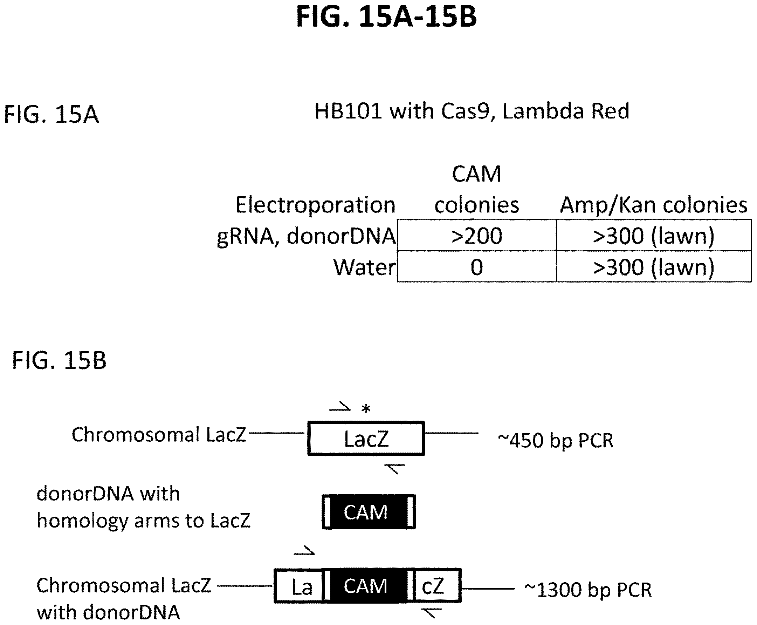

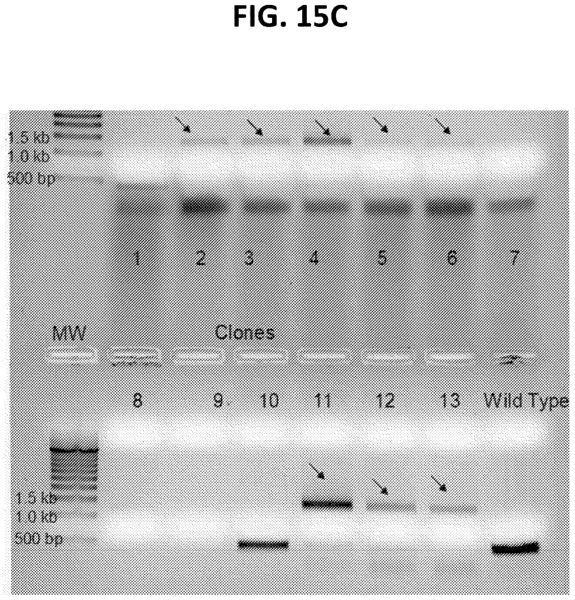

**FIGS. 15A-15C.** Cas9-mediated integration of chloramphenicol (CAM) resistance in bacteria. (A) Number of CAM$^+$ colonies electroporated with either gRNA/donorDNA or water (control). (B) PCR schema for detection of wild-type LacZ and Cas9-directed integration of CAM-resistance cassette into LacZ. (C) PCR results from 13 colonies screened. Arrows indicate successful integration of CAM-resistance cassette.

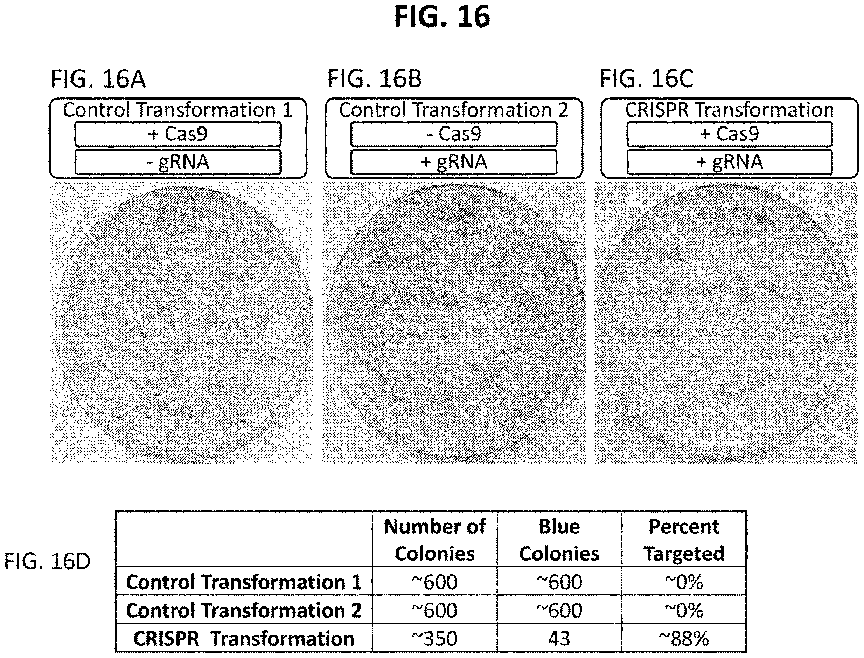

FIGS. 16A-16D. Blue/white screening to detect CRISPR and lambda red dependent stop codon inserted into LacZ reading frame. (A-C) Representative bacterial plates used for blue/white screening. Bacteria were transformed with plasmids containing the following expression cassettes: a Cas9 cassette but no gRNA expressing cassette (A, Control Transformation 1), a gRNA expressing cassette but no Cas9 expressing cassette (B, Control Transformation 2), or a gRNA expressing cassette and a Cas9 expressing cassette (C, CRISPR Transformation.) Without CRISPR activity, 100% of cells were blue showing normal β-galactosidase activity. When CRISPR was activated, only 43 of the ~350 colonies seen were blue while approximately 88% of 350 colonies were white displaying a lack of β-galactosidase activity. (D) Table of results.

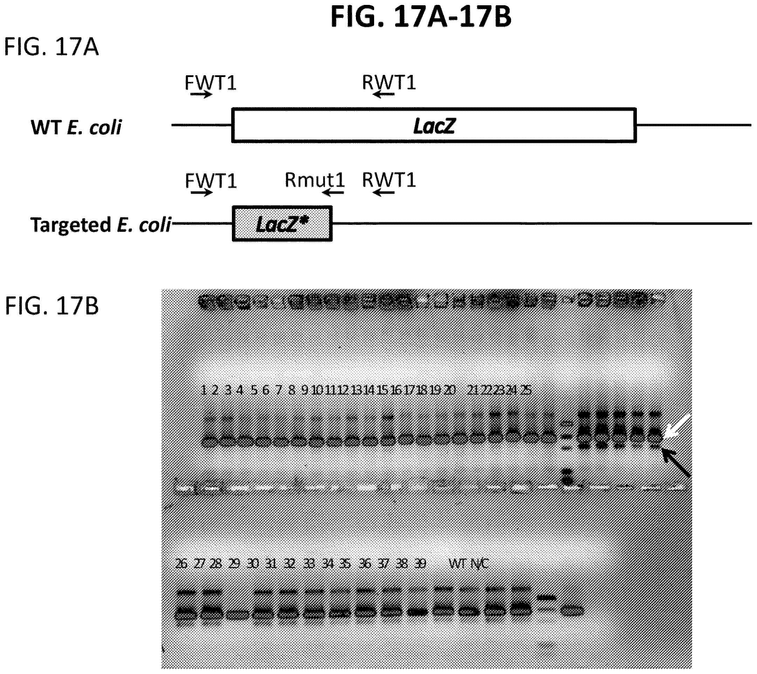

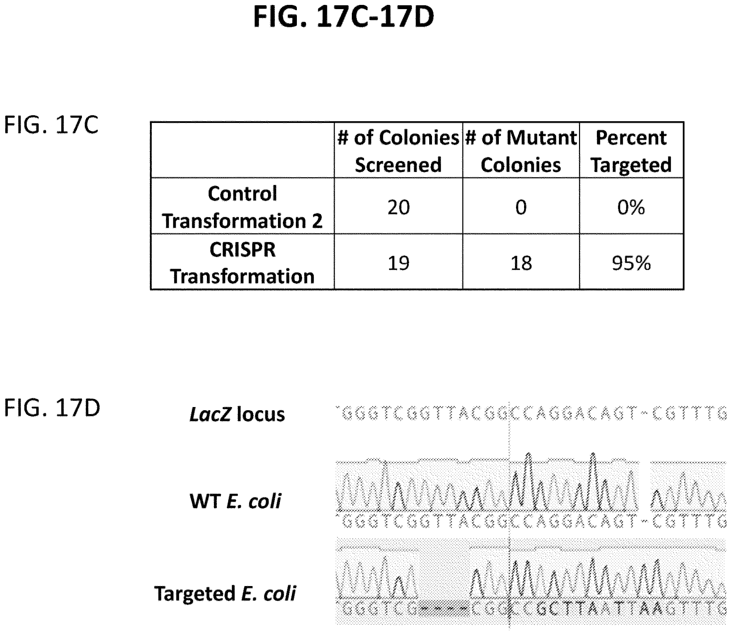

**FIG. 17A-17D.** PCR screening and sequencing to detect CRISPR and lambda red dependent stop codon inserted into LacZ reading frame. (A) PCR genotyping strategy. Wild-type and *mutant E. coli* (FWT1 and FWT2) produced a 686 base pair amplicon. LacZ mutant *E. coli* (FWT1 and Rmut1) produce a 550 base pair amplicon. (B) DNA gel image from genotyping PCR. Lanes 1-20 are from control transformation 2. Lanes 21-39 are from CRISPR transformation. Wild-type band is white arrow. Mutant only band is black arrow. (C) Table of results from PCR genotyping. (D) Representative Sanger sequencing with wild-type LacZ locus sequence and traces from wild-type *E. coli* and premature stop codon inserted *E. coli.*

DETAILED DESCRIPTION OF THE INVENTION

**I. Introduction**

Provided herein are compositions, kits, and methods for using the CRISPR/Cas system for altering gene expression in microbial organism cells and/or microbial organisms. As described herein, the control of gene expression in a cell or an organism (e.g., a microbe, e.g., yeast) due to CRISPR/Cas activity can be visualized using polynucleotides comprising coding sequences for proteins that produce a detectable phenotype in the cell or organism, e.g., chromogenic and/or fluorogenic proteins, proteins that regulate odor, proteins that regulate morphology, resistance to an antibiotic, or enzymes that produce a detectable enzymatic product. In particular, control of gene expression in the cell or organism is visualized using polynucleotides comprising coding sequences for chromogenic and/or fluorogenic proteins. Endogenous genes or genomic targets are targeted for alteration using the CRISPR/Cas system. Through the use of an inducible promoter to control expression of the CRISPR/Cas system, changes in phenotype (e.g., color) in the cell or organism due to gene disruption and repair can be readily detected.

Also provided herein are compositions, kits, and methods for using lambda red recombineering, with the CRISPR/Cas system, for altering gene expression in microbial organism cells and/or microbial organisms. As described herein, lambda red can be used on its own to target and replace regions within genomic DNA, although lambda red recombineering is less efficient without the directed nuclease activity from Cas9-sgRNA functional units. Thus, the success rate of lambda red recombineering is quantifiably higher when it is used in conjunction with CRISPR/Cas. Accordingly, the compositions, kits, and methods provided herein can be used for comparative analyses of gene targeting when the CRISPR/Cas system is used versus in its absence.

## II. Definitions

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry, and nucleic acid chemistry and hybridization described below are those well-known and commonly employed in the art. Standard techniques are used for nucleic acid synthesis. The techniques and procedures are generally performed according to conventional methods in the art and various general references (see generally, Sambrook et al. MOLECULAR CLONING: A LABORATORY MANUAL, 2d ed. (1989) Cold Spring Harbor Laboratory Press, Cold Spring Harbor, N.Y.), which are provided throughout this document.

As used herein, the term "Cas nuclease" or "Cas" refers to CRISPR associated protein, an RNA-guided nuclease that introduces a double stranded break in nucleic acid. In some embodiments, the Cas nuclease is CRISPR associated protein 9 ("Cas9 nuclease" or "Cas9").

The term "guide RNA" or "gRNA," as used herein, refers to a nucleic acid sequence that guides a nuclease (e.g., Cas nuclease) to a target nucleic acid site to be cleaved. Typically, a gRNA comprises a "scaffold" sequence for binding the nuclease and a "targeting" sequence that defines the target nucleic acid site (e.g., genomic DNA site) to be cleaved. In some embodiments, the gRNA comprises a targeting sequence that has a length of about 20 nucleotides.

The terms "lambda red" or "lambda red system," as used herein, refers to a lambda red recombineering system that is derived from the lambda red bacteriophage. The lambda red recombineering system has three components: lambda exonuclease ("Exo"), beta protein ("Beta"), and gamma protein ("Gam"). In some embodiments, "a polynucleotide encoding lambda red" refers to a polynucleotide that encodes the Exo, Beta, and Gam components of the lambda red system.

As used herein, the terms "nucleic acid" and "polynucleotide" interchangeably refer to DNA, RNA, and polymers thereof in single-stranded, double-stranded, or multi-stranded form. The term includes single-, double- or multi-stranded DNA or RNA, genomic DNA, cDNA, DNA-RNA hybrids, or a polymer comprising purine and/or pyrimidine bases or other natural, chemically modified, biochemically modified, non-natural, synthetic or derivatized nucleotide bases. In some embodiments, a nucleic acid can comprise a mixture of DNA, RNA and analogs thereof. Unless specifically limited, the term encompasses nucleic acids containing known analogs of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, single nucleotide polymorphisms (SNPs), and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)). The term nucleic acid is used interchangeably with gene, cDNA, and mRNA encoded by a gene.

Sequences are "substantially identical" to each other if they have a specified percentage of nucleotides that are the same (e.g., at least 60%, 65%, 70%, 75%, 80%, 85%, 90%, 91%, 92%, 93%, 94%, 95%, 96%, 97%, 98%, or 99% identity over a specified region), when compared and aligned for maximum correspondence over a comparison window, or designated region as measured using one of the following sequence comparison algorithms or by manual alignment and visual inspection.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window," as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are well-known in the art. Optimal alignment of sequences for comparison can be conducted, e.g., by the local homology algorithm of Smith & Waterman, Adv. Appl Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Accelrys), or by manual alignment and visual inspection.

Algorithms suitable for determining percent sequence identity and sequence similarity are the BLAST and BLAST 2.0 algorithms, which are described in Altschul et al. (Nuc. Acids Res. 25:3389-402, 1977), and Altschul et al. (J. Mol. Biol. 215:403-10, 1990), respectively. Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al.,* supra). These initial neighborhood word hits act as seeds for initiating searches to find longer HSPs containing them. The word hits are extended in both directions along each sequence for as far as the cumulative alignment score can be increased. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLAST program uses as defaults a word length (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands.

The BLAST algorithm also performs a statistical analysis of the similarity between two sequences (see, e.g., Karlin & Altschul, Proc. Nat'l. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The term "promoter" refers to regions or sequence located upstream and/or downstream from the start of transcription and which are involved in recognition and binding of RNA polymerase and other proteins to initiate transcription.

A polynucleotide sequence is "heterologous" to an organism or a second polynucleotide sequence if it originates from a foreign species, or, if from the same species, is modified from its original form. For example, when a promoter is said to be operably linked to a heterologous coding sequence, it means that the coding sequence is derived from one species whereas the promoter sequence is derived another, different species; or, if both are derived from the same species, the coding sequence is not naturally associated with the promoter (e.g., is a different gene in the same species).

The term "operably linked" refers to a functional linkage between a nucleic acid expression control sequence (such as a promoter, or array of transcription factor binding sites) and a second nucleic acid sequence, wherein the expression control sequence directs transcription of the nucleic acid corresponding to the second sequence.

The term "expression cassette" refers to a nucleic acid construct that, when introduced into a host cell, results in transcription and/or translation of an RNA or polypeptide, respectively.

A "vector" refers to a polynucleotide, which when independent of the host chromosome, is capable replication in a host organism. Preferred vectors include plasmids and typically have an origin of replication. Vectors can comprise, e.g., transcription and translation terminators, transcription and translation initiation sequences, and promoters useful for regulation of the expression of the particular nucleic acid.

### III. **Engineered Cells and Organisms**

Engineered microbial organisms and engineered microbial organism cells are provided that comprise one or more phenotype coding sequences, a Cas nuclease, and a guide RNA (gRNA) that corresponds to the phenotype coding sequence. In some embodiments, the microbial organisms and engineered microbial organism cells comprise two or more phenotype coding sequences and further comprise a separate gRNA that corresponds to each of the two or more phenotype coding sequences. The terms "first," "second," "third," "fourth," and "fifth," when used with reference to polynucleotide sequences, promoters, and auxotrophic, antibiotic, or other selectable markers, is simply to more clearly distinguish the polynucleotide sequences, promoters, and auxotrophic, antibiotic, or other selectable markers, and is not intended to indicate order.

The engineered microbial organism (including engineered eukaryotic organism) or the engineered microbial cell (including engineered eukaryotic cell) may comprise:

an endogenous gene or genomic region to be targeted for gene alteration, wherein the endogenous gene or genomic region is a functional endogenous gene or genomic region having a detectable phenotype or comprises a disruption that prevents expression of the detectable phenotype; or one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype;

a heterologous polynucleotide sequence comprising a promoter operably linked to a polynucleotide encoding a Cas nuclease; and

a heterologous polynucleotide sequence comprising a guide RNA (gRNA) that targets the endogenous gene, genomic region, or phenotype coding sequence.

The engineered microbial organism or the engineered microbial organism cell may comprise:

an endogenous gene or genomic region to be targeted for gene alteration, wherein the endogenous gene or genomic region is a functional endogenous gene or genomic region having a detectable phenotype or comprises a disruption that prevents expression of the detectable phenotype; or a heterologous polynucleotide sequence comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype;

a heterologous polynucleotide sequence comprising a promoter operably linked to a polynucleotide encoding lambda red; and

a heterologous polynucleotide sequence comprising a homologous donor DNA sequence.

The engineered microbial organism or the engineered microbial organism cell may comprise:

an endogenous gene or genomic region to be targeted for gene alteration, wherein the endogenous gene or genomic region is a functional endogenous gene or genomic region having a detectable phenotype or comprises a disruption that prevents expression of the detectable phenotype; or a heterologous polynucleotide sequence comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype;

a heterologous polynucleotide sequence comprising a promoter operably linked to a polynucleotide encoding a Cas nuclease (e.g., Cas9 nuclease);

a heterologous polynucleotide sequence comprising a promoter operably linked to a polynucleotide encoding lambda red;

a heterologous polynucleotide sequence comprising a gRNA that targets the endogenous gene, genomic region, or chromogenic coding sequence; and

a heterologous polynucleotide sequence comprising a homologous donor DNA sequence.

The engineered cell comprises both a polynucleotide encoding a Cas nuclease and a polynucleotide encoding lambda red, and it is preferred that the Cas nuclease polynucleotide sequence and the lambda red polynucleotide sequence are operably linked.

The engineered microbial organism or the engineered microbial organism cell may comprise:

a heterologous polynucleotide sequence comprising a chromogenic coding sequence that encodes a functional chromogenic protein;

a heterologous polynucleotide sequence comprising a promoter operably linked to a polynucleotide encoding a Cas nuclease (e.g., Cas9 nuclease);

a heterologous polynucleotide sequence comprising a gRNA that targets the endogenous gene, genomic region, or chromogenic coding sequence; and

a heterologous polynucleotide sequence comprising a homologous donor DNA sequence.

The phenotype may be a chromogenic phenotype, e.g., a color or fluorescence. The chromogenic coding sequence may encode a non-fluorescent color protein, or a fluorogenic protein, e.g., a green fluorescent protein (GFP), blue fluorescent protein (BFP), red fluorescent protein (RFP), orange fluorescent protein (OFP), yellow fluorescent protein (YFP), cyan fluorescent protein (CFP), coral fluorescent protein, and derivatives or variants thereof. In particular, the chromogenic coding sequence encodes a fluorogenic protein and the homologous donor DNA comprises a mutation that prevents expression of the fluorogenic protein. Further, the chromogenic coding sequence encodes a fluorogenic protein and the homologous donor DNA comprises a mutation that modifies expression of the fluorogenic protein from a first color to a second color (e.g., from GFP to BFP or YFP). Further, the chromogenic coding sequence comprises an engineered disruption that prevents expression of the detectable protein (e.g., a "broken" fluorogenic protein) and the homologous donor DNA comprises a mutation that restores expression of the fluorogenic protein.

The engineered microbial organism (including engineered eukaryotic organism) or the engineered microbial cell (including an engineered eukaryotic cell) comprises:

a first heterologous polynucleotide sequence comprising a first phenotype coding sequence operably linked to a first promoter, wherein the first phenotype coding sequence either (i) encodes a functional first protein having a first detectable phenotype or (ii) comprises an engineered disruption in a sequence encoding a first protein that prevents expression of the first detectable phenotype;

a second heterologous polynucleotide sequence comprising a second phenotype coding sequence operably linked to a second promoter, wherein the second phenotype coding sequence either (i) encodes a functional second protein having a second detectable phenotype or (ii) comprises an engineered disruption in a sequence encoding a second protein that prevents expression of the second detectable phenotype;

a third heterologous polynucleotide sequence comprising a third promoter operably linked to a polynucleotide encoding a Cas nuclease (e.g., Cas9 nuclease);

a fourth heterologous polynucleotide sequence comprising a first gRNA that targets the first chromogenic coding sequence; and

a fifth heterologous polynucleotide sequence comprising a second gRNA that targets the second chromogenic coding sequence.

The detectable phenotype or each of the one or more detectable phenotypes (e.g., each of the first detectable phenotype and the second detectable phenotype) may be a detectable color, fluorescence, scent, enzymatic activity, antibiotic resistance (gain or loss), morphology, or lethality. In some embodiments, both the first detectable phenotype and the second detectable phenotype are the same category of phenotype (e.g., both are detectable colors and/or fluorescences; both are detectable scents; both are detectable enzymatic activities; both are antibiotic resistance (gain or loss); or both are detectable morphologies). In some embodiments, the first detectable phenotype is a different category of phenotype than the second detectable phenotype (e.g., one is a detectable color and/or fluorescence and one is a detectable scent).

The first detectable phenotype and the second detectable phenotype is a detectable color and/or fluorescence, and the engineered microbial organism (including an engineered eukaryotic organism) or the engineered microbial cell (including an engineered eukaryotic cell) may comprise:

a first heterologous polynucleotide sequence comprising a first chromogenic coding sequence operably linked to a first promoter, wherein the first chromogenic coding sequence either (i) encodes a functional first chromogenic protein or (ii) comprises an engineered disruption in a sequence encoding a first chromogenic protein that prevents expression of the first chromogenic protein;

a second heterologous polynucleotide sequence comprising a second chromogenic coding sequence operably linked to a second promoter, wherein the second chromogenic coding sequence either (i) encodes a functional second chromogenic protein or (ii) comprises an engineered disruption in a sequence encoding a second chromogenic protein that prevents expression of the second chromogenic protein;

a third heterologous polynucleotide sequence comprising a third promoter operably linked to a polynucleotide encoding a Cas9 nuclease;

a fourth heterologous polynucleotide sequence comprising a first guide RNA (gRNA) that targets the first chromogenic coding sequence; and

a fifth heterologous polynucleotide sequence comprising a second gRNA that targets the second chromogenic coding sequence.

In some embodiments, the engineered microbial organism is a eukaryotic organism.

The engineered microbial organism cell may be a cell obtained from a prokaryotic organism, or eukaryotic organism as described herein. In some embodiments, the engineered microbial organism cell is, e.g., a cell from a bacterium, phytoplasma, virus, viroid, protozoan, rickettsia, or fungus. In some embodiments, the engineered cell is a prokaryotic cell, e.g., from a bacterium. In some embodiments, the engineered cell is eukaryotic cell, e.g., is a yeast cell.

*Cells and Organisms*

The engineered microbial organism cell may be a cell from a microbial organism, e.g., a bacterium, phytoplasma, virus, viroid, protozoan, rickettsia, or fungus. In some embodiments, the engineered cell is a eukaryotic cell. Further, the engineered cell may be a prokaryotic.

The engineered microbial organism cell may be fungal, e.g., from a species of yeast, mold, or filamentous fungus. Further, the cell may be a eukaryotic cell from a filamental fungus, e.g., a species of *Achlya, Acremonium, Aspergillus, Aureobasidium, Bjerkandera, Ceriporiopsis, Cephalosporium, Chrysosporium, Cochliobolus, Corynascus, Cryphonectria, Cryptococcus, Coprinus, Coriolus, Diplodia, Endothis, Fusarium, Gibberella, Gliocladium, Humicola, Hypocrea, Myceliophthora, Mucor, Neurospora, Penicillium, Podospora, Phlebia, Piromyces, Pyricularia, Rhizomucor, Rhizopus, Schizophyllum, Scytalidium, Sporotrichum, Talaromyces, Thermoascus, Thielavia, Trametes, Tolypocladium, Trichoderma, Verticillium,* or *Volvariella.* Further, the cell may be a eukaryotic cell from a yeast, e.g., a species of *Candida, Hansenula, Saccharomyces, Schizosaccharomyces, Pichia, Kluyveromyces,* or *Yarrowia,* or may be a eukaryotic cell from a species of *Saccharomyces,* e.g., *S. cerevisiae,* or may be a eukaryotic cell from a species of *Schizosaccharomyces,* e.g., *S. pombe.*

The cell may be a prokaryotic cell from a bacterium, e.g., a species *of Escherichia, Streptomyces, Zymonas, Acetobacter, Citrobacter, Synechocystis, Rhizobium, Clostridium, Corynebacterium, Streptococcus, Xanthomonas, Lactobacillus, Lactococcus, Bacillus, Alcaligenes, Pseudomonas, Aeromonas, Azotobacter, Comamonas, Mycobacterium, Rhodococcus, Gluconobacter, Ralstonia, Acidithiobacillus, Microlunatus, Geobacter, Geobacillus, Arthrobacter, Flavobacterium, Serratia, Saccharopolyspora, Thermus, Stenotrophomonas, Chromobacterium, Sinorhizobium, Saccharopolyspora, Agrobacterium, Paracoccus,* or *Pantoea.* In particular, the cell may be a prokaryotic cell from a species of *Escherichia,* e.g., *E. coli.*

The cell may be a protist cell, e.g., a species of *Dictyostelium.*

The engineered microbial organism may be a microbial organism, e.g., a bacterium, phytoplasma, virus, viroid, protozoan, rickettsia, or fungus. In some embodiments, the engineered microbial organism is eukaryotic. The engineered microbial organism may be prokaryotic.

The engineered microbial organism may be fungal, i.e., a eukaryotic organism within the kingdom of fungi. Fungi may include yeasts, molds, and filamentous fungi. In some embodiments, the eukaryotic organism is a filamental fungus, e.g., a species of *Achlya, Acremonium, Aspergillus, Aureobasidium, Bjerkandera, Ceriporiopsis, Cephalosporium, Chrysosporium, Cochliobolus, Corynascus, Cryphonectria, Cryptococcus, Coprinus, Coriolus, Diplodia, Endothis, Fusarium, Gibberella, Gliocladium, Humicola, Hypocrea, Myceliophthora, Mucor, Neurospora, Penicillium, Podospora, Phlebia, Piromyces, Pyricularia, Rhizomucor, Rhizopus, Schizophyllum, Scytalidium, Sporotrichum, Talaromyces, Thermoascus, Thielavia, Trametes, Tolypocladium, Trichoderma, Verticillium,* or *Volvariella.* In particular, the eukaryotic organism may be a yeast, e.g., a species of *Candida, Hansenula, Saccharomyces, Schizosaccharomyces, Pichia, Kluyveromyces,* or *Yarrowia,* or may be is a species of *Saccharomyces,* e.g., *S. cerevisiae,* or may be a species of *Schizosaccharomyces,* e.g., *S. pombe.*

The engineered microbial organism may be bacterial, e.g., a species of *Escherichia, Streptomyces, Zymonas, Acetobacter, Citrobacter, Synechocystis, Rhizobium, Clostridium, Corynebacterium, Streptococcus, Xanthomonas, Lactobacillus, Lactococcus, Bacillus, Alcaligenes, Pseudomonas, Aeromonas, Azotobacter, Comamonas, Mycobacterium, Rhodococcus, Gluconobacter, Ralstonia, Acidithiobacillus, Microlunatus, Geobacter, Geobacillus, Arthrobacter, Flavobacterium, Serratia, Saccharopolyspora, Thermus, Stenotrophomonas, Chromobacterium, Sinorhizobium, Saccharopolyspora, Agrobacterium, Paracoccus,* or *Pantoea.* In sparticular, the engineered organism may be a species of *Escherichia,* e.g., *E. coli.*

The engineered microbial organism organism may be a protist, e.g., a species of *Dictyostelium.*

*Phenotype Coding Sequences*

The engineered microbial organisms and engineered microbial organism cells disclosed herein comprise one or more phenotype coding sequences, or the engineered organism or engineered cell comprises one phenotype coding sequence, or the engineered organism or engineered cell comprises multiple phenotype coding sequences, e.g., 2, 3, 4, 5, 6, or more phenotype coding sequences. The phenotype coding sequences may be chromogenic coding sequences. As used herein, the term "chromogenic" encompasses both color and fluorescence as the detectable phenotype. The phenotype coding sequences may be scent coding sequences, or enzymatic activity coding sequences, , or morphology coding sequences, , or lethality coding sequences, or sequences that code for the gain or loss of resistance to an antibiotic. For example, the antibiotic resistance phenotype coding sequences can be used for replacing one antibiotic resistance for another in the organism or cell (i.e., simultaneous gain and loss of function).

As set forth above, the engineered microbial organism or engineered microbial organism cell may comprise multiple chromogenic coding sequences, e.g., 2, 3, 4, 5, 6, or more chromogenic coding sequences. A chromogenic coding sequence may encode a fluorogenic protein, e.g., a green fluorescent protein (GFP), blue fluorescent protein (BFP), red fluorescent protein (RFP), orange fluorescent protein (OFP), yellow fluorescent protein (YFP), cyan fluorescent protein (CFP), coral fluorescent proteins, luciferase, and derivatives or variants thereof. A chromogenic coding sequence may encode a coral fluorescent protein, or a chromogenic (non- fluorogenic) protein. Chromogenic coding sequences are commercially available, e.g., ProteinPaintbox® fluorescent and chromogenic proteins (ATUM, Newark, CA).

The one or more phenotype coding sequences may encode for one or more components of a metabolic pathway where providing a substrate for the enzymatic reaction results in the production of a smell. As an example, the alcohol acetyltransferase gene, ATF1, can be introduced into a cell (e.g., bacterial cell) and in the presence of isoamyl alcohol, the cell metabolizes the isoamyl alcohol to emit the smell of bananas.

The one or more phenotype coding sequences may encode for gain or loss of resistance to an antibiotic. As an example, a first phenotype coding sequence encoding for resistance to a first antibiotic (e.g., Kanamyin) can be disrupted or replaced with a second phenotype coding sequence encoding for resistance to a second antibiotic (e.g., Spectinomycin).

One or more phenotype coding sequences may be optimized or enhanced for expression in the organism or cell in which the coding sequence is to be expressed, e.g., a cell or organism as described herein. The chromogenic coding sequence may be optimized or enhanced for expression in the organism, e.g., the engineered organism is a yeast and the chromogenic coding sequence is yeast-enhanced. Enhanced or optimized chromogenic sequences are described in the art (see, e.g., Keppler-Ross et al., Genetics, 2008, 179:705-710).

One or more of the phenotype coding sequences (e.g., chromogenic coding sequences) are in-frame and encodes a functional protein (e.g., a functional chromogenic protein).One or more of the phenotype coding sequences (e.g., chromogenic coding sequences) may comprise an engineered disruption in the polynucleotide sequence that prevents expression of the protein having the detectable phenotype (e.g., chromogenic protein).The engineered disruption may be an insertion of a whole open reading frame (ORF) into an existing ORF. The engineered disruption may be an insertion of about 1 to about 20 nucleotides relative to the sequence that encodes the functional protein, e.g., about 1-20, 1-15, 1-10, 2-20, 2-15, 3-20, 3-10, 4-20, 4-10, 5-20, or 5-10 nucleotides relative to the sequence that encodes the functional protein. The engineered disruption may be an insertion of no more than about 20 nucleotides relative to the sequence that encodes the functional protein, e.g., no more than about 15 nucleotides, no more than about 10 nucleotides, or no more than about 5 nucleotides relative to the sequence that encodes the functional protein. Further, the engineered organism or engineered cell may comprise at least a first phenotype coding sequence (e.g., chromogenic coding sequence) that encodes a functional first protein (e.g., first chromogenic protein) and further may comprise at least a second phenotype coding sequence (e.g., chromogenic coding sequence) that comprises an engineered disruption in the polynucleotide sequence that prevents expression of the second protein (e.g., second chromogenic protein).

The engineered microbial organism or engineered microbial organism cell may comprise one or more phenotype coding sequences (e.g., chromogenic coding sequences) and further comprises a coding sequence for a metabolic pathway gene that, depending on whether the gene is expressed or gene expression is prevented, results in a change in color in the organism. As an example, the engineered organism or cell can express a coding sequence for a gene within the adenine biosynthetic pathway (e.g., ADE2 or ADE5) or for a gene within the carotenoid synthesis pathway (e.g., phytoene synthase or lycopene beta cyclase). The coding sequence for the adenine biosynthetic pathway gene (e.g., ADE2 or ADE5) or the carotenoid synthesis pathway may contain an engineered disruption in the sequence that prevents expression of the protein, which in turn causes the organism to have a pink or red color.

The engineered microbial organism or engineered microbial organism cell comprises one or more phenotype coding sequences (e.g., chromogenic coding sequences) and further comprises a coding sequence for a metabolic pathway gene that, depending on whether the gene is expressed or gene expression is prevented, results in a nutrient requirement. As an example, the engineered microbial organism or engineered microbial organism cell can express a coding sequence for a gene within the leucine biosynthetic pathway (e.g. leuB). The coding sequence for the leucine biosynthetic pathway gene (e.g., leuB) may contain an engineered disruption in the sequence that prevents expression of the protein, which in turn causes the organism to have a leucine nutrient requirement.

Each of the phenotype coding sequences (e.g., chromogenic coding sequences) and/or coding sequences for a metabolic pathway gene that alters color in the organism (e.g., ADE2) may be operably linked to a promoter. The coding sequence for the metabolic pathway gene that alters color in the organism (e.g., ADE2) may be a sequence containing an engineered disruption that prevents expression of the metabolic pathway gene, and is operably linked to its native promoter. A phenotype coding sequence (e.g., chromogenic coding sequence may be operably linked to a constitutive (constitutively active) promoter or an inducible promoter. The promoter may be a constitutively active promoter. Suitable constitutively active promoters include Tyr tRNA, Sigma 70 consensus sequence, TEF1, RPL18B, RNR2, TDH3, REV1, PGK, and ADH1.

Each polynucleotide comprising a phenotype coding sequence (e.g., chromogenic coding sequence) as described herein (e.g., a polynucleotide comprising a chromogenic coding sequence that is operably linked to an inducible promoter or a constitutively active promoter) may be in a separate expression cassette, expression vector, or plasmid than the other polynucleotide sequences disclosed herein (e.g., polynucleotides comprising other phenotype coding sequences, polynucleotides encoding a Cas nuclease, or polynucleotides encoding a guide RNA).A first polynucleotide comprising a first phenotype coding sequence as described herein may be in the same expression cassette, vector, or plasmid as a second polynucleotide comprising a second phenotype coding sequence as described herein. Furthermore, two, three, four, or more polynucleotides comprising two, three, four or more phenotype sequences may be in the same expression cassette, vector, or plasmid. The one or more polynucleotides comprising one or more phenotype coding sequences as described herein may be stably integrated into the genomic material (e.g., chromosome) of a cell or organism as described herein.

An expression vector or plasmid, engineered microbial organism cell, or engineered microbial organism as described herein may comprise multiple identical copies (e.g., at least 2, 3, 4, 5, 10, 20, 30, 40, 50, 60, 70, 80, 90, 100 or more copies) of a phenotype coding sequence. An expression vector or plasmid, engineered microbial organism cell, or engineered microbial organism as described herein may comprise multiple identical copies (e.g., at least 2, 3, 4, 5, 10, 20, 30, 40, 50, 60, 70, 80, 90, 100 or more copies) of a first phenotype coding sequence and multiple identical copies (e.g., at least 2, 3, 4, 5, 10, 20, 30, 40, 50, 60, 70, 80, 90, 100 or more copies) of a second phenotype coding sequence.

*Cas Nucleases*

A Cas9 nuclease may be used to create a double-stranded break (DSB) in one or more phenotype coding sequences (e.g., chromogenic coding sequences and/or coding sequences for a metabolic pathway gene) as described herein. The Cas nuclease may be a Cas9 nuclease. The CRISPR/Cas system and Cas activity are described, e.g., in Jinek et al., Science, 2012, 337:816-821; and in Jinek et al., eLife, 2013, 2:e00471. Briefly, Cas cleaves DNA to generate blunt DSBs at sites defined by a guide RNA sequence. The DSBs can then be repaired in the cell or organism by homology-directed repair (HDR), nonhomologous end-joining (NHEJ), or alternative end-joining (A-EJ). The coding sequence that is targeted by Cas (e.g., Cas9) may encode a functional chromogenic protein or functional metabolic pathway protein, and the cleavage and repair of the chromogenic coding sequence or coding sequence for a metabolic pathway gene disrupts expression of the chromogenic protein or metabolic pathway protein. The coding sequence that is targeted by Cas (e.g., Cas9) may comprise an engineered disruption in the sequence that prevents expression of a functional chromogenic protein or functional metabolic pathway protein, and the cleavage and repair of the chromogenic coding sequence or coding sequence for a metabolic pathway gene results in the expression of a functional chromogenic protein or metabolic pathway protein. The coding sequence that is targeted by Cas9 may encode a functional chromogenic protein or functional metabolic pathway protein, and the cleavage and repair of the chromogenic coding sequence or coding sequence for a metabolic pathway gene changes expression of the chromogenic protein to a different chromogene or metabolic pathway protein to a different metabolic pathway.

Cas nucleases, such as Cas9 nucleases, can be derived from any of a variety of bacterial species, including *Streptococcus pyogenes, Streptococcus thermophilus, Veillonella atypical, Fusobacterium nucleatum, Filifactor alocis, Solobacterium moorei, Coprococcus catus, Treponema denticola, Peptoniphilus duerdenii, Catenibacterium mitsuokai, Streptococcus mutans, Listeria innocua, Staphylococcus pseudintermedius, Acidaminococcus intestine, Olsenella uli, Oenococcus kitaharae, Bifidobacterium bifidum, Lactobacillus rhamnosus, Lactobacillus gasseri, Finegoldia magna, Mycoplasma mobile, Mycoplasma gallisepticum, Mycoplasma ovipneumoniae, Mycoplasma canis, Mycoplasma synoviae, Eubacterium rectale, Eubacterium dolichum, Lactobacillus coryniformis* subsp. *Torquens, Ilyobacter polytropus, Ruminococcus albus, Akkermansia muciniphila, Acidothermus cellulolyticus, Bifidobacterium longum, Bifidobacterium dentium, Corynebacterium diphtheria, Elusimicrobium minutum, Nitratifractor salsuginis, Sphaerochaeta globus, Fibrobacter succinogenes* subsp. *Succinogenes, Bacteroides fragilis, Capnocytophaga ochracea, Rhodopseudomonas palustris, Prevotella micans, Prevotella ruminicola, Flavobacterium columnare, Aminomonas paucivorans, Rhodospirillum rubrum, Candidates Puniceispirillum marinum, Verminephrobacter eiseniae, Ralstonia syzygii, Dinoroseobacter shibae, Azospirillum, Nitrobacter hamburgensis, Bradyrhizobium, Wolinella succinogenes, Helicobacter mustelae, Bacillus cereus, Acidovorax ebreus, Pasteurella multocida* subsp. *Multocida, Sutterella wadsworthensis, proteobacterium, Parasutterella excrementihominis, Wolinella succinogenes,* and *Francisella novicida.*

Cas9 polypeptide sequences, and polynucleotide sequences encoding Cas polypeptides, are known in the art. For example, the amino acid sequence of the *Streptococcus pyogenes* wild-type Cas9 polypeptide is set forth, *e.g.,* in NBCI Ref. Seq. No. NP_269215, and the amino acid sequence of *Streptococcus thermophilus* wild-type Cas9 polypeptide is set forth, *e.g.,* in NBCI Ref. Seq. No. WP_011681470. The Cas9 nuclease may be a variant, e.g., a variant that has enhanced specificity (e.g., Cas9-nickase or dCas9-FokI). Cas9 variants and methods of engineering and screening Cas9 variants are described in the art (see, e.g., Murovec et al., Plant Biotechnology Journal, 2017, 15:917-926; Sadhu et al., bioRxiv, 2017, doi: https://doi.org/10.1101/147637; and Casini et al., Nature Biotechnology, 2018, 36:265-271).

The Cas9 polynucleotide sequence may be codon-optimized for expression in a particular cell or organism. For example, the Cas9 polynucleotide sequence may be codon-optimized for expression in human cells (see, DiCarlo et al., Nucleic Acids Res, 2013, 41:4336-4343). Furthermore, the Cas9 polynucleotide sequence may be codon-optimized for expression in a microbial cell or organism as described herein, e.g., for expression in yeast or bacteria. For example, a yeast codon-optimized Cas9 polynucleotide sequence is provided as SEQ ID NO:1, an *E. coli* codon optimized Cas9 polynucleotide is provided as SEQ ID NO:2, and *a S. pyogenes* Cas9 nuclease is provided as SEQ ID NO:3.

The Cas nuclease may be a variant or derivative of Cas9. For example, the Cas nuclease may be a nuclease that is substantially identical (e.g., at least 70, 75, 80, 85, 90, or 95% identical) to a Cas9 nuclease as disclosed herein.

The Cas nuclease may be a nuclease that is substantially identical (e.g., at least 70, 75, 80, 85, 90, or 95% identical) to a *S. pyogenes* Cas9 nuclease, e.g., the *S. pyogenes* Cas9 nuclease set forth in NBCI Ref. Seq. No. NP_269215.

The Cas nuclease may be a destabilized variant of Cas9. Destabilized Cas9 nucleases are known in the art (see, e.g., Senturk et al., Nat Commun, 2017, 8: 14370).

The Cas nuclease may be a Cas-12a nuclease (also known as Cpf1). Cas-12a nucleases are known in the art (see, e.g., Yan et al., ApplEnviron Microbiol, 2017, 83(17); doi: 10.1128/AEM.00947-17). Furthermore, the Cas nuclease may be a Cas-like nuclease.

A polynucleotide encoding a Cas9 nuclease may be operably linked to an inducible promoter. An inducible promoter may be a promoter that can be turned on and off by adding or removing an inducing agent. An inducing agent may be a molecule such as doxycycline, tetracycline, galactose, arabinose, rhamnose, metal ions, alcohol, or a steroid compound. An inducible promoter may be a promoter that is activated by environmental conditions, for example, light or temperature. The promoter may be a galactose inducible promoter. The promoter may be a arabinose inducible promoter. The promoter may be a rhamnose-inducible promoter. The promoter may be a doxycycline inducible promoter. Inducible promoters are described in the art (see, e.g., Mumberg et al., Nucleic Acids Res., 1994, 22:5767-5768; Cao et al., Nucleic Acids Res, 2016, 44:e149). A polynucleotide encoding a Cas9 nuclease may be operably linked to a constitutively active promoter, e.g., a constitutively active promoter as described herein.

An expression cassette, expression vector, or plasmid comprising a yeast or bacteria codon-optimized Cas9 nuclease polynucleotide sequence is provided. The yeast codon-optimized Cas9 nuclease polynucleotide sequence has the sequence of SEQ ID NO: 1 or is substantially identical to SEQ ID NO:1 (e.g., has at least 70%, 75%, 80%, 85%, 90%, 91%, 92%, 93%, 94%, 95%, 96%, 97%, 98%, or 99% identity to SEQ ID NO:1). The E. *coli* codon-optimized Cas9 nuclease polynucleotide sequence has the sequence of SEQ ID NO:2 or is substantially identical to SEQ ID NO:2 (e.g., has at least 70%, 75%, 80%, 85%, 90%, 91%, 92%, 93%, 94%, 95%, 96%, 97%, 98%, or 99% identity to SEQ ID NO:2). An expression cassette, expression vector, or plasmid comprising a *S. pyogenes* Cas9 nuclease polynucleotide sequence is provided. The S. *pyogenes* Cas9 nuclease polynucleotide sequence has the sequence of SEQ ID NO:3 or is substantially identical to SEQ ID NO:3 (e.g., has at least 70%, 75%, 80%, 85%, 90%, 91%, 92%, 93%, 94%, 95%, 96%, 97%, 98%, or 99% identity to SEQ ID NO:3). The Cas9 nuclease polynucleotide sequence may further comprise a tag (e.g., a FLAG tag, an HA tag, a His tag, or a GST tag). The expression cassette, expression vector, or plasmid comprising a yeast codon-optimized Cas9 nuclease polynucleotide sequence may further comprise an inducible promoter as described herein (e.g., a galactose inducible promoter).

The polynucleotide sequence comprising a polynucleotide encoding a Cas nuclease may further comprise a sequence for a selectable marker, such as an auxotrophic marker, an antibiotic resistance marker, or other selectable marker. In particular, the selectable marker may be suitable for selection of bacterial cells.

The polynucleotide sequence comprising a polynucleotide encoding a Cas nuclease may further comprise a sequence for an auxotrophic marker. The auxotrophic marker may be a yeast auxotrophic marker. Examples of auxotrophic markers include LEU2, URA3, LYS2 HIS3, MET17, and TRP1.

The polynucleotide sequence comprising a polynucleotide encoding a Cas nuclease may further comprise a sequence for an antibiotic resistance marker. Examples of antibiotic resistance markers include Ampicillin, Chloramphenicol, Kanamycin, Streptomycin, Tetracycline, Spectinomycin, Gentamycin, and Zeocin.

A polynucleotide encoding a Cas9 nuclease as described herein (e.g., a polynucleotide encoding a Cas9 nuclease that is operably linked to an inducible or constitutive promoter) may be in a separate expression cassette, expression vector, or plasmid than the other polynucleotide sequences disclosed herein (e.g., polynucleotides comprising a phenotype coding sequence or polynucleotides encoding a guide RNA). A polynucleotide encoding a Cas9 nuclease as described herein may be in the same expression cassette, vector, or plasmid as a polynucleotide comprising a phenotype coding sequence as described herein. Further, a polynucleotide encoding a Cas9 nuclease as described herein may be stably integrated into the genomic material (e.g., chromosome) of a cell or organism as described herein.

*Lambda Red*

A lambda red recombinase system is used for making targeted changes as disclosed herein. Lambda red is a genetic engineering tool that enables homologous recombination in bacteria ("recombineering"). The lambda red system, which is derived from lambda bacteriophage, has three components: (1) lambda exonuclease (Exo), which digests the 5' ended strand of dsDNA; (2) beta protein (Beta), which binds to ssDNA and enables strand annealing; and (3) gamma protein (Gam), which binds to the bacterial RecBCD enzyme and inhibits it from digesting linear DNA introduced into *E. coli.* The lambda red system is described in the art (see, e.g., Pyne et al., Applied and Environmental Microbiology, 2015, 81:5103-5114).

The lambda red system (e.g., lambda red components Exo, Beta, and Gam) may be integrated into the genome of an engineered bacterial cell or organism. Further, the lambda red system (e.g., lambda red components Exo, Beta, and Gam) may be provided on a plasmid. Further, the lambda red system (e.g., lambda red components Exo, Beta, and Gam) may be provided on a bacterial artificial chromosome.

The lambda red system (e.g., lambda red components Exo, Beta, and Gam) may be under constitutive expression, e.g., a polynucleotide encoding lambda red (e.g., lambda red components Exo, Beta, and Gam) is operably linked to a constitutively active promoter. The lambda red system (e.g., lambda red components Exo, Beta, and Gam) may be under inducible expression, e.g., a polynucleotide encoding lambda red (e.g., lambda red components Exo, Beta, and Gam) is operably linked to an inducible promoter. An inducible promoter may be a promoter that can be turned on and off by adding or removing an inducing agent. Further, an inducing agent may be a molecule such as doxycycline, tetracycline, galactose, arabinose, rhamnose, metal ions, alcohol, or a steroid compound. Further, an inducible promoter may be a promoter that is activated by environmental conditions, for example, light or temperature. The promoter may be a galactose inducible promoter. The promoter may be an arabinose inducible promoter. The promoter may be a rhamnose-inducible promoter. The promoter may be a doxycycline inducible promoter. The inducer for expression of the lambda red system may be the same as the inducer of another component of the engineered cell (e.g., the inducer for expression of the lambda red system is the same as an inducer that is used for inducing expression of Cas nuclease). The inducer for expression of the lambda red system may be different from the inducer of another component of the engineered cell (e.g., the inducer for expression of the lambda red system is different from an inducer that is used for inducing expression of Cas nuclease).

In particular, lambda red recombineering is used with or without the CRISPR/Cas system to target and replace regions within genomic DNA. The CRISPR/Cas system is used with or without lambda red recombineering to target and replace regions within genomic DNA. The compositions, kits, and methods disclosed herein comprise both Cas9/CRISPR components and lambda red recombineering components for using lambda red recombineering in conjunction with Cas9/CRISPR for altering gene expression in cells and/or organisms. The lambda red system may be provided on a separate plasmid or BAC from other gene editing components (e.g., on a separate plasmid or BAC as Cas9/CRISPR components). The lambda red system may be provided on the same plasmid or BAC as other gene editing components (e.g., on the same plasmid or BAC as Cas9/CRISPR components).

*Genetic Regulatory Elements*

Cas9 and/or lambda red protein expression is regulated by genetic regulatory elements. Thus, the engineered cells and/or organisms disclosed herein comprise components comprising one or more genetic regulatory elements. For example, inducible promoters typically have a certain level of "leakiness," which can make tight regulation of proteins difficult to precisely regulate even in the absence of the inducing element. In order to have more precise regulation of the expression of Cas9 nuclease and the lambda red proteins, inducible promoters (such as inducible promoters operably linked to Cas9 and lambda red) will have loxP or loxP variant sequences that flank a transcription terminator sequence directly following the promoter. In conjunction with an inducible promoter, transcription of Cas9 and lambda red ORFs are blocked by the insertion of transcription terminator sequences directly following the promoter sequence. These termination sequences may be flanked by loxP sites or loxP variants (e.g., lox2722). These terminator sequences may be flanked by FRT sites or FRT variants (e.g., F5). Cre-recombinase or Flp recombinase expression cassettes may be introduced into the engineered cells and/or organisms (e.g., bacteria, fungi, plant, or animal cell), causing the excision of the repressor elements and allowing for induction of transcription of polynucleotide sequences.

*Guide RNAs*

Guide RNAs ("gRNAs") are described herein that target the phenotype coding sequences or the endogenous gene or genomic region to be modified. In general, a gRNA is an RNA sequence having sufficient complementarity with a target polynucleotide sequence to hybridize with the target sequence and direct sequence-specific binding of a CRISPR complex to the target sequence. The degree of complementarity between a gRNA and its corresponding target sequence, when optimally aligned using a suitable alignment algorithm, is about or more than about 50%, 60%, 75%, 80%, 85%, 90%, 95%, 97.5%, 99%, or more. A distinct gRNA is provided for each of the phenotype coding sequences and/or endogenous genes or genomic regions to be modified. Thus, an engineered organism or engineered cell comprising a first phenotype coding sequence and a second phenotype coding sequence may further comprise a polynucleotide sequence that comprises a first gRNA that targets the first phenotype coding sequence and a polynucleotide sequence that comprises a second gRNA that targets the second phenotype coding sequence.

A gRNA may comprise a sequence of at least about 15, 16, 17, 18, 19, 20, 21, 22, 23, 24, or 25 nucleotides in length (e.g., about 18-22 nucleotides in length or about 18-20 nucleotides in length) that is complementary to a portion of a phenotype coding sequence and may further comprise a sequence that binds the Cas9 nuclease. A gRNA may comprise a nucleotide sequence that is complementary to a portion of a phenotype coding sequence that is adjacent to a Protospacer Adjacent Motif (PAM) sequence. A gRNA may further comprise a homologous donor polynucleotide sequence. The homologous donor polynucleotide sequence may comprise a restriction enzyme recognition site. The homologous donor polynucleotide sequence may have a length of at least 8 nucleotides, at least 10, 15, 20, 25, 50, 100, 200, 300, 400, 500, 600, 700, 800, 900, or 1000 nucleotides or more. Further, the homologous donor polynucleotide sequence may have two arms with identical or substantially identical sequences adjacent to the gRNA target site with a specific mutation in between the two arms. The arms may be 20-1000 nucleotides or more and the mutation may be 1-1000 nucleotides or more.

Methods of designing gRNAs to target a sequence of interest and methods of optimizing gRNA structure to improve genomic editing efficiency are known in the art (see, e.g., Dang et al., Genome Biology, 2015, 16:280; Lee et al., Exp Physiol, 2017, doi: 10.1113/EP08604). A gRNA may be selected to reduce the degree of secondary structure within the gRNA. Secondary structure may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A. R. Gruber et al., 2008, Cell 106(1): 23-24; and PA Carr and GM Church, 2009, Nature Biotechnology 27(12): 1151-62).

The polynucleotide sequence comprising a gRNA that targets a phenotype coding sequence may further comprise a heterologous promoter. The promoter may be a constitutively active promoter. Suitable constitutively active promoters include Tyrosine tRNA, Sigma 70 consensus sequence, TEF1, RPL18B, RNR2, TDH3, REV1, PGK, and ADH1.

The polynucleotide sequence comprising a gRNA that targets a phenotype coding sequence may further comprise a heterologous promoter. The promoter may be inducible. Suitable inducible promoters include arabinose-inducible (pBAD), galactose-inducible (GAL1), or rhamnose-inducible (pRha).

The polynucleotide sequence comprising a gRNA that targets a phenotype coding sequence may further comprise a sequence for an auxotrophic marker. Each polynucleotide sequence comprising a gRNA may further comprise a sequence for an auxotrophic marker, wherein each gRNA has a different auxotrophic marker. The auxotrophic marker may be a yeast auxotrophic marker. Examples of auxotrophic markers include LEU2, URA3, LYS2 HIS3, MET17, and TRP1.

The polynucleotide sequence comprising a gRNA that targets a phenotype coding sequence may further comprise a sequence for an antibiotic resistance marker., Each polynucleotide sequence comprising a gRNA may further comprise a sequence for an antibiotic resistance marker, wherein each gRNA has a different antibiotic resistance marker. Examples of antibiotic resistance markers include Ampicillin, Chloramphenicol, Kanamycin, Streptomycin, Tetracycline, Spectinomycin, Gentamycin, and Zeocin.

Each polynucleotide comprising a gRNA as described herein (e.g., a polynucleotide comprising a gRNA and further comprising a promoter and/or an auxotrophic or antibiotic marker) may be in a separate expression cassette, expression vector, or plasmid than the other polynucleotide sequences disclosed herein (e.g., polynucleotides comprising other gRNA, polynucleotides comprising a phenotype coding sequence or polynucleotides encoding a guide RNA).

### Components and Modifications for Alternative End-Joining and/or Non-Homologous End-Joining

Cells or organisms (e.g., bacteria) may be engineered to express proteins that are components of the non-homologous end-joining (NHEJ) system. For example, bacteria may be genetically engineered to express *Mycobacterium tuberculosis* Ku and Ligase D (LigD) proteins. These proteins allow bacteria to perform NHEJ, as described in Malyarchuk et al., DNA Repair, 2007, 6:1413-1424.

For example, wild-type or genetically altered (e.g., Ku and LigD-expressing) bacteria that express chromogenic proteins or antibiotic resistance markers will have one or more of these coding sequences targeted and cleaved by Cas9. DSBs can be repaired through NHEJ, which is prone to error and will result in the loss of function of the targeted coding sequence. Changes in the frequency of loss of function of one or more coding sequences in wild-type versus genetically altered bacteria can be determined, e.g., in accordance with a method, system, or kit as disclosed herein.

Cells or organisms (e.g., bacteria) may be engineered to express or overexpress proteins that are components of the alternative end-joining (A-EJ) system. For example, bacteria may be genetically engineered to express, or to express at higher levels, the components RecBCD and LigA. RecBCD is a nuclease/helicase complex that unwinds and degrades the DNA ends and is made up of three polypeptides: RecB, RecC, and RecD. LigA is DNA ligase that is responsible for alternative end-joining. An overview of the alternative end-joining repair mechanism and components is described, e.g., in Chayot et al., PNAS, 2010, 1017:2141-2146.

### IV. Methods of Altering Gene Expression

In the fourth aspect, methods of altering gene expression are provided. The method comprises culturing an engineered microbial organism cell or microbial organism as described herein (e.g., an engineered microbial organism cell such as a yeast cell or an engineered microbial organism such as yeast) to form a population of engineered microbial organism cells or engineered microbial organisms, wherein the culturing is performed under conditions that result in expression of the Cas nuclease in at least one engineered microbial organism cell or engineered microbial organism, wherein the Cas nuclease cleaves an endogenous gene, genomic region, or phenotype coding sequence; thereby altering gene expression in at least one engineered microbial organism cell or engineered microbial organism.

The method of altering gene expression comprises:

providing a microbial organism cell or microbial organism as described herein (e.g., a prokaryotic or eukaryotic cell such as a yeast cell, or a microbial organism or eukaryotic organism as described herein);
transforming the microbial organism cell or microbial organism with one or more polynucleotide sequences as described herein (e.g., a first heterologous polynucleotide sequence comprising a first phenotype coding sequence operably linked to a first promoter, a second heterologous polynucleotide sequence comprising a second phenotype coding sequence operably linked to a second promoter, a third heterologous polynucleotide sequence comprising a third promoter operably linked to a polynucleotide encoding a Cas nuclease, a fourth heterologous polynucleotide sequence comprising a first gRNA that targets the first phenotype coding sequence, and a fifth heterologous polynucleotide sequence comprising a second gRNA that targets the second phenotype coding sequence) to express the polynucleotide sequences; and
culturing the transformed microbial organism cell or microbial organism to form a population of cells or organisms, wherein the culturing is performed under conditions that result in expression of the Cas nuclease in at least one cell or organism, wherein the Cas nuclease cleaves a phenotype coding sequence (e.g., one or more of the first phenotype coding sequence and the second phenotype coding sequence); thereby altering gene expression in at least one cell or organism.

In particular, when the promoter that is operably linked to the polynucleotide encoding the Cas nuclease (e.g., Cas9 nuclease) is an inducible promoter, the culturing may be in the presence of an inducer to induce expression of the Cas nuclease in at least one engineered cell or organism. The polynucleotide encoding Cas nuclease may be operably linked to a galactose-inducible promoter and the method may comprise culturing the engineered cells or organisms in the presence of galactose to induce the expression of Cas. The polynucleotide encoding Cas nuclease may be operably linked to an arabinose-inducible promoter and the method may comprise culturing the engineered cells or organisms in the presence of arabinose to induce the expression of Cas. The polynucleotide encoding Cas nuclease may be operably linked to a rhamnose-inducible promoter and the method may comprise culturing the engineered microbial organism cells or microbial organisms in the presence of rhamnose to induce the expression of Cas.

In particular, when a polynucleotide comprising a gRNA is operably linked to a promoter that is an inducible promoter, the culturing may be in the presence of an inducer to induce expression of the gRNA in at least one engineered microbial organism cell or microbial organism. The polynucleotide comprising the gRNA may be operably linked to a galactose-inducible promoter and the method may comprise culturing the engineered microbial organism cells or microbial organisms in the presence of galactose to induce the expression of the gRNA. The polynucleotide comprising the gRNA may be operably linked to an arabinose-inducible promoter and the method may comprise culturing the engineered cells or organisms in the presence of arabinose to induce the expression of the gRNA. The polynucleotide comprising the gRNA may be operably linked to a rhamnose-inducible promoter and the method may comprise culturing the engineered cells or organisms in the presence of rhamnose to induce the expression of the gRNA. The polynucleotide comprising the gRNA may be operably linked to a constitutively active promoter, and the gRNA may be constitutively expressed.

Means of transforming microbial organism cells and microbial organisms are known in the art. Transforming the microbial organism cell or microbial organism with the polynucleotide sequences as described herein (e.g., donor DNA, gRNA, phenotype coding sequence polynucleotides, Cas nuclease polynucleotides or expression cassettes, or lambda red polynucleotides or expression cassettes) may comprise delivering the polynucleotide sequences through chemical transformation of plasmids or electroporation. Transforming the cell or organism with the polynucleotide sequences as described herein (e.g., donor DNA, gRNA, phenotype coding sequence polynucleotides, Cas nuclease polynucleotides or expression cassettes, or lambda red polynucleotides or expression cassettes) may comprise delivering the polynucleotide sequences through transformation by silicon-carbide whiskers.

The method may further comprise screening the population of engineered microbial organism cells or microbial organisms to identify at least one engineered microbial organism cell that exhibits a change in phenotype as compared to the phenotype of the engineered microbial organism cell or microbial organism prior to the culturing step. In particular, the method may further comprise screening the population of engineered microbial organism cells or engineered microbial organisms to identify at least one engineered microbial organism cell or engineered microbial organism that exhibits a color change as compared to the color of the engineered microbial organism cell or engineered microbial organism prior to the culturing step. For example, as detailed in Example 1 below, the engineered microbial organism cell or engineered microbial organism may comprise chromogenic coding sequences for blue, red, and yellow genes. In the absence of Cas (e.g., Cas9) nuclease expression, each of these chromogenic proteins are expressed, causing the cell or organism to have a brown phenotype. In the presence of an inducer (e.g., galactose), one or more of the chromogenic coding sequences is disrupted due to the activity of Cas, which results in a loss of expression of one or more of the chromogenic proteins and changes the color phenotype of the cell or organism.

The method of altering gene expression may comprise disrupting expression of a detectable phenotype as described herein (e.g., disrupting expression of a chromogenic protein). The method of altering gene expression may comprise restoring expression of a detectable phenotype by repairing a phenotype coding expression to permit expression of a functional protein having a detectable phenotype. In some embodiments, the method of altering gene expression comprises a combination of disrupting expression of one detectable phenotype and restoring expression of another detectable phenotype.

A first phenotype coding sequence may encode a functional first protein having a first detectable phenotype and the Cas (e.g., Cas9) nuclease may cleave the first phenotype coding sequence and may disrupt expression of the first protein. A first phenotype sequence may comprise an engineered disruption in a sequence encoding a first protein that prevents expression of the first detectable phenotype, and the method may comprise cleaving the first coding sequence at the engineered disruption with the Cas (e.g., Cas9) nuclease and repairing the first coding sequence to permit expression of the first protein.

A second phenotype coding sequence may encode a functional second protein having a second detectable phenotype and the Cas (e.g., Cas9) nuclease may cleave the second phenotype coding sequence and may disrupt expression of the second protein. A second phenotype sequence may comprise an engineered disruption in a sequence encoding a second protein that prevents expression of the second detectable phenotype, and the method may comprise cleaving the second coding sequence at the engineered disruption with the Cas (e.g., Cas9) nuclease and repairing the second coding sequence to permit expression of the second protein.

The first phenotype coding sequence may encode a functional first protein having a first detectable phenotype and the method may comprise cleaving the first phenotype coding sequence with the Cas (e.g., Cas9) nuclease and disrupting expression of the first protein; and the second phenotype sequence may comprise an engineered disruption in a sequence encoding a second protein that prevents expression of the second detectable phenotype, and the method may comprise cleaving the second coding sequence at the engineered disruption with the Cas (e.g., Cas9) nuclease and repairing the second coding sequence to permit expression of the second protein.

The first phenotype sequence may comprise an engineered disruption in a sequence encoding a first protein that prevents expression of the first detectable phenotype, and the method may comprise cleaving the first coding sequence at the engineered disruption with the Cas (e.g., Cas9) nuclease and repairing the first coding sequence to permit expression of the first protein; and the second phenotype coding sequence may encode a functional second protein having a second detectable phenotype and the method may comprise cleaving the second phenotype coding sequence with the Cas (e.g., Cas9) nuclease and disrupting expression of the second protein.

In particular, e.g., as shown in Example 7 or Example 9 below, the engineered microbial organism cell or microbial organism may comprise a phenotype coding sequence encoding a functional first protein having a detectable phenotype (e.g., a GFP protein), and the method may comprise cleaving the coding sequence with the Cas (e.g., Cas9) nuclease, thereby disrupting expression of the first protein. The engineered disruption is defined by the sequence in the homologous repair donor sequence.

In particular, e.g., as shown in Example 8 or Example 10, the engineered microbial organism cell or microbial organism may comprise a phenotype coding sequence encoding a functional first protein having a detectable phenotype (e.g., a GFP protein), and the method may comprise cleaving the coding sequence with the Cas (e.g., Cas9) nuclease and may modify the expression of the first protein, e.g., to instead express a second phenotype (e.g., YFP or BFP). The engineered changes are defined by the sequence in the homologous repair donor sequence.

*Counter selection with Auxotrophic Markers*

Furthermore, the microbial organism cell or microbial organism may comprise one or more polynucleotide sequences that comprises an auxotrophic marker, and the method may further comprise: culturing the population of engineered microbial organism cells or microbial organisms in the presence of one or more counterselection agents for the auxotrophic marker(s); and selecting for one or more engineered microbial organism cells or organisms that do not express the polynucleotide sequence associated with the auxotrophic marker being counterselected; thereby preventing expression of the gRNA associated with the auxotrophic marker being counterselected and preventing alteration of the chromogenic coding sequence targeted by the gRNA associated with the auxotrophic marker being counterselected.

As an example, a microbial organism comprising a plasmid that comprises a first gRNA that targets a first chromogenic coding sequence and the URA3 auxotrophic marker can be counterselected by culturing the organism in the presence of 5-FOA (e.g., by growing the microbial organism on media containing 5-FOA). As a result, the first gRNA will not be expressed and the first chromogenic coding sequence will not be targeted for gene editing. A microbial organism comprising a plasmid that comprises a second gRNA that targets a second chromogenic coding sequence and the LYS2 auxotrophic marker can be counterselected by culturing the organism in the presence of alpha-adipic acid (e.g., growing the organism on media containing alpha-adipic acid). As a result, the second gRNA will not be expressed and the second chromogenic coding sequence will not be targeted for gene editing.

*Selection with Antibiotic Resistance Markers*

Furthermore, the microbial organism cell or microbial organism comprises one or more polynucleotide sequences that comprises an antibiotic resistance marker, and the method further comprises culturing the population of engineered microbial organism cells or microbial organisms in the presence of one or more selection agents for the antibiotic resistance marker(s).

*Lambda Red Recombineering*

Furthermore, the methods of altering gene expression comprise the use of the lambda red system for targeting and replacing a gene or genomic region (e.g., a phenotype coding region as disclosed herein that is introduced into the microbial organism cell or microbial organism, or an endogenous gene or region within the genome of the cell or organism). Lambda red is a genetic engineering tool that enables homologous recombination in bacteria ("recombineering"). The lambda red system, which is derived from lambda bacteriophage, has three components: (1) lambda exonuclease (Exo), which digests the 5' ended strand of dsDNA; (2) beta protein (Beta), which binds to ssDNA and enables strand annealing; and (3) gamma protein (Gam), which binds to the bacterial RecBCD enzyme and inhibits it from digesting linear DNA introduced into *E. coli.* The lambda red system is described in the art (see, e.g., Pyne et al., Applied and Environmental Microbiology, 2015, 81:5103-5114).

The method of altering gene expression comprises:

providing a microbial organism cell or microbial organism as described herein (e.g., a prokaryotic or eukaryotic cell such as a yeast cell, or a microbial organism or eukaryotic organism as described herein);

transforming the microbial organism cell or microbial organism with one or more polynucleotide sequences as described herein (e.g., a heterologous polynucleotide sequence comprising a phenotype coding sequence operably linked to a promoter, a heterologous polynucleotide sequence comprising a promoter operably linked to a polynucleotide encoding lambda red, and a heterologous polynucleotide sequence comprising a homologous donor DNA sequence); and

culturing the transformed microbial organism cell or microbial organism to form a population of microbial organism cells or microbial organisms, wherein the culturing is performed under conditions that result in expression of the lambda red in at least one microbial organism cell or microbial organism, wherein the lambda red catalyzes the homologous recombination of a donor DNA sequence at an endogenous gene, genomic region, or phenotype coding sequence in the engineered microbial organism cell; thereby altering gene expression in at least one microbial organism cell or microbial organism.

In particular, when the promoter that is operably linked to the polynucleotide encoding the lambda red is an inducible promoter, the culturing is in the presence of an inducer to induce expression of the lambda red in at least one engineered cell or organism. The polynucleotide encoding lambda red may be operably linked to a galactose-inducible promoter and the method comprises culturing the engineered microbial organism cells or microbial organisms in the presence of galactose to induce the expression of lambda red. Further, the polynucleotide encoding lambda red may be operably linked to an arabinose-inducible promoter and the method comprises culturing the engineered microbial organism cells or microbial organisms in the presence of arabinose to induce the expression of lambda red. The polynucleotide encoding lambda red nuclease may be operably linked to a rhamnose-inducible promoter and the method comprises culturing the engineered microbial organism cells or microbial organisms in the presence of rhamnose to induce the expression of lambda red.

The methods disclosed herein comprise the use of lambda red recombineering with or without the CRISPR/Cas system. In particular, the methods disclosed herein comprise the use of the CRISPR/Cas system with lambda red recombineering. Without being bound to a particular theory, it is expected that inclusion of homologous recombination by lambda red will increase the efficiency of CRISPR/Cas9-mediated gene editing in bacterial cells. Thus, a method or module for altering gene expression comprises using lambda red recombineering in both the absence and presence of the CRISPR/Cas system (e.g., Cas9 nuclease and gRNA components as disclosed herein) and comparing the rate of gene targeting in the absence of Cas9 nuclease and gRNA to the rate of gene targeting in the presence of Cas9 nuclease and gRNA.

*Targeting Endogenous or Genomic Targets*

The methods of altering gene expression comprise targeting an endogenous phenotype or a genomic locus (e.g., a gene or a genomic region of the cell or organism). For example, the CRISPR/Cas system and/or lambda red recombineering system is used in an engineered microbial organism cell or microbial organism as disclosed herein in order to target an endogenous gene or genomic region in the genome of the microbial organism cell or microbial organism. In particular, the targeting comprises disrupting the function of an endogenous gene or genomic region in the genome of the microbial organism cell or microbial organism, e.g., by introducing a polynucleotide sequence that comprises a stop codon or other sequence that prevents the expression of a functional protein, or by introducing a polynucleotide sequence that comprises an open reading frame for a different gene in order to change the function of the endogenous gene. The targeting comprises restoring the function of a disrupted endogenous gene or genomic region in the genome of the microbial organism cell or microbial organism, e.g., by introducing a polynucleotide sequence that repairs a broken gene or genomic region (e.g., promoter) or a prematurely terminated gene or genomic region in order to restore the native function of the gene or genomic region.

The method of altering gene expression comprises:

providing a microbial organism cell or microbial organism as described herein (e.g., a prokaryotic or eukaryotic cell such as a yeast cell, or a microbial organism or eukaryotic organism as described herein);

transforming the microbial organism cell or microbial organism with one or more polynucleotide sequences as described herein (e.g., a heterologous polynucleotide sequence comprising a promoter operably linked to a polynucleotide encoding a Cas nuclease, a heterologous polynucleotide sequence comprising a promoter operably linked to a polynucleotide encoding lambda red, one or more heterologous polynucleotide sequences comprising a gRNA that targets the endogenous gene or genomic region, and/or one or more donor DNAs) to express the polynucleotide sequences; and

culturing the transformed microbial organism cell or microbial organism to form a population of cells or organisms, wherein the culturing is performed under conditions that result in expression of the Cas nuclease in at least one microbial organism cell or microbial organism, wherein the Cas nuclease cleaves at the endogenous gene or genomic region; thereby altering gene expression in at least one microbial organism cell or microbial organism.

In particular, the method of altering gene expression comprises targeting an endogenous gene or genomic region that is functional (e.g., exhibits a detectable phenotype, such as a detectable color, fluorescence, scent, enzymatic activity, antibiotic resistance, or morphology) and transforming the microbial organism cell or microbial organism with a donor DNA sequence that disrupts the endogenous gene or genomic region (e.g., a sequence that results in a premature termination codon), thereby altering gene expression in the microbial organism cell or microbial organism.

Further, the method of altering gene expression comprises targeting an endogenous gene or genomic region that is functional (e.g., exhibits a detectable phenotype, such as a detectable color, fluorescence, scent, enzymatic activity, antibiotic resistance, or morphology) and transforming the microbial organism cell or microbial organism with a donor DNA sequence that encodes a different function (e.g., a sequence comprising an ORF for a different gene, such as an antibiotic resistance cassette) to replace the endogenous function of the targeted gene or genomic region with a new function, thereby altering gene expression in the microbial organism cell or microbial organism.

Further, the method of altering gene expression comprises targeting an endogenous gene or genomic region that is non-functional (e.g., due to the presence of a polynucleotide sequence that results in a premature termination codon) and transforming the microbial organism cell or microbial organism with a donor DNA sequence that restores the function of the gene or genomic region, thereby altering gene expression in the cell or organism.

**V. Kits**

In the third aspect, kits comprising the polynucleotides, expression cassettes, expression vectors, plasmids, and/or microbial organism cells as described herein are provided. In particular, the kit comprises:

one or more polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype;

a polynucleotide sequence comprising a promoter operably linked to a polynucleotide encoding a Cas nuclease; and

a polynucleotide sequence comprising a guide RNA (gRNA) that targets the phenotype coding sequence.

The kit may further comprise one or more polynucleotide sequences comprising a homologous donor DNA sequence (e.g., dsDNA or ssDNA donor sequence).

In particular, the kit comprises:

a first polynucleotide sequence comprising a first chromogenic coding sequence operably linked to a first promoter, wherein the first chromogenic coding sequence either (i) encodes a functional first chromogenic protein or (ii) comprises an engineered disruption in a sequence encoding a first chromogenic protein that prevents expression of the first chromogenic protein;

a second polynucleotide sequence comprising a second chromogenic coding sequence operably linked to a second promoter, wherein the second chromogenic coding sequence either (i) encodes a functional second chromogenic protein or (ii) comprises an engineered disruption in a sequence encoding a second chromogenic protein that prevents expression of the second chromogenic protein;

a third polynucleotide sequence comprising an inducible promoter operably linked to a polynucleotide encoding a Cas nuclease;

a fourth polynucleotide sequence comprising a first guide RNA (gRNA) that targets the first chromogenic coding sequence; and

a fifth polynucleotide sequence comprising a second gRNA that targets the second chromogenic coding sequence.

Furthermore, the kit comprises:

an engineered microbial organism cell or microbial organism comprising an endogenous gene or genomic region to be targeted for gene alteration, or one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype; and a heterologous polynucleotide sequence comprising a promoter operably linked to a polynucleotide encoding a Cas nuclease and a heterologous polynucleotide sequence comprising a guide RNA (gRNA) that targets the endogenous gene, genomic region, or phenotype coding sequence; and/or a heterologous polynucleotide sequence comprising a promoter operably linked to a polynucleotide encoding lambda red.

Furthermore, the kit may comprise an engineered microbial organism cell or microbial organism comprising an endogenous gene or genomic region to be targeted for gene alteration, or one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype, and further comprising one or both of a Cas nuclease and a lambda red system integrated into the genome of the engineered cell or organism. The engineered cell or organism may comprise one of the Cas nuclease or the lambda red system integrated into the genome of the engineered cell or organism, and the kit may further comprise a heterologous polynucleotide sequence encoding the other of the Cas nuclease or the lambda red system. In some embodiments, the kit further comprises one or more polynucleotide sequences comprising a homologous donor DNA sequence (e.g., dsDNA or ssDNA donor sequence).

The kit may comprise:

an engineered microbial organism cell or microbial organism comprising one or more heterologous polynucleotide sequences comprising a phenotype coding sequence operably linked to a promoter, wherein the phenotype coding sequence either (i) encodes a functional protein having a detectable phenotype, or (ii) comprises an engineered disruption in a sequence encoding a protein that prevents expression of the detectable phenotype; and

one or more of a heterologous polynucleotide sequence comprising a promoter operably linked to a polynucleotide encoding a Cas nuclease, a heterologous polynucleotide sequence comprising a gRNA that targets the phenotype coding sequence, and a heterologous polynucleotide sequence comprising a homologous donor DNA sequence.

The kit may be for use in targeting an endogenous phenotype or a genomic locus of an engineered cell or organism and may comprise:

an engineered microbial organism cell or microbial organism (e.g., a prokaryotic or eukaryotic cell such as a yeast cell, or a microbial organism or eukaryotic organism as described herein) that comprises an endogenous gene or genomic region to be targeted;

a polynucleotide sequence comprising a promoter operably linked to a polynucleotide encoding a Cas nuclease;

a polynucleotide sequence comprising a gRNA that targets the endogenous gene or genomic region of the engineered microbial organism cell or microbial organism; and

one or more polynucleotide sequences comprising a homologous donor DNA sequence (e.g., dsDNA or ssDNA donor sequence).

In the kits disclosed herein, the kit may further comprise a heterologous polynucleotide sequence comprising a promoter operably linked to a polynucleotide encoding lambda red.

A kit that is for use in targeting an endogenous phenotype or a genomic locus of an engineered microbial organism cell or microbial organism may comprise an engineered microbial organism cell or microbial organism organism that comprises an endogenous gene or genomic region to be targeted and one or both of a Cas nuclease and a lambda red system integrated into the genome of the engineered microbial organism cell or microbial organism. The engineered microbial organism cell or microbial organism organism may comprise one of the Cas nuclease or the lambda red system integrated into the genome of the engineered cell or organism, and the kit may further comprise a heterologous polynucleotide sequence encoding the other of the Cas nuclease or the lambda red system. The kit may further comprise one or more polynucleotide sequences comprising a homologous donor DNA sequence (e.g., dsDNA or ssDNA donor sequence).

The kit may further comprise one or more reagents for transforming the one or more heterologous polynucleotide sequences into the engineered microbial organism cell or microbial organism. Moreover, the kit may further comprise one or more reagents for inducing expression of the Cas nuclease, inducing expression of the gRNA, or preventing expression of the gRNA.

The polynucleotide sequences (e.g., as described in Section III above) may be in one or more expression cassettes, expression vectors, and/or plasmids. The kit may further comprise one or more cells or organisms (e.g., microbial, prokaryotic, protist, or eukaryotic cells or organisms as described herein, e.g., as in Section III above). The kit may comprise a prokaryotic cell, or the kit may comprise a protist cell, or the kit may comprise a eukaryotic cell.

The polynucleotide sequences, expression cassettes, expression vectors, and/or plasmids may be in a microbial organism cell or microbial organism as described herein. Thus, in the third aspect, kits are provided that comprise an engineered microbial organism cell or engineered microbial organism as described herein. The engineered microbial organism may be fungal (e.g., yeast), the engineered microbial organism may be prokaryotic (e.g., bacterial), or the engineered microbial organism may be eukaryotic (e.g., yeast).

The microbial organism, polynucleotide sequence, expression cassette, expression vector, and/or plasmid that is provided in the kit may be in lyophilized form. In particular, the lyophilized microbial organism, polynucleotide sequence, expression cassette, expression vector, and/or plasmid is reconstituted by the end user of the kit. Thus, the kit may further comprise instructions for reconstituting the lyophilized microbial organism, polynucleotide sequence, expression cassette, expression vector, and/or plasmid.

The microbial organism and polynucleotide sequences, expression cassettes, expression vectors, and/or plasmids may be provided separately in the kit. In particular, a microbial organism and polynucleotide sequences, expression cassettes, expression vectors, and/or plasmids may be provided separately in the kit in lyophilized form.

The kit may further comprise one or more culturing reagents. In particular, the kit may further comprise one or more transfection reagents. The kit may further comprise one or more transformation reagents. The kit may comprise one or more reagents comprising culture medium (e.g., liquid medium), selective medium, solid plating medium, media supplements, plates, tubes, loops, or other plasticware. The kit may further comprise an inducer for the inducible promoter. The kit may comprise galactose. The kit may comprise arabinose. The kit may comprise rhamnose. The kit may comprise counterselection agents for the auxotrophic markers as described herein (e.g., 5-fluoro-orotic acid or alpha-adipic acid). The kit may comprise selection agents for the antibiotic resistance markers as described herein (e.g., Ampicillin, Kanamycin, Spectinomycin, Gentamycin, or Zeocin).

The kit may further comprise one or more reagents for detecting the genotype of the engineered microbial organism cell or engineered microbial organism, wherein the one or more reagents comprise DNA polymerases, primers, dNTPs, restriction enzymes, or buffers. The kit may further comprise equipment for transforming, culturing, detecting phenotypes, or genotyping a microbial organism cell or microbial organism. For example, the kit may comprise antibodies, western detection reagents, protein transfer equipment, pipets, pipettors, incubators, incubator shakers, thermal cyclers, DNA electrophoresis equipment, power supplies, waterbaths, sequencing materials, or bioinformatics software.

The kit may further comprise instruction materials containing directions (i.e., protocols) for the practice of the methods described herein (e.g., for altering gene expression in a cell or organism as described herein). Furthermore, the kit may comprise instructions for analyzing a microbial organism cell or microbial organism as described herein (e.g., for inducing Cas9 activity, detecting one more changes in a detectable phenotype, and/or analyzing a genotype for a cell or organism as described herein). The kit may comprise instructions for designing gRNAs and other CRISPR/Cas components as described herein. The kit may comprise a curriculum (e.g., a curriculum for educators) for using the microbial organism cells, compositions, kits, and/or methods as disclosed herein for teaching fundamental principles in gene editing.

While the instructional materials typically comprise written or printed materials they are not limited to such. Any medium capable of storing such instructions and communicating them to an end user is contemplated for it. Such media include electronic storage media (e.g., magnetic discs, tapes, chips), optical media (e.g., CD-ROM). Such media may also include addresses to internet sites that provide such instructional materials.

## VI. Systems and Methods for Education and Training

In the fourth aspect, systems and methods for education and training in the core processes of gene expression and gene editing are provided. In particular, the polynucleotide sequences, expression cassettes, expression vectors, plasmids, cells, organisms, and reagents disclosed herein are provided as a modular system. The system can be formulated as a series of modules that collectively represent the various stages in gene expression and alteration of gene expression in a cell or organism, with each stage or module containing a full set of reagents, media, and other disposable components, as well as an instruction manual with directions for performing standard procedures for each stage, and an assessment module for evaluating the users' understanding of the processes taught. Certain modules can be sold or purchased independently of the system as a whole, such as for the purposes of replenishing a supply for a particular stage, offering the instructor or trainee a choice at the point of use, or adapting the system to smaller or larger numbers of trainees.

For example, the system can comprise one or more of the following modules.

Module for expressing a detectable phenotype in a microbial organism cell or microbial organism: A module for expressing one or more detectable phenotypes (e.g., detectable colors and/or fluorescences; detectable scents; detectable enzymatic activities; resistance to an antibiotic; or detectable morphologies) in a microbial organism cell or microbial organism is provided. The module may comprise a set of reagents and materials for providing, generating, or culturing a cell or organism that expresses a detectable phenotype. Further, the module may comprise one or more of a microbial organism cell or microbial organism (e.g., a prokaryotic or eukaryotic cell or organism), one or more heterologous polynucleotides comprising a phenotype coding sequence operably linked to a promoter, a heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the chromogenic coding sequence, and a heterologous polynucleotide sequence comprising a homologous donor DNA sequence, as described herein. Further, the module may comprise a cell or organism comprising one or more heterologous polynucleotides comprising a phenotype coding sequence operably linked to a promoter, and further may comprise one or more heterologous polynucleotides (e.g., heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the chromogenic coding sequence, and a heterologous polynucleotide sequence comprising a homologous donor DNA sequence) for transforming into the microbial organism cell or microbial organism. In some embodiments, the module comprises or more culturing reagents, transfection reagents, and/or transformation reagents.

Module for expressing a broken gene in a cell or organism: A module for expressing one or more phenotype coding sequences comprising an engineered disruption in a protein-encoding sequence or promoter region that prevents expression of a detectable phenotype (e.g., a broken gene such as a broken chromogenic gene) in a microbial organism cell or microbial organism is provided. The module may comprise a set of reagents and materials for providing, generating, or culturing a microbial organism cell or microbial organism having a broken gene. The module may comprise one or more of a microbial organism cell or microbial organism (e.g., a prokaryotic or eukaryotic cell or organism), one or more heterologous polynucleotides operably linked to a promoter, wherein the polynucleotide comprises an engineered disruption in a sequence encoding a protein that prevents expression of the detectable phenotype, a heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the chromogenic coding sequence, and a heterologous polynucleotide sequence comprising a homologous donor DNA sequence, as described herein. The module may comprise a c microbial organism ell or microbial organism comprising one or more heterologous polynucleotides comprising an engineered disruption in a sequence encoding a protein or promoter region that prevents expression of a detectable phenotype (e.g., a broken gene such as a broken chromogenic gene), and may further comprise one or more heterologous polynucleotides (e.g., heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the chromogenic coding sequence, and a heterologous polynucleotide sequence comprising a homologous donor DNA sequence) for transforming into the microbial organism cell or microbial organism. The module may comprise or more culturing reagents, transfection reagents, and/or transformation reagents.

Module for gene editing: A module for altering expression of a detectable phenotype (e.g., to change the detectable phenotype into a second detectable phenotype or to disrupt expression of the detectable phenotype) or for repairing a broken gene (e.g., repairing a broken gene such that a detectable phenotype is expressed) is provided. The module may comprise a set of reagents and materials for altering and/or repairing gene expression. The module may comprise a heterologous polynucleotide sequence comprising a promoter (e.g., an inducible promoter) operably linked

28

to a polynucleotide encoding a Cas nuclease, a heterologous polynucleotide sequence comprising a promoter operably linked to a polynucleotide encoding lambda red, a heterologous polynucleotide sequence comprising a gRNA that targets the chromogenic coding sequence, and/or a heterologous polynucleotide sequence comprising a homologous donor DNA sequence. The module may comprise one or more reagents for inducing expression of a Cas nuclease and/or the lambda red system or for inducing or preventing the expression of a gRNA, e.g., an inducer for an inducible promoter or a selection agent or counterselection agent for an antibiotic or auxotrophic marker. Further, the module may comprise or more culturing reagents, transfection reagents, and/or transformation reagents.

Module for endogenous or genomic targeting: A module for targeting an endogenous gene or genomic region in a microbial organism cell or microbial organism (e.g., disrupting the function of the endogenous gene or genomic region, replacing the function of the endogenous gene or genomic region with a new function, or restoring the function of the endogenous gene or genomic region) is provided. The module may comprise a set of reagents and materials for providing, generating, or culturing a microbial organism cell or microbial organism that expresses an endogenous gene or genomic region to be targeted. The module may comprise one or more of a c microbial organism ell or microbial organism (e.g., a prokaryotic or eukaryotic cell or organism) comprising an endogenous gene or genomic region to be targeted, a heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the endogenous gene or genomic region, and a heterologous polynucleotide sequence comprising a homologous donor DNA sequence, as described herein. The module may comprise a microbial organism cell or microbial organism comprising an endogenous gene or genomic region to be targeted, and further comprises one or more heterologous polynucleotides (e.g., heterologous polynucleotide sequence comprising a promoter operably linked to a polynucleotide encoding a Cas nuclease, a heterologous polynucleotide sequence comprising a promoter operably linked to a polynucleotide encoding lambda red, a heterologous polynucleotide sequence comprising a gRNA that targets the endogenous gene or genomic region, and a heterologous polynucleotide sequence comprising a homologous donor DNA sequence) for transforming into the microbial organism cell or microbial organism. Further, the module may comprise one or more culturing reagents, transfection reagents, and/or transformation reagents.

Module for analyzing the microbial organism cell or microbial organism: A module for detecting a phenotype, a change in phenotype, and/or a genotype of a microbial organism cell or microbial organism following a gene editing event is provided. The module may comprise a set of reagents and materials for detecting or analyzing a phenotype or genotype. For example, the module may comprise one or more reagents for detecting the genotype of an engineered microbial organism cell or engineered microbial organism, e.g., DNA polymerases, primers, dNTPs, restriction enzymes, or buffers. The module may comprise equipment for transforming, culturing, detecting phenotypes, or genotyping an engineered cell or organism.

Assessment module: An assessment module containing materials for use by educators or trainers can also be included or otherwise made available. The assessment module might serve the purpose of aiding educators or trainers in evaluating a trainee's understanding of the purpose and use of the materials and procedures, and in identifying particular areas in which the trainee needs improvement or needs to pay greater attention to details such as timing, operating conditions, proper use of equipment, or other aspects of the procedures.

The system may comprise an instruction manual containing the entire procedure, including all steps to be followed for each module and procedures for using each piece of equipment and each reagent in each module, as well as the results to be expected from each procedure when the procedures are performed correctly.

## VII. Examples

The following examples are provided to illustrate the claimed invention.

### Example 1: Kaleidoscope Organism Containing Multiple Color Genes

The organism can contain and express multiple color genes. Depending on which of these genes are knocked out, the organism's phenotype will change colors. As shown in FIG. 1, an organism (e.g., *E. coli* or yeast) contains multiple color genes, and the genes necessary for targeted knock out of each of these genes (e.g., Cas9 system with color gene specific guide RNAs). These genetic elements can be chromosomal or on plasmid(s). In the case of three color genes (e.g., Red, Blue and Yellow), the organism will be brownish when all three genes are expressed. If the Cas9 system and one or more of the gRNA's are expressed then the color of the organism will change due to the knock-out of one or more of the color genes. For example:

Color Genes A + B + C expressed = Brown Phenotype (blue, red and yellow genes expressed)

Color Genes A + B + C expressed + Cas9 expressed + gRNA A expressed → Green Phenotype (blue and yellow genes expressed)

Color Genes A + B + C expressed + Cas9 expressed + gRNA B expressed → Orange Phenotype (red and yellow genes expressed)

Color Genes A + B + C expressed + Cas9 expressed + gRNA C expressed → Purple Phenotype (blue and red genes expressed)

Color Genes A + B + C expressed + Cas9 expressed + gRNA A and gRNA B expressed → Yellow Phenotype (yellow gene expressed)

Color Genes A + B + C expressed + Cas9 expressed + gRNA A and gRNA C expressed → Blue Phenotype (blue gene expressed)

Color Genes A + B + C expressed + Cas9 expressed + gRNA C and gRNA B expressed → Red Phenotype (red gene expressed)

Color Genes A + B + C expressed + Cas9 expressed + gRNA A, gRNA B and gRNA C expressed → white or beige Phenotype (no color genes expressed)

### Example 2: Yeast Version 1

A schematic depicting Yeast Version 1 is shown in FIG. 2. A yeast strain constitutively expresses two different proteins (A and B). The proteins are chromogenic or fluorescent (herein referred to as chromogenic protein A or B), but share the characteristic that they are visible by eye when expressed in yeast. This strain will also carry episomal plasmids that constitutively express a gRNA to either chromogenic protein A or B (gRNA A or gRNA B). The plasmid expressing gRNA A will carry the URA3 auxotrophic marker, whereas the plasmid expressing gRNA B will carry the LYS2 auxotrophic marker. A third plasmid or integrated expression cassette will express Cas9 under the control of a galactose-inducible promoter. In the absence of galactose, no gene editing events shall take place and the yeast will be the color resulting from coexpression of chromogenic protein A and B. In the presence of galactose, Cas9 expression is induced and gRNA A or B will be incorporated into Cas9, leading to targeting of chromogenic gene A or B. In the event that both chromogenic gene A and B are targeted, the yeast will no longer express chromogenic protein A or B and the yeast colonies will appear white in color.
Plating yeast on galactose and 5-fluoro-orotic acid (5-FOA) will both induce Cas9 expression and counterselect for yeast carrying the plasmid expressing gRNA A, which carries the URA3 auxotrophic marker. As a result, only gRNA B will be expressed and incorporated into Cas9, leading to targeting of chromogenic gene B. In the event that chromogenic gene B is targeted and disrupted, then the yeast will no longer express chromogenic protein B and the yeast will appear as the color of chromogenic protein A. Plating yeast on galactose and alpha adipic acid (alpha AA) will both induce Cas9 expression and counterselect for yeast carrying the plasmid expressing gRNA B, which carries the LYS2 auxotrophic marker. As a result, only gRNA A will be expressed and incorporated into Cas9, leading to targeting of chromogenic gene A. In the event that chromogenic gene A is targeted and disrupted, then the yeast will no longer express chromogenic protein A and the yeast will appear as the color of chromogenic protein B.

### Example 3: Yeast Version 2

A schematic depicting Yeast Version 2 is shown in FIG. 3. A yeast strain constitutively expresses a chromogenic protein (A). This strain will also carry episomal plasmids that constitutively express a gRNA to either chromogenic protein A (gRNA A) or the ADE2 gene (gRNA B). The plasmid expressing gRNA A will carry the URA3 auxotrophic marker, whereas the plasmid expressing gRNA B will carry the LYS2 auxotrophic marker. A third plasmid or integrated expression cassette will express Cas9 under the control of a galactose-inducible promoter.
In the absence of galactose, no gene editing events shall take place and the yeast will be the color resulting from expression of chromogenic protein A. In the presence of galactose, Cas9 expression is induced and gRNA A or B will be incorporated into Cas9, leading to targeting of chromogenic gene A or the ADE2 gene. In the event that both chromogenic gene A and the ADE2 gene are targeted, the yeast will no longer express chromogenic protein A or ADE2 and the yeast colonies will appear red in color. Plating yeast on galactose and 5-fluoro-orotic acid (5-FOA) will both induce Cas9 expression and counterselect for yeast carrying the plasmid expressing gRNA A, which carries the URA3 auxotrophic marker. As a result, only gRNA B will be expressed and incorporated into Cas9, leading to targeting of the ADE2 gene. In the event that the ADE2 gene is targeted and disrupted, then the yeast will no longer express ADE2 and the yeast will appear as the color of the combination of chromogenic protein A and ade2 yeast (chromogenic protein A + red). Plating yeast on galactose and alpha adipic acid (alpha AA) will both induce Cas9 expression and counterselect for yeast carrying the plasmid expressing gRNA B, which carries the LYS2 auxotrophic marker. As a result, only gRNA A will be expressed and incorporated into Cas9, leading to targeting of chromogenic gene A. In the event that chromogenic gene A is targeted and disrupted, then the yeast will no longer express chromogenic protein A and the yeast will appear white.

### Example 4: Yeast Version 3

A schematic depicting Yeast Version 3 is shown in FIG. 4. A yeast strain constitutively expresses a yeast-enhanced red fluorescent protein (yEmRFP, Keppler-Ross et al 2008 *Genetics*). This strain will also carry episomal plasmids that constitutively express a gRNA to either the yEmRFP or the ADE2 gene (gRNA A or gRNA B, respectively). The plasmid expressing gRNA A will carry the URA3 auxotrophic marker, whereas the plasmid expressing gRNA B will carry the LYS2 auxotrophic marker. A third plasmid or integrated expression cassette will express Cas9 under the control of a galactose-inducible promoter.

In the absence of galactose, no gene editing events shall take place and the yeast will be the color resulting from expression of yEmRFP (purple, see Keppler-Ross et al, 2008). In the presence of galactose, Cas9 expression is induced and gRNA A or B will be incorporated into Cas9, leading to targeting of yEmRFP or the ADE2 gene. In the event that both yEmRFP and the ADE2 gene are targeted, the yeast will no longer express yEmRFP or ADE2 and the yeast colonies will appear red in color. Plating yeast on galactose and 5-fluoro-orotic acid (5-FOA) will both induce Cas9 expression and counterselect for yeast carrying the plasmid expressing gRNA A, which carries the URA3 auxotrophic marker. As a result, only gRNA B will be expressed and incorporated into Cas9, leading to targeting of the ADE2 gene.

### Example 5: Yeast Version 4

A schematic depicting Yeast Version 4 is shown in FIG. 5. A yeast strain constitutively expresses a yeast-enhanced red fluorescent protein (yEmRFP, Keppler-Ross et al 2008 *Genetics*). The yeast strain will have an ADE2 gene containing an engineered disruption, similar to an intron, that causes a frameshift and prevents expression of ADE2. This strain will also carry episomal plasmids that constitutively express a gRNA to either the yEmRFP or two gRNAs specific to the 5' and 3' ends of the engineered disruption in the ADE2 gene (gRNA A or gRNA B/C, respectively). The plasmid expressing gRNA A will carry the URA3 auxotrophic marker, whereas the plasmid expressing gRNA B/C will carry the LYS2 auxotrophic marker. A third plasmid or integrated expression cassette will express Cas9 under the control of a galactose-inducible promoter.

In the absence of galactose, no gene editing events shall take place and the yeast will be the color resulting from expression of yEmRFP and lack of ADE2 (light purple, see Keppler-Ross et al, 2008, Figure 1A). In the presence of galactose, Cas9 expression is induced and gRNA A or B will be incorporated into Cas9, leading to targeting of yEmRFP or the ADE2 gene. In the event that both yEmRFP and the ADE2 gene are targeted, the yeast will no longer express yEmRFP or ADE2 and the yeast colonies will appear white in color. Plating yeast on galactose and 5-fluoro-orotic acid (5-FOA) will both induce Cas9 expression and counterselect for yeast carrying the plasmid expressing gRNA A, which carries the URA3 auxotrophic marker. As a result, only gRNA B/C will be expressed and incorporated into Cas9, leading to targeting of the disrupted ADE2 gene. In the event that the disrupted ADE2 gene is targeted and corrected, then the yeast will express ADE2 and the yeast will appear as the color of the combination of yEmRFP and ADE2 yeast (purple). Plating yeast on galactose and alpha adipic acid (alpha AA) will both induce Cas9 expression and counterselect for yeast carrying the plasmid expressing gRNA B/C, which carries the LYS2 auxotrophic marker. As a result, only gRNA A will be expressed and incorporated into Cas9, leading to targeting of yEmRFP. In the event that yEmRFP is targeted and disrupted, then the yeast will no longer express yEmRFP and the yeast will appear red.

### Example 6: Bacteria Version 1

An exemplary schematic depicting Bacteria Version 1 is shown in FIG. 6. Bacteria express (constitutively or inducibly) a protein. The protein is chromogenic or fluorescent (such as GFP, as depicted in FIG. 6), and may be visible by eye when expressed in bacteria. An episomal plasmid that expresses a gRNA to the protein (e.g., GFP) can be transformed into the bacteria. The plasmid expressing gRNA carries the Kanamycin antibiotic resistance marker and also expresses Cas9 under the control of an inducible promoter. In the absence of an inducer, no gene editing events take place and the bacteria will be the color resulting from expression of the chromogenic protein (e.g., GFP). In the presence of an inducer, Cas9 expression is induced and the gRNA will be incorporated into Cas9, leading to targeting of GFP. Repair of the Cas9 cleavage is directed by the HR donor DNA contained in the transformed plasmid, allowing incorporation of specific mutations that prevent GFP expression. In the event that GFP is targeted, the bacteria will no longer express GFP and the bacterial colonies will appear white or beige in color.

### Example 7: Bacteria Version 2

An exemplary schematic depicting Bacteria Version 2 is shown in FIG. 7. Bacteria express (constitutively or inducibly) a protein. The protein is chromogenic or fluorescent (such as GFP, as depicted in FIG. 7), and may be visible by eye when expressed in bacteria. An episomal plasmid that expresses a gRNA to GFP can be transformed into the bacteria. The plasmid expressing gRNA carries an antibiotic resistance marker and also expresses Cas9 under the control of an inducible promoter. In the absence of an inducer, no gene editing events take place and the bacteria will be the color resulting from expression of the chromogenic protein (e.g., GFP). In the presence of an inducer, Cas9 expression is induced and the gRNA will be incorporated into Cas9, leading to targeting of GFP. Repair of the Cas9 cleavage is directed by the HR donor DNA contained in the transformed plasmid, allowing incorporation of specific mutations that prevent GFP expression. In the event that GFP is targeted, the bacteria will express BFP or YFP and the bacterial colonies will appear blue or yellow in color, respectively.

### Example 8: Bacteria Version 3

An exemplary schematic depicting Bacteria Version 3 is shown in FIG. 8. Bacteria expresses (constitutively or inducibly) a protein. The protein is chromogenic or fluorescent (such as GFP, as depicted in FIG. 8), and may be visible by eye when expressed in bacteria. An episomal plasmid that expresses a gRNA to GFP can be transformed into the bacteria. The plasmid expressing gRNA carries an antibiotic resistance marker and also expresses Cas9 under the control of an inducible promoter. In the absence of an inducer, no gene editing events shall take place and the bacteria will be the color resulting from expression of the chromogenic protein (e.g., GFP). In the presence of an inducer, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that GFP to BFP or YFP. In the event that GFP is targeted, the bacteria will no longer express GFP and the bacterial colonies will appear white or beige in color.

### Example 9: Bacteria Version 4

An exemplary schematic depicting Bacteria Version 4 is shown in FIG. 9. Bacteria express (constitutively or inducibly) a protein. The protein is chromogenic or fluorescent (such as GFP, as depicted in FIG. 9), and may be visible by eye when expressed in bacteria. An episomal plasmid that expresses a gRNA to GFP can be transformed into the bacteria. The plasmid expressing gRNA carries an antibiotic resistance marker and also expresses Cas9 under the control of an inducible promoter. In the absence of an inducer, no gene editing events shall take place and the bacteria will be the color resulting from expression of the chromogenic protein (e.g., GFP). In the presence of an inducer, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that convert GFP to BFP or YFP. In the event that GFP is targeted, the bacteria will express BFP or YFP and the bacterial colonies will appear blue or yellow in color, respectively.

### Example 10: Bacteria Version 5

An exemplary schematic depicting Bacteria Version 5 is shown in FIG. 10. Bacteria express a non-functional chromogenic or fluorescent (such as GFP) protein under the control of a constitutive or inducible promoter. An episomal plasmid that expresses a gRNA to GFP can be transformed into the bacteria. The plasmid expressing gRNA also expresses Cas9 under the control of an inducible promoter; if the GFP protein is under the control of an inducible promoter, then the inducible promoter for the GFP and the inducible promoter for Cas9 do not rely on the same method of induction (e.g., do not use the same inducing agent). In the absence of an inducer for the Cas9 promoter, no gene editing events shall take place and the bacteria are the color white. In the presence of an inducer for the Cas9 promoter, Cas9 expression is induced and the gRNA will be incorporated into Cas9, leading to targeting of GFP. Repair of the Cas9 cleavage is directed by the HR donor contained in the transformed plasmid, allowing incorporation of specific mutations that restore GFP expression. In the event that GFP is targeted, the bacteria will express a functional GFP and the bacterial colonies will appear green in color.

***Example 11: Bacteria Version 6***

An exemplary schematic depicting Bacteria Version 6 is shown in FIG. 11. Bacteria express a non-functional chromogenic or fluorescent (such as GFP) protein under the control of a constitutive or inducible promoter. An episomal plasmid that expresses a gRNA to GFP can be transformed into the bacteria. The plasmid expressing gRNA also expresses Cas9 under the control of an inducible promoter; if the GFP protein is under the control of an inducible promoter, then the inducible promoter for the GFP and the inducible promoter for Cas9 do not rely on the same method of induction (e.g., do not use the same inducing agent). In the absence of an inducer for the Cas9 promoter, no gene editing events shall take place and the bacteria are the color white. In the presence of an inducer for the Cas9 promoter, Cas9 expression is induced and the gRNA will be incorporated into Cas9, leading to targeting of GFP. Repair of the Cas9 cleavage is directed by the ssDNA HR donor co-transformed with the plasmid, allowing incorporation of specific mutations that restore GFP expression. In the event that GFP is targeted, the bacteria will express a functional GFP and the bacterial colonies will appear green in color.

***Example 12: Bacteria Version 7***

An exemplary schematic depicting Bacteria Version 7 is shown in FIG. 12. Bacteria have been genetically altered to have inducible Cas9 and inducible lambda red expression cassettes inserted within the bacterial genome. Both of these inducible promoters have loxP or loxP variant sequences that flank a transcription terminator sequence directly following the promoter. These gene regulatory elements will block transcription, preventing expression of the induced proteins.

These genetically altered bacteria are transformed with a single plasmid that contains an antibiotic resistance cassette (ARC) lacking its own promoter that is flanked on the 5' and 3' ends with short portions of an endogenous target gene (LacZ in Figure 12), a gRNA that recognizes the targeted gene ("gRNA-LZ" in Figure 12), a constitutively expressed Cre-recombinase cassette, and a "self-destruct" gRNA that targets a self region within the plasmid, termed a non-repairable DSB site ("gRNA-plasmid 1" in Figure 12). Upon expression of Cre recombinase, both of the transcription termination sequences will be excised, leaving a single loxP or loxP variant "scar" behind, and transcription and translation will become undeterred. A constitutively active Cas9 cassette will initiate Cas9 expression. The plasmid will be destroyed, and only bacteria that integrate the ARC will survive selection by that antibiotic. By design, there is a heightened risk for off-target insertion within the bacterial genome other than targeted gene. Upon induction of CRISPR-Cas and lambda red, recombination repair will allow the cell to express the antibiotic resistance protein and lose normal targeted gene expression (LacZ in Figure 12). Bacteria are screened for loss of the normal targeted gene's function and the ability to survive in the presence of the antibiotic.

***Example 13: Bacteria Version 8***

An exemplary schematic depicting Bacteria Version 8 is shown in FIG. 13. Bacteria have been genetically altered to have inducible Cas9 and Ku and LigD expression cassettes present. Additionally, an endogenous gene is replaced with a chromogenic protein ORF. These altered bacteria will express the chromogenic protein upon activation of the endogenous gene.

The genetically altered bacteria are transformed with a single plasmid that has a cassette driving expression of an sgRNA that recognizes the chromogenic protein. Upon induction, a DSB will occur within the chromogenic protein. In contrast to unaltered *E coli*, the altered bacteria are able to perform non-homologous end joining (NHEJ) based repair of the DSB break. Ku and LigD proteins will recognize the DSB and rejoin the two broken ends. Because this method of DSB repair is prone to error, a significant percentage of *E. coli* will lose the ability to express the chromogenic protein. A user can quantify the percentage of bacteria that lose the expression of the chromogenic protein, as compared to the percentage that retain expression.

***Example 14: Bacteria Version 9***

An exemplary schematic depicting Bacteria Version 9, a two-plasmid system for simultaneous loss and gain of function, is shown in FIG. 14. Bacteria are genetically altered to have inducible CRISPR activity and inducible lambda red expression cassettes. As an example, unaltered HB101 strain or HME63 strain *E. coli* express β-galactosidase under control of the native or modified lacZ promoter, which is induced by the presence of Isopropyl β-D-1-thiogalactopyranoside (IPTG). In HME63 strain bacteria, an arabinose inducible lambda red cassette is located within the bacteria's chromosome. For HB101 strain bacteria the arabinose inducible lambda red cassette is located in an additional plasmid already present in the bacteria. The bacteria, e.g., one of the HME63 or HB101 bacterial strains, are transformed with two unique plasmids. The first plasmid contains a constitutively expressing gRNA that recognizes the lacZ gene and an antibiotic resistance cassette (ARC) lacking its own promoter that is flanked on the 5' and 3' ends with short portions of the lacZ gene. The ARC is in the same reading frame as β-galactosidase. The second plasmid contains a rhamnose-inducible promoter driving Cas9 nuclease. In the presence of all four of the described elements, a CRISPR-Cas mediated double strand break (DSB) will occur within the genome at the lacZ gene. Lambda red proteins will recognize the DSB and initiate homologous recombination based repair utilizing the lacZ flanked ARC. *E. coli* are screened for loss of blue color from X-gal stain and the ability to survive in the presence of the novel antibiotic. Only upon successful CRISPR-Cas initiated recombination repair will the modified *E. coli* be able to both express the antibiotic resistance protein and lose normal β-galactosidase expression.

### Example 15: Cas9-Mediated Targeting of Genomic Locus

*E. coli* HB101 bacteria were transformed with a plasmid containing a Cas9 endonuclease and a plasmid containing the Lambda red recombinase genes. The cells were subsequently transformed with single-stranded gRNA targeting a genomic target (LacZ) and double-stranded donor DNA containing a chloramphenicol (CAM) gene flanked by LacZ homology arms. Cells were plated on CAM plates and Ampicillin/Kanamycin plates. Cells that underwent homologous recombination acquired resistance to CAM were able to grow on CAM plates. CAM-resistant colonies were screened by PCR. The PCR product for wild type LacZ is 450 bp, whereas the PCR product for LacZ after Cas9-mediated recombination of the donor DNA is 1300 bp. Of the 10 colonies producing a PCR result, 8 were positive for the Cas9-mediated recombination of the donor DNA.

### Example 16: Cas9-Mediated Repair of Genomic Locus

A LacZ DNA donor plasmid was created to insert a stop codon into the β-galactosidase reading frame, thereby creating a 163 amino acid truncated non-functioning enzyme. HB 101 bacteria containing a lambda red expressing plasmid and the LacZ donor plasmid were transformed with plasmids containing the following expression cassettes: a Cas9 cassette but no gRNA expressing cassette (Fig 16A, Control Transformation 1), a gRNA expressing cassette but no Cas9 expressing cassette (Fig 16B, Control Transformation 2), or a gRNA expressing cassette and a Cas9 expressing cassette (Fig 16C, CRISPR Transformation). All bacteria were grown in the presence of ampicillin, kanamycin, streptomycin, rhamnose, arabinose, X-gal substrate, and IPTG. IPTG induces the LacZ promoter to drive expression of β-galactosidase, which converts the colorless X-gal substrate into a blue product. Only bacteria with intact β-galactosidase will become blue while cells that lack functional β-galactosidase will remain white. 100% of cells that did not have CRISPR activity showed normal β-galactosidase. When CRISPR was activated, approximately 88% of 350 colonies were white, displaying a lack of β-galactosidase activity, while 43 of the ~350 colonies seen were blue suggesting the presence of functional β-galactosidase (Fig. 16D).

In order to further verify genomic targeting of the LacZ locus, polymerase chain reaction based genotyping was performed. Two oligonucleotides (FWT1 and FWT2) produce a 686 base pair amplicon in wild-type and targeted bacteria. Correctly targeted LacZ mutant *E. coli* produce a 550 base pair amplicon from FWT1 and Rmut1 oligonucleotides (Fig. 17A). 0 of 20 colonies from control transformation 2 showed a 550 base pair mutant PCR product. 18 of 19 colonies from CRISPR transformations produced both a wild-type band and the mutant band (white and black arrow, respectively, FIG 17B-C). Sanger sequencing was performed on the PCR product of several positive mutants to confirm the expected insertion of the premature stop codon. All showed identical sequence matching the donor plasmid (representative sequence trace, Fig. 17D).

INFORMAL SEQUENCE LISTING

SEQ ID NO:1 - Yeast codon optimized Cas9

```
ATGTATCCATATGATGTTCCAGATTACGCTCCACCTAAGAAGAAACGTAAGGTTGACAAAAAGTACTCCATCGGTTT
AGATATTGGTACCAACTCTGTCGGTTGGGCCGTTATTACTGATGAATACAAGGTTCCATCTAAGAAGTTCAAAGTTT
TAGGTAACACTGATAGACACTCCATTAAGAAGAATTTGATCGGTGCTTTGTTGTTCGATTCCGGTGAAACCGCCGAA
GCTACCAGATTAAAGAGAACCGCTAGAAGAAGATATACCAGAAGAAAGAACAGAATTTGTTACTTGCAAGAAATTTT
CTCCAACGAAATGGCCAAGGTTGATGATTCTTTCTTTCACAGATTGGAAGAATCCTTTTTAGTCGAAGAAGATAAGA
AACACGAAAGACACCCAATCTTCGGTAACATTGTCGACGAAGTCGCTTATCACGAAAAATATCCAACTATTTACCAC
TTGAGAAAGAAGTTAGTCGACTCCACCTACAAAGCTGACTTGAGATTGATTTATTTGGCTTTGGCTCACATGATTAA
GTTCAGAGGTCACTTCTTAATCGAAGGTGACTTGAACCCTGACAATTCCGATGTTGACAAGTTGTTCATCCAATTGG
TCCAAACCTATAATCAATTGTTCGAAGAAATCCAATCAACGCTTCCGGTGTTGACGCTAAAGCCATCTTGTCTGCC
AGATTGTCCAAGTCCCGTCGTTTAGAAAACTTGATTGCTCAATTGCCAGGTGAAAAGAAGAACGGTTTGTTTGGTAA
CTTGATTGCTTTGTCCTTGGGTTTAACCCCAAACTTCAAGTCTAACTTCGATTTGGCCGAAGATGCTAAGTTGCAAT
TGTCCAAGGATACTTACGATGACGATTTGGATAACTTATTGGCCCAAATCGGTGACCAATACGCTGACTTGTTCTTG
GCCGCTAAGAACTTATCCGACGCCATCTTGTTGTCCGACATTTTAAGAGTTAACACTGAAATTACCAAGGCCCCATT
GTCCGCCTCCATGATCAAGAGATACGACGAACACCACCAAGACTTGACCTTATTGAAGGCTTTAGTTCGTCAACAAT
TACCAGAAAAGTATAAAGAAATCTTCTTTGATCAATCTAAAAACGGTTACGCTGGTTATATTGATGGTGGTGCCTCT
CAAGAAGAATTCTACAAATTTATCAAGCCTATCTTAGAAAAAATGGACGGTACCGAAGAATTATTGGTCAAGTTAAA
CAGAGAAGATTTGTTGCGTAAGCAACGTACTTTCGACAACGGTTCCATCACCCATCAAATCCACTTGGGTGAATTGC
ACGCTATCTTAAGAAGACAAGAAGATTTCTACCCATTCTTGAAAGATAATAGAGAAAAAATTGAAAAATTTTGACT
TTCAGAATTCCTTACTACGTTGGTCCATTAGCCAGAGGTAACTCTAGATTTGCTTGGATGACTAGAAAGTCCGAAGA
AACTATTACCCCATGGAACTTCGAAGAAGTCGTTGACAAGGGTGCTTCCGCTCAATCCTTCATTGAAAGAATGACCA
ATTTCGATAAAAACTTACCAAACGAAAAGGTTTTGCCAAAGCACTCTTTGTTATATGAATACTTCACCGTTTACAAC
GAATTGACTAAAGTCAAGTACGTTACCGAAGGTATGAGAAAGCCAGCTTTCTTGTCTGGTGAGCAAAGAAGGCTAT
TGTTGACTTATTATTCAAAACTAACAGAAAGGTCACTGTCAAGCAATTGAAGGAAGATTATTTCAAAAAGATCGAAT
GCTTCGACTCTGTTGAAATCTCTGGTGTTGAAGATAGATTCAACGCTTCCTTGGGTACCTATCACGATTTGTTGAAA
ATCATCAAGGACAAGGACTTTTTGGATAACGAGGAAAACGAAGACATTTTAGAAGATATTGTTTTGACTTTGACCTT
GTTCGAAGACAGAGAAATGATCGAAGAAAGATTGAAGACCTACGCTCATTTGTTCGACGATAAAGTCATGAAACAAT
TGAAGAGAAGAAGATATACCGGTTGGGGTAGATTATCTAGAAAGTTAATTAATGGTATTAGAGACAAGCAATCCGGT
AAGACCATCTTGGATTTCTTAAAATCTGACGGTTTCGCTAACCGTAACTTCATGCAATTGATTCATGATGACTCTTT
GACCTTCAAAGAAGATATTCAAAAGGCTCAAGTCTCTGGTCAAGGTGATTCTTTGCACGAACATATTGCTAACTTGG
CTGGTTCCCCTGCCATTAAGAAGGGTATTTTGCAAACTGTTAAGGTCGTTGACGAATTGGTCAAGGTTATGGGTAGA
CACAAACCAGAAAACATCGTCATTGAAATGGCCAGAGAAAACCAAACCACCCAAAAAGGTCAAAGAACTCCCGTGA
AAGAATGAAGAGAATCGAAGAAGGTATCAAGGAGTTGGGTTCTCAAATTTTAAAGGAACATCCAGTCGAGAACACTC
AATTGCAAAACGAAAAGTTGTACTTGTACTACTTACAAAACGGTAGAGATATGTACGTCGATCAAGAATTAGACATT
AATAGATTGTCTGACTACGACGTCGACCATATTGTCCCACAATCTTTCTTAAAGGACGATTCCATTGACAACAAAGT
TTTAACTAGATCCGACAAAAACAGAGGTAAGTCTGATAACGTTCCTTCTGAAGAAGTCGTCAAGAAGATGAAGAACT
ACTGGAGACAATTGTTGAACGCCAAATTGATCACCCAAAGAAAGTTCGACAACTTAACCAAGGCTGAAAGAGGTGGT
TTGTCTGAATTGGATAAGGCTGGTTTTATTAAGAGACAATTGGTCGAAACCAGACAAATTACTAAACATGTTGCTCA
AATCTTGGATTCTCGTATGAATACCAAATACGACGAAACGATAAATTGATTAGAGAAGTCAAGGTTATTACTTTGA
AGTCCAAGTTGGTTTCTGATTTCCGTAAGGACTTCCAATTCTACAAAGTTAGAGAAATTAACAATTACCACCACGCT
CATGACGCTTACTTGAACGCTGTCGTTGGTACTGCCTTGATTAAGAAGTACCCAAAATTGGAATCCGAATTCGTTTA
TGGTGACTACAAAGTTTACGATGTCAGAAAATGATTGCTAAGTCTGAACAAGAGATTGGTAAAGCTACTGCCAAAT
ATTTCTTTTACTCTAACATCATGAACTTCTTTAAGACCGAAATCACTTTAGCTAACGGTGAAATTCGTAAGAGACCA
TTAATCGAAACTAACGGTGAAACTGGTGAAATTGTCTGGGATAAGGGTAGAGATTTCGCCACCGTTCGTAAGGTTTT
GTCTATGCCTCAAGTTAATATCGTCAAGAAGACCGAAGTCCAAACCGGTGGTTTTTCTAAGGAATCTATCTTGCCAA
AGAGAAATTCTGACAAGTTGATTGCTAGAAAGAAAGACTGGGATCCAAAGAAGTACGGTGGTTTTGACTCCCCAACT
GTTGCTTACTCCGTTTTGGTTGTTGCTAAGGTTGAAAAGGGTAAGTCTAAGAAGTTAAAGTCTGTTAAGGAATTGTT
GGGTATTACCATTATGGAAAGATCTTCTTTTGAGAAAAACCCAATTGACTTTTTAGAGGCTAAGGGTTACAAGGAAG
TTAAGAAGGACTTGATCATTAAATTGCCAAAGTATTCTTTGTTCGAATTGGAAAACGGTCGTAAGCGTATGTTGGCC
TCTGCTGGTGAATTACAAAAGGGTAACGAATTAGCTTTGCCTTCTAAATACGTTAACTTTTTATACTTGGCTTCCCA
TTACGAAAAGTTAAAAGGTTCTCCAGAAGACAATGAACAAAAACAATTGTTCGTTGAACAACACAAGCATTACTTGG
ACGAAATTATTGAACAAATTTCTGAATTTTCCAAGAGAGTTATCTTAGCCGACGCTAACTTGGACAAGGTCTTGTCT
GCTTACAATAAGCATAGAGACAAGCCAATCCGTGAACAAGCCGAAACATCATTCACTTGTTCACTTTGACTAACTT
GGGTGCTCCAGCTGCCTTCAAGTACTTCGACACCACTATCGACAGAAAGAGATACACTTCTACTAAGGAGGTTTTAG
ATGCCACCTTGATTCACCAATCTATTACTGGTTTGTACGAGACTAGAATTGATTTGTCCCAATTAGGTGGTGATCCA
CCTAAGAAGAAGAGAAAGGTTTAA
```

**SEQ ID NO:2** - *E. coli* codon optimized Cas9

```
ATGTATCCATACGACGTGCCTGACTATGCGGACAAAAAGTACTCCATCGGTTTGGACATCGGGACGAATAGCGTTGGGTG
GGCGGTGATTACAGATGAATATAAGGTGCCTAGTAAAAAATTCAAAGTATTAGGCAATACCGATCGTCATAGCATTAAGA
AGAACCTGATTGGAGCATTGCTTTTTGATTCGGGTGAAACCGCAGAAGCAACGCGTTTGAAGCGCACAGCACGTCGTCGC
```

```
TACACACGCCGCAAAAATCGTATTTGTTATCTTCAAGAAATTTTTTCAAATGAGATGGCAAAGGTCGACGATTCTTTTTT
CCATCGTTTAGAGGAATCTTTTCTTGTGGAGGAAGATAAGGAGCACGAGCGTCACCCAATCTTTGGTAATATTGTCGACG
AAGTTGCGTACCATGAAAAGTACCCAACGATCTACCACCTGCGTAAGAAATTGGTGGACTCGACATACAAGGCGGATCTT
CGCTTGATCTATCTTGCTTTGGCCCACATGATTAAGTTCCGCGGGCATTTCTTAATTGAAGGAGACTTAAACCCGGATAA
CTCAGATGTTGACAAGCTTTTTATTCAGCTTGTGCAAACTTACAATCAACTTTTCGAAGAAAACCCTATCAACGCCTCTG
GTGTGGATGCGAAGGCGATCCTTTCGGCGCGCCTGTCAAAGAGTCGTCGCTTGGAGAATTTGATTGCGCAGTTGCCGGGG
GAGAAGAAAATGGCCTGTTTGGAAACCTGATTGCGCTTTCTCTTGGATTAACTCCCAACTTTAAGTCAAACTTCGACTT
GGCTGAGGATGCCAAGTTACAGCTGTCCAAAGATACCTACGATGATGATCTTGATAACTTGCTGGCTCAAATCGGTGACC
AATACGCGGATCTTTTCTTGGCCGCGAAGAACTTGTCTGACGCAATCCTTTTATCGGACATCCTGCGCGTTAACACAGAG
ATCACTAAAGCCCCTCTGTCTGCATCAATGATTAAACGCTACGACGAACACCATCAGGATTTGACATTACTGAAAGCCCT
TGTACGTCAACAACTTCCGGAAAAGTACAAAGAAATCTTCTTCGATCAGTCTAAAAACGGGTACGCCGGGTATATTGATG
GTGGCGCTTCACAGGAGGAGTTTTACAAATTCATTAAACCTATTCTGGAGAAATGGATGGAACGGAAGAGTTGTTGGTG
AAGCTTAATCGTGAAGACCTTTTGCGCAAGCAGCGTACGTTCGATAACGGGTCAATCACACACCAAATCCACTTGGGCGA
GTTACATGCAATTCTTCGCCGTCAGGAAGATTTCTACCCTTTCTTGAAAGATAACCGCGAGAAGATTGAAAAGATTTTGA
CTTTTCGTATCCCCTACTACGTGGGTCCTTTAGCCCGTGGAAACAGTCGCTTCGCCTGGATGACCCGCAAGTCAGAAGAA
ACGATCACCCCCTGGAATTTTGAGGAGGTTGTGGATAAGGGGGCGTCAGCGCAAAGCTTCATCGAACGCATGACGAACTT
CGATAAGAACTTACCTAATGAGAAAGTGCTGCCAAAACATAGTCTTCTTTATGAGTACTTCACTGTTTACAATGAGTTAA
CTAAGGTAAAGTATGTTACAGAAGGGATGCGTAAACCCGCATTTTTATCCGGTGAGCAAAAGAAAGCTATCGTGGATTTG
TTATTTAAGACTAACCGCAAAGTAACAGTCAAACAATTAAAAGAAGACTACTTTAAGAAAATTGAGTGCTTTGACTCAGT
GGAGATCTCTGGTGTCGAGGACCGCTTTAATGCCTCATTGGGAACTTATCACGACTTACTGAAGATTATTAAAGATAAAG
ACTTTCTTGACAACGAAGAGAACGAAGATATTCTGGAGGACATCGTCTTAACACTGACTCTGTTCGAGGATCGCGAGATG
ATTGAGGAACGCTTGAAAACTTATGCCCACTTATTTGATGACAAAGTGATGAAACAACTTAAACGTCGTCGCTACACCGG
ATGGGGTCGTTTATCACGTAAATTAATCAACGGCATTCGCGATAAGCAGTCCGGCAAAACAATCCTTGATTTTCTGAAGT
CCGACGGATTCGCGAATCGCAACTTTATGCAGCTGATCCACGATGATAGTCTTACCTTCAAGGAGGATATTCAAAAAGCC
CAGGTATCGGGGCAAGGTGACTCCCTGCACGAACATATTGCGAATTGGCCGGGTCTCCGGCAATCAAGAAAGGTATTTT
ACAGACCGTTAAAGTCGTGGATGAATTGGTTAAGGTAATGGGCCGTCATAAACCGGAGAATATCGTAATTGAAATGGCGC
GTGAGAATCAGACAACTCAGAAAGGACAAAAGAATagcCGCGAACGTATGAAACGTATTGAAGAGGGAATCAAAGAATTA
GGGAGTCAGATCTTAAAAGAACATCCAGTTGAAAACACCCAATTGCAAAACGAAAAGTTATACTTATACTACCTTCAGAA
CGGCCGCGATATGTACGTAGATCAGGAATTAGACATTAACCGCCTGTCAGATTACGATGTCGACCATATTGTTCCTCAGT
CTTTCTTAAAGGACGATAGCATCGATAATAAAGTTTTAACTCGTTCGGATAAAAACCGTGGAAAATCCGACAACGTCCCA
TCTGAGGAGGTAGTCAAGAAGATGAAGAACTACTGGCGTCAGTTACTGAACGCGAAATTGATCACTCAGCGCAAATTTGA
TAATTTGACTAAAGCCGAACGCGGTGGTTTGTCGGAGTTAGACAAAGCCGGCTTCATCAAACGCCAGCTTGTAGAGACCC
GCCAGATTACGAAGCACGTTGCCCAGATTCTTGACAGCCGTATGAACACCAAGTACGATGAGAATGATAAACTGATTCGC
GAGGTGAAGGTAATCACGTTAAAGAGCAAACTGGTAAGTGATTTTCGTAAGGACTTTCAATTTTACAAAGTGCGCGAGAT
CAACAACTATCACCATGCGCATGATGCCTATTTGAATGCCGTAGTCGGTACAGCTTTGATTAAGAAGTATCCTAAGTTGG
AGTCAGAATTtGTCTACGGCGACTACAAGGTGTATGACGTACGCAAGATGATTGCGAAGTCCGAGCAGGAAATTGGCAAG
GCCACTGCTAAGTACTTCTTTTATTCTAACATTATGAACTTCTTCAAAACCGAAATCACCCTTGCGAACGGGGAAATTCG
TAAGCGCCCGTTGATCGAAACAAATGGCGAGACTGGTGAAATTGTTTGGGACAAAGGTCGCGATTTTGCAACGGTGCGTA
AAGTATTAAGTATGCCCCAGGTAAATATTGTCAAGAAGACGGAAGTGCAAACCGGTGGGTTTTCTAAAGAATCAATTTTG
CCGAAGCGTAATTCTGATAAATTAATTGCGCGCAAAAAGGACTGGGACCCTAAGAAATACGGCGGGTTCGACTCACCCAC
GGTCGCCTATTCCGTGTTAGTTGTTGCGAAAGTCGAGAAAGGTAAAAGCAAGAAACTTAAGTCTGTTAAGGAATTGTTAG
GAATCACGATTATGGAACGTAGCTCATTTGAGAAAAACCCCATTGACTTTTTGGAGGCGAAAGGTTACAAGGAAGTTAAA
AAAGACCTGATTATTAAACTTCCCAAGTACAGCCTTTTCGAACTGGAAATGGTCGTAAGCGCATGCTGGCGTCGGCCGG
TGAACTGCAAAAGGGGAATGAGTTGGCCCTGCCATCGAAGTATGTGAATTTCTTATCCTGGCGTCCCATTACGAAAAAT
TAAAAGGCTCACCGGAAGATAATGAGCAAAAGCAGCTGTTTGTGGAGCAACACAAACATTACTTAGATGAATTATCGAA
CAGATTTCGGAGTTCAGCAAGCGCGTGATTCTTGCGGACGCGAATCTTGATAAGGTTTTATCTGCTTACAACAAGCACCG
CGACAAGCCGATCCGTGAGCAAGCTGAGAACATTATTCATTTATTCACACTGACTAATCTTGGGGCGCCAGCTGCCTTTA
AATACTTTGACACCACCATTGACCGCAAACGCTACACAAGTACAAAAGAAGTCTTAGACGCGACACTGATTCATCAATCC
ATCACTGGTTTATATGAAACACGCATCGATCTTTCGCAACTGGGAGGTGATTAA
```

SEQ ID NO:3 - *S. pyogenes* Cas9

```
ATGGATAAGAAATACTCAATAGGCTTAGATATCGGCACAAATAGCGTCGGATGGGCGGTGATCACTGATGAATATAAGGTTCCGTC
TAAAAAGTTCAAGGTTCTGGGAAATACAGACCGCCACAGTATCAAAAAAAATCTTATAGGGGCTCTTTTATTTGACAGTGGAGAGA
CAGCGGAAGCGACTCGTCTCAAACGGACAGCTCGTAGAAGGTATACACGTCGGAAGAATCGTATTTGTTATCTACAGGAGATTTTT
TCAAATGAGATGGCGAAAGTAGATGATAGTTTCTTTCATCGACTTGAAGAGTCTTTTTTGGTGGAAGAAGACAAGAAGCATGAACG
TCATCCTATTTTTGGAAATATAGTAGATGAAGTTGCTTATCATGAGAAATATCCAACTATCTATCATCTGCGAAAAAAATTGGTAG
ATTCTACTGATAAAGCGGATTTGCGCTTAATCTATTTGGCCTTAGCGCATATGATTAAGTTTCGTGGTCATTTTTTGATTGAGGGA
GATTTAAATCCTGATAATAGTGATGTGGACAAACTATTTATCCAGTTGGTACAAACCTACAATCAATTATTTGAAGAAAACCTAT
TAACGCAAGTGGAGTAGATGCTAAAGCGATTCTTTCTGCACGATTGAGTAAATCAAGACGATTAGAAATCTCATTGCTCAGCTCC
CCGGTGAGAAGAAAATGGCTTATTTGGGAATCTCATTGCTTTGTCATTGGGTTTGACCCCTAATTTTAAATCAAATTTTGATTTG
GCAGAAGATGCTAAATTACAGCTTTCAAAAGATACTTACGATGATGATTTAGATAATTTATTGGCGCAAATTGGAGATCAATATGC
TGATTTGTTTTTGGCAGCTAAGAATTTATCAGATGCTATTTTACTTTCAGATATCCTAAGAGTAAATACTGAAATAACTAAGGCTC
CCCTATCAGCTTCAATGATTAAACGCTACGATGAACATCATCAAGACTTGACTCTTTTAAAAGCTTTAGTTCGACAACAACTTCCA
GAAAAGTATAAAGAAATCTTTTTTGATCAATCAAAAAACGGATATGCAGGTTATATTGATGGGGGAGCTAGCCAAGAAGAATTTTA
TAAATTTATCAAACCAATTTTAGAAAAAATGGATGGTACTGAGGAATTATTGGTGAAACTAAATCGTGAAGATTTGCTGCGCAAGC
AACGGACCTTTGACAACGGCTCTATTCCCCATCAAATTCACTTGGGTGAGCTGCATGCTATTTTGAGAAGACAAGAAGACTTTTAT
CCATTTTTAAAAGACAATCGTGAGAAGATTGAAAAAATCTTGACTTTTCGAATTCCTTATTATGTTGGTCCATTGGCGCGTGGCAA
TAGTCGTTTTGCATGGATGACTCGGAAGTCTGAAGAAACAATTACCCCATGGAATTTTGAAGAAGTTGTCGATAAAGGTGCTTCAG
CTCAATCATTTATTGAACGCATGACAAACTTTGATAAAAATCTTCCAAATGAAAAAGTACTACCAAAACATAGTTTGCTTTATGAG
```

```
TATTTTACGGTTTATAACGAATTGACAAAGGTCAAATATGTTACTGAAGGAATGCGAAAACCAGCATTTCTTTCAGGTGAACAGAA
GAAAGCCATTGTTGATTTACTCTTCAAAACAAATCGAAAAGTAACCGTTAAGCAATTAAAAGAAGATTATTTCAAAAAAATAGAAT
GTTTTGATAGTGTTGAAATTTCAGGAGTTGAAGATAGATTTAATGCTTCATTAGGTACCTACCATGATTTGCTAAAAATTATTAAA
GATAAAGATTTTTTGGATAATGAAGAAATGAAGATATCTTAGAGGATATTGTTTTAACATTGACCTTATTTGAAGATAGGGAGAT
GATTGAGGAAAGACTTAAAACATATGCTCACCTCTTTGATGATAAGGTGATGAAACAGCTTAAACGTCGCCGTTATACTGGTTGGG
GACGTTTGTCTCGAAAATTGATTAATGGTATTAGGGATAAGCAATCTGGCAAAACAATATTAGATTTTTTGAAATCAGATGGTTTT
GCCAATCGCAATTTTATGCAGCTGATCCATGATGATAGTTTGACATTTAAAGAAGACATTCAAAAAGCACAAGTGTCTGGACAAGG
CGATAGTTTACATGAACATATTGCAAATTTAGCTGGTAGCCCTGCTATTAAAAAAGGTATTTTACAGACTGTAAAAGTTGTTGATG
AATTGGTCAAAGTAATGGGGCGGCATAAGCCAGAAAATATCGTTATTGAAATGGCACGTGAAAATCAGACAACTCAAAAGGGCCAG
AAAAATTCGCGAGAGCGTATGAAACGAATCGAAGAAGGTATCAAAGAATTAGGAAGTCAGATTCTTAAAGAGCATCCTGTTGAAAA
TACTCAATTGCAAAATGAAAAGCTCTATCTCTATTATCTCCAAAATGGAAGAGACATGTATGTGGACCAAGAATTAGATATTAATC
GTTTAAGTGATTATGATGTCGATCACATTGTTCCACAAAGTTTCCTTAAAGACGATTCAATAGACAATAAGGTCTTAACGCGTTCT
GATAAAAATCGTGGTAAATCGGATAACGTTCCAAGTGAAGAAGTAGTCAAAAAGATGAAAAACTATTGGAGACAACTTCTAAACGC
CAAGTTAATCACTCAACGTAAGTTTGATAATTTAACGAAAGCTGAACGTGGAGGTTTGAGTGAACTTGATAAAGCTGGTTTTATCA
AACGCCAATTGGTTGAAACTCGCCAAATCACTAAGCATGTGGCACAAATTTTGGATAGTCGCATGAATACTAAATACGATGAAAAT
GATAAACTTATTCGAGAGGTTAAAGTGATTACCTTAAAATCTAAATTAGTTTCTGACTTCCGAAAAGATTTCCAATTCTATAAAGT
ACGTGAGATTAACAATTACCATCATGCCCATGATGCGTATCTAAATGCCGTCGTTGGAACTGCTTTGATTAAGAAATATCCAAAAC
TTGAATCGGAGTTTGTCTATGGTGATTATAAAGTTTATGATGTTCGTAAAATGATTGCTAAGTCTGAGCAAGAAATAGGCAAAGCA
ACCGCAAAATATTTCTTTTACTCTAATATCATGAACTTCTTCAAAACAGAAATTACACTTGCAAATGGAGAGATTCGCAAACGCCC
TCTAATCGAAACTAATGGGGAAACTGGAGAAATTGTCTGGGATAAAGGGCGAGATTTTGCCACAGTGCGCAAAGTATTGTCCATGC
CCCAAGTCAATATTGTCAAGAAAACAGAAGTACAGACAGGCGGATTCTCCAAGGAGTCAATTTTACCAAAAAGAAATTCGGACAAG
CTTATTGCTCGTAAAAAAGACTGGGATCCAAAAAAATATGGTGGTTTTGATAGTCCAACGGTAGCTTATTCAGTCCTAGTGGTTGC
TAAGGTGGAAAAAGGGAAATCGAAGAAGTTAAAATCCGTTAAAGAGTTACTAGGGATCACAATTATGGAAAGAAGTTCCTTTGAAA
AAAATCCGATTGACTTTTTAGAAGCTAAAGGATATAAGGAAGTTAAAAAAGACTTAATCATTAAACTACCTAAATATAGTCTTTTT
GAGTTAGAAAACGGTCGTAAACGGATGCTGGCTAGTGCCGGAGAATTACAAAAAGGAAATGAGCTGGCTCTGCCAAGCAAATATGT
GAATTTTTTATATTTAGCTAGTCATTATGAAAAGTTGAAGGGTAGTCCAGAAGATAACGAACAAAAACAATTGTTTGTGGAGCAGC
ATAAGCATTATTTAGATGAGATTATTGAGCAAATCAGTGAATTTTCTAAGCGTGTTATTTTAGCAGATGCCAATTTAGATAAAGTT
CTTAGTGCATATAACAAACATAGAGACAAACCAATACGTGAACAAGCAGAAAATATTATTCATTTATTTACGTTGACGAATCTTGG
AGCTCCCGCTGCTTTTAAATATTTTGATACAACAATTGATCGTAAACGATATACGTCTACAAAAGAAGTTTTAGATGCCACTCTTA
TCCATCAATCCATCACTGGTCTTTATGAAACACGCATTGATTTGAGTCAGCTAGGAGGTGAC
```

## Claims

1. An engineered microbial organism cell comprising:

    (a) an endogenous gene or genomic region to be targeted that encodes a functional protein having a first detectable phenotype, and a heterologous polynucleotide sequence encoding a phenotype coding sequence operably linked to a promoter that (i) encodes a second functional protein having a second detectable phenotype, or (ii) comprises an engineered disruption in a sequence encoding the second protein that prevents expression of the second detectable phenotype for gene alteration or
    one or more heterologous polynucleotide sequences comprising:

        a first phenotype coding sequence operably linked to a promoter, wherein the first phenotype coding sequence either (i) encodes a first functional protein having a first detectable phenotype, or (ii) comprises an engineered disruption in a sequence encoding protein that prevents expression of the first detectable phenotype; and

a second phenotype coding sequence operably linked to a promoter, wherein the second phenotype coding sequence either (i) encodes a second functional protein having a second detectable phenotype, or (ii) comprises an engineered disruption in a sequence encoding the second protein that prevents expression of the second detectable phenotype;

wherein each of the first and the second detectable phenotypes is a detectable color, fluorescence, scent, enzymatic activity, antibiotic resistance, morphology or lethality;

(b) a heterologous polynucleotide sequence comprising a promoter operably linked to a polynucleotide encoding a Cas nuclease and a heterologous polynucleotide sequence comprising a guide RNA (gRNA) that targets the first phenotype coding sequence, a gRNA that targets the second phenotype coding sequence; and a heterologous polynucleotide sequence comprising a promoter operably linked to a polynucleotide encoding lambda red.

2. The engineered microbial organism cell of claim 1, wherein (b) further comprises a first and/or second homologous donor DNA sequence.

3. The engineered microbial organism cell of claim 1 or 2, comprising

(a) an endogenous gene or genomic region to be targeted for gene alteration, wherein the endogenous gene or genomic region to be targeted for gene alteration is a functional gene or genomic region and the donor DNA sequence is a sequence that disrupts the functional gene or genomic region or is a sequence that replaces the functional gene or genomic region with a different functional gene or different functional genomic region; or

(b) the endogenous gene or genomic region to be targeted is a disrupted gene or genomic region and wherein the donor DNA sequence is a sequence that restores the function of the gene or genomic region.

4. The engineered microbial organism cell of any of claims 1 to 3, comprising one or more heterologous polynucleotide sequences comprising a phenotype coding sequence operably linked to a promoter, wherein the phenotype coding sequence encodes a functional protein having a detectable phenotype.

5. The engineered microbial organism cell of claim 1, comprising:

a heterologous polynucleotide sequence comprising a chromogenic coding sequence that encodes a functional chromogenic protein;

a heterologous polynucleotide sequence comprising a promoter operably linked to a polynucleotide encoding a Cas nuclease; a heterologous polynucleotide sequence comprising a gRNA that targets the chromogenic coding sequence; and a heterologous polynucleotide sequence comprising a homologous donor DNA sequence, preferably

(i) the polynucleotide sequence comprising the gRNA that targets the chromogenic coding sequence is operably linked to the polynucleotide sequence comprising the promoter operably linked to the polynucleotide encoding a Cas nuclease; or

(ii) the polynucleotide sequence comprising the gRNA that targets the chromogenic coding sequence is operably linked to the heterologous polynucleotide sequence comprising a homologous donor DNA sequence; or

(iii) the polynucleotide sequence comprising the gRNA that targets the chromogenic coding sequence and the heterologous polynucleotide sequence comprising a homologous donor DNA sequence are operably linked to the polynucleotide sequence comprising the promoter operably linked to the polynucleotide encoding a Cas nuclease; or

(iv) the heterologous polynucleotide sequence comprising the homologous donor DNA sequence comprises a mutation that prevents expression of the chromogenic protein or

(v) the heterologous polynucleotide sequence comprising the homologous donor DNA sequence comprises a mutation that modifies expression of the chromogenic protein from a first color to a second color.

6. The engineered microbial organism cell of claim 1, comprising:

a heterologous polynucleotide sequence comprising an engineered disruption in a sequence encoding a chromogenic protein that prevents expression of the functional chromogenic protein;

a heterologous polynucleotide sequence comprising a promoter operably linked to a polynucleotide encoding a Cas nuclease;

a heterologous polynucleotide sequence comprising a gRNA that targets the polynucleotide sequence comprising the engineered disruption in the sequence encoding the chromogenic protein; and

a heterologous polynucleotide sequence comprising a homologous donor DNA sequence for repairing the engineered disruption.

7. The engineered microbial organism cell of claim 1, comprising:

a first heterologous polynucleotide sequence comprising a first phenotype coding sequence operably linked to a first promoter, wherein the first phenotype coding sequence either (i) encodes a functional first protein having a first detectable phenotype, or (ii) comprises an engineered disruption in a sequence encoding a first protein that prevents expression of the first detectable phenotype;

a second heterologous polynucleotide sequence comprising a second phenotype coding sequence operably linked to a second promoter, wherein the second phenotype coding sequence either (i) encodes a functional second protein having a second detectable phenotype, or (ii) comprises an engineered disruption in a sequence encoding a second protein that prevents expression of the second detectable phenotype;

a third heterologous polynucleotide sequence comprising a third promoter operably linked to a polynucleotide encoding a Cas nuclease;

a fourth heterologous polynucleotide sequence comprising a first gRNA that targets the first phenotype coding sequence; and

a fifth heterologous polynucleotide sequence comprising a second gRNA that targets the second phenotype coding sequence.

8. The engineered microbial organism cell of claim 1, wherein the first or the second detectable phenotype is a detectable fluorescence, and wherein the phenotype coding sequence either (i) encodes a functional fluorescent protein, or (ii) comprises an engineered disruption in a sequence encoding a protein that prevents expression of the fluorescent protein, preferably the fluorescent protein is a coral fluorescent protein.

9. The engineered microbial organism cell of claim 7, wherein the first detectable phenotype and the second detectable phenotype is a detectable color and/or fluorescence, and wherein:

the first chromogenic coding sequence either (i) encodes a functional first chromogenic protein or (ii) comprises an engineered disruption in a sequence encoding a first chromogenic protein that prevents expression of the first chromogenic protein; and

the second chromogenic coding sequence either (i) encodes a functional second chromogenic protein or (ii) comprises an engineered disruption in a sequence encoding a second chromogenic protein that prevents expression of the second chromogenic protein.

10. The engineered microbial organism cell of any of claims 1 to 9, wherein one or more of the polynucleotide sequences further comprises an auxotrophic, antibiotic, or other selectable marker.

11. The engineered microbial organism cell of claim 7, wherein the fourth polynucleotide sequence further comprises a first auxotrophic, antibiotic, or other selectable marker and the fifth polynucleotide sequence further comprises a second auxotrophic, antibiotic, or other selectable marker, wherein the first auxotrophic, antibiotic, or other selectable marker and the second auxotrophic, antibiotic, or other selectable marker are different markers.

12. The engineered microbial organism cell of any of claims 1 to 11, wherein the polynucleotide sequence comprising a gRNA further comprises a homologous donor polynucleotide sequence.

13. The engineered microbial organism cell of any of claims 1 to 12, wherein the Cas nuclease is a Cas9 nuclease or a destabilized variant of Cas9, preferably the polynucleotide encoding the Cas nuclease is optimized for expression in the cell, most preferably

(i) the microbial organism cell is a yeast cell and the polynucleotide encoding the Cas nuclease encodes a Cas9 nuclease that is codon optimized for expression in yeast; or

(ii) the microbial organism cell is a bacterial cell and the polynucleotide encoding the Cas nuclease encodes a Cas9 nuclease that is codon optimized for expression in bacteria; or

(iii) the Cas nuclease is a *S. pyogenes* Cas9 nuclease.

**14.** The engineered microbial organism cell of claim 9, wherein

(i) the first chromogenic coding sequence encodes a functional first chromogenic protein and/or the second chromogenic coding sequence encodes a functional second chromogenic protein; or
(ii) the first coding sequence comprises an engineered disruption in a sequence encoding a first chromogenic protein that prevents expression of the first chromogenic protein, and/or the second coding sequence comprises an engineered disruption in a sequence encoding a second chromogenic protein that prevents expression of the second chromogenic protein; or
(iii) the first chromogenic coding sequence encodes a functional first chromogenic protein and the second coding sequence comprises an engineered disruption in a sequence encoding a second chromogenic protein that prevents expression of the second chromogenic protein; or
(iv) the first coding sequence comprises an engineered disruption in a sequence encoding a first chromogenic protein that prevents expression of the first chromogenic protein and the second chromogenic coding sequence encodes a functional second chromogenic protein.

**15.** The engineered microbial organism cell of any of claims 1 to 14, wherein

(i) the c microbial organism ell is a prokaryotic cell; or
(iii) the microbial organism cell is a eukaryotic cell, preferably the eukaryotic cell is a yeast cell.

**16.** An engineered microbial organism comprising the engineered microbial organism cell of any of claims 1 to 15, preferably the microbial organism is in lyophilized form.

**17.** A kit comprising:

the engineered microbial organism cell of any of claims 1 to 15 or the engineered microbial organism of claim 16; and
one or more reagents comprising culture medium, selective medium, media supplements, solid plating medium, plates, tubes, loops, or other plasticware,
preferably the kit further comprises one or more heterologous polynucleotide sequences comprising a homologous donor DNA.

**18.** The engineered microbial organism cell of any of claims 1 to 15, the engineered microbial organism of claim 16, and/or the kit of claim 17 which is/are for use in education and training.

**19.** A method of altering gene expression in a cell to generate a cell for use in education and training, the method comprising:
culturing the engineered microbial organism cell of any of claims 1 to 15 or the engineered microbial organism of claim 16 to form a population of engineered cells, wherein the culturing is performed under conditions that result in expression of

(i) the Cas nuclease in at least one engineered microbial organism cell, wherein the Cas nuclease cleaves an endogenous gene, genomic region, or phenotype coding sequence in the engineered microbial organism cell, thereby altering gene expression in at least one engineered cell, preferably the promoter that is operably linked to the polynucleotide encoding the Cas nuclease is an inducible promoter, and wherein the culturing is in the presence of an inducer to induce expression of the Cas nuclease in at least one engineered microbial organism cell; or
(ii) the lambda red in at least one engineered cell, wherein the lambda red catalyzes the homologous recombination of a donor DNA sequence at an endogenous gene, genomic region, or phenotype coding sequence in the engineered cell, thereby altering gene expression in at least one engineered cell, preferably the lambda red is under the control of an inducible promoter, and wherein the culturing is in the presence of an inducer to induce expression of the lambda red in at least one engineered cell.

**20.** A modular system for education and training an individual in laboratory procedures and the use of laboratory equipment for gene expression and gene editing, the modular system comprising:

(a) a module for expressing a detectable phenotype and/or for expressing a broken gene in a cell or organism, comprising the engineered microbial organism cell of any of claims 1 to 15 or the engineered microbial organism of claim 16 and further comprising one or more reagents for culturing reagents, transfecting, and/or transforming the cell or organism;

(b) a module for altering expression of the detectable phenotype and/or for repairing the broken gene in the cell or organism, comprising one or more reagents for expressing a Cas nuclease, expressing a gRNA, or preventing expression of a gRNA in the cell or organism;

(c) a module for analyzing the cell or organism, comprising one or more reagents for detecting the alteration of gene expression and/or repair of the broken gene in the cell or organism; and

(d) an instruction manual, comprising instructions for use of each of the modules, preferably the modular system further comprises an assessment module, comprising materials for evaluating a user's performance or skills in utilizing the modules (a), (b), and (c) and the instruction manual (d).


**Patentansprüche**

1. Gentechnisch veränderte Zelle eines mikrobischen Organismus, umfassend:

(a) ein endogenes Gen oder einen genomischen Bereich, der zur Gen-Veränderung angesteuert werden soll und der ein funktionelles Protein codiert, das einen ersten nachweisbaren Phänotyp aufweist, und eine heterologe Polynucleotidsequenz, die eine phänotypcodierende Sequenz codiert, welche funktionell mit einem Promotor verknüpft ist, der (i) ein zweites funktionelles Protein codiert, das einen zweiten nachweisbaren Phänotyp aufweist, oder (ii) eine gentechnisch erzeugte Disruption in einer Sequenz, die das zweite Protein codiert, umfasst, welche die Expression des zweiten nachweisbaren Phänotyps verhindert, oder eine oder mehrere heterologe Polynucleotidsequenzen, umfassend:

eine den ersten Phänotyp codierende Sequenz, die funktionell mit einem Promotor verknüpft ist, wobei die den ersten Phänotyp codierende Sequenz entweder (i) ein erstes funktionelles Protein mit einem ersten nachweisbaren Phänotyp codiert oder (ii) eine gentechnisch erzeugte Disruption in einer Sequenz umfasst, die das Protein codiert, welches die Expression des ersten nachweisbaren Phänotyps verhindert; und eine den zweiten Phänotyp codierende Sequenz, die funktionell mit einem Promotor verknüpft ist, wobei die den zweiten Phänotyp codierende Sequenz entweder (i) ein zweites funktionelles Protein mit einem zweiten nachweisbaren Phänotyp codiert oder (ii) eine gentechnisch erzeugte Disruption in einer Sequenz umfasst, die das zweite Protein codiert, welches die Expression des zweiten nachweisbaren Phänotyps verhindert;
wobei es sich bei dem ersten und dem zweiten nachweisbaren Phänotyp jeweils um eine nachweisbare Farbe, Fluoreszenz, Geruch, enzymatische Aktivität, Antibiotikumresistenz, Morphologie oder Letalität handelt;

(b) eine heterologe Polynucleotidsequenz, die einen Promotor umfasst, der funktionell mit einem Polynucleotid verknüpft ist, das eine Cas-Nuclease codiert, und eine heterologe Polynucleotidsequenz, die eine Guide-RNA (gRNA), welche die den ersten Phänotyp codierende Sequenz ansteuert, eine gRNA, welche die den zweiten Phänotyp codierende Sequenz ansteuert, umfasst; und eine heterologe Polynucleotidsequenz, die einen Promotor umfasst, der funktionell mit einem Polynucleotid verknüpft ist, das Lambda-Red codiert.

2. Gentechnisch veränderte Zelle eines mikrobischen Organismus gemäß Anspruch 1, wobei (b) weiterhin eine erste und/oder zweite homologe Spender-DNA-Sequenz umfasst.

3. Gentechnisch veränderte Zelle eines mikrobischen Organismus gemäß Anspruch 1 oder 2, umfassend

(a) ein endogenes Gen oder einen genomischen Bereich, der zur Gen-Veränderung angesteuert werden soll, wobei das endogene Gen oder der genomische Bereich, der zur Gen-Veränderung angesteuert werden soll, ein funktionelles Gen oder genomischer Bereich ist und die Spender-DNA-Sequenz eine Sequenz ist, die das funktionelle Gen oder den funktionellen genomischen Bereich unterbricht, oder eine Sequenz ist, die das funktionelle Gen oder den funktionellen genomischen Bereich durch ein anderes funktionelles Gen oder einen anderen funktionellen genomischen Bereich ersetzt; oder

(b) das endogene Gen oder der genomische Bereich, der angesteuert werden soll, ein unterbrochenes Gen oder genomischer Bereich ist und wobei die Spender-DNA-Sequenz eine Sequenz ist, die die Funktion des Gens oder genomischen Bereichs wiederherstellt.

4.  Gentechnisch veränderte Zelle eines mikrobischen Organismus gemäß einem der Ansprüche 1 bis 3, umfassend eine oder mehrere heterologe Polynucleotidsequenzen, die eine phänotypcodierende Sequenz umfassen, welche mit einem Promotor funktionell verknüpft ist, wobei die phänotypcodierende Sequenz ein funktionelles Protein mit einem nachweisbaren Phänotyp codiert.

5.  Gentechnisch veränderte Zelle eines mikrobischen Organismus gemäß Anspruch 1, umfassend:

    eine heterologe Polynucleotidsequenz, die eine chromogencodierende Sequenz umfasst, die ein chromogenes funktionelles Protein codiert;
    eine heterologe Polynucleotidsequenz, die einen Promotor umfasst, der funktionell mit einem Polynucleotid verknüpft ist, das eine Cas-Nuclease codiert; eine heterologe Polynucleotidsequenz, die eine gRNA, welche die chromogencodierende Sequenz ansteuert, umfasst; und eine heterologe Polynucleotidsequenz, die eine homologe Spender-DNA-Sequenz umfasst, wobei vorzugsweise
    (i) die Polynucleotidsequenz, die die gRNA umfasst, welche die chromogencodierende Sequenz ansteuert, funktionell mit der Polynucleotidsequenz verknüpft ist, die den Promotor umfasst, der funktionell mit dem eine Cas-Nuclease codierenden Polynucleotid verknüpft ist; oder
    (ii) die Polynucleotidsequenz, die die gRNA umfasst, welche die chromogencodierende Sequenz ansteuert, funktionell mit der heterologen Polynucleotidsequenz verknüpft ist, die eine homologe Spender-DNA-Sequenz umfasst; oder
    (iii) die Polynucleotidsequenz, die die gRNA umfasst, welche die chromogencodierende Sequenz ansteuert, und die heterologe Polynucleotidsequenz, die eine homologe Spender-DNA-Sequenz umfasst, funktionell mit der Polynucleotidsequenz verknüpft sind, die den Promotor umfasst, der funktionell mit dem eine Cas-Nuclease codierenden Polynucleotid verknüpft ist; oder
    (iv) die heterologe Polynucleotidsequenz, die die homologe Spender-DNA-Sequenz umfasst, eine Mutation umfasst, die die Expression des chromogenen Proteins verhindert, oder
    (v) die heterologe Polynucleotidsequenz, die die homologe Spender-DNA-Sequenz umfasst, eine Mutation umfasst, die die Expression des chromogenen Proteins von einer ersten Farbe zu einer zweiten Farbe modifiziert.

6.  Gentechnisch veränderte Zelle eines mikrobischen Organismus gemäß Anspruch 1, umfassend:

    eine heterologe Polynucleotidsequenz, die eine gentechnisch erzeugte Disruption in einer Sequenz, die ein chromogenes Protein codiert, umfasst, welche die Expression des funktionellen chromogenen Proteins verhindert;
    eine heterologe Polynucleotidsequenz, die einen Promotor umfasst, der funktionell mit einem Polynucleotid verknüpft ist, das eine Cas-Nuclease codiert;
    eine heterologe Polynucleotidsequenz, die eine gRNA umfasst, welche die Polynucleotidsequenz ansteuert, die die gentechnisch erzeugte Disruption in der Sequenz, die das chromogene Protein codiert, umfasst; und
    eine heterologe Polynucleotidsequenz, die eine homologe Spender-DNA-Sequenz umfasst, um die gentechnisch erzeugte Disruption zu reparieren.

7.  Gentechnisch veränderte Zelle eines mikrobischen Organismus gemäß Anspruch 1, umfassend:

    eine erste heterologe Polynucleotidsequenz, die eine erste phänotypcodierende Sequenz umfasst, welche mit einem ersten Promotor funktionell verknüpft ist, wobei die erste phänotypcodierende Sequenz entweder (i) ein erstes funktionelles Protein mit einem ersten nachweisbaren Phänotyp codiert oder (ii) eine gentechnisch erzeugte Disruption in einer Sequenz umfasst, die ein erstes Protein codiert, welches die Expression des ersten nachweisbaren Phänotyps verhindert;
    eine zweite heterologe Polynucleotidsequenz, die eine zweite phänotypcodierende Sequenz umfasst, welche mit einem zweiten Promotor funktionell verknüpft ist, wobei die zweite phänotypcodierende Sequenz entweder (i) ein zweites funktionelles Protein mit einem zweiten nachweisbaren Phänotyp codiert oder (ii) eine gentechnisch erzeugte Disruption in einer Sequenz umfasst, die ein zweites Protein codiert, welches die Expression des zweiten nachweisbaren Phänotyps verhindert;
    eine dritte heterologe Polynucleotidsequenz, die einen dritten Promotor umfasst, der funktionell mit einem Polynucleotid verknüpft ist, das eine Cas-Nuclease codiert;
    eine vierte heterologe Polynucleotidsequenz, die eine erste gRNA, welche die erste phänotypcodierende Sequenz ansteuert, umfasst; und
    eine fünfte heterologe Polynucleotidsequenz, die eine zweite gRNA, welche die zweite phänotypcodierende Sequenz ansteuert, umfasst.

**8.** Gentechnisch veränderte Zelle eines mikrobischen Organismus gemäß Anspruch 1, wobei der erste oder der zweite nachweisbare Phänotyp eine nachweisbare Fluoreszenz ist und wobei die phänotypcodierende Sequenz entweder (i) ein fluoreszierendes funktionelles Protein codiert oder (ii) eine gentechnisch erzeugte Disruption in einer Sequenz umfasst, die ein Protein codiert, welches die Expression des fluoreszierenden Proteins verhindert, wobei vorzugsweise das fluoreszierende Protein ein fluoreszierendes Korallenprotein ist.

**9.** Gentechnisch veränderte Zelle eines mikrobischen Organismus gemäß Anspruch 7, wobei der erste nachweisbare Phänotyp und der zweite nachweisbare Phänotyp eine nachweisbare Farbe und/oder Fluoreszenz sind und wobei:

die erste chromogencodierende Sequenz entweder (i) ein erstes funktionelles chromogenes Protein codiert oder (ii) eine gentechnisch erzeugte Disruption in einer Sequenz, die ein erstes chromogenes Protein codiert, umfasst, welche die Expression des ersten chromogenen Proteins verhindert; und
die zweite chromogencodierende Sequenz entweder (i) ein zweites funktionelles chromogenes Protein codiert oder (ii) eine gentechnisch erzeugte Disruption in einer Sequenz, die ein zweites chromogenes Protein codiert, umfasst, welche die Expression des zweiten chromogenen Proteins verhindert.

**10.** Gentechnisch veränderte Zelle eines mikrobischen Organismus gemäß einem der Ansprüche 1 bis 9, wobei eine oder mehrere der Polynucleotidsequenzen weiterhin einen auxotrophen-, antibiotischen- oder anderen selektierbaren Marker umfassen.

**11.** Gentechnisch veränderte Zelle eines mikrobischen Organismus gemäß Anspruch 7, wobei die vierte Polynucleotidsequenz weiterhin einen ersten auxotrophen-, antibiotischen- oder anderen selektierbaren Marker umfasst und die fünfte Polynucleotidsequenz weiterhin einen zweiten auxotrophen-, antibiotischen- oder anderen selektierbaren Marker umfasst, wobei der erste auxotrophen-, antibiotischen- oder andere selektierbare Marker und der zweite auxotrophen-, antibiotischen- oder andere selektierbare Marker unterschiedliche Marker sind.

**12.** Gentechnisch veränderte Zelle eines mikrobischen Organismus gemäß einem der Ansprüche 1 bis 11, wobei die Polynucleotidsequenz, die eine gRNA umfasst, weiterhin eine homologe Spender-Polynucleotidsequenz umfasst.

**13.** Gentechnisch veränderte Zelle eines mikrobischen Organismus gemäß einem der Ansprüche 1 bis 12, wobei die Cas-Nuclease eine Cas9-Nuclease oder eine destabilisierte Variante von Cas9 ist, wobei vorzugsweise das Polynucleotid, das die Cas-Nuclease codiert, für die Expression in der Zelle optimiert ist, wobei am meisten bevorzugt

(i) die Zelle des mikrobischen Organismus eine Hefezelle ist und das Polynucleotid, das die Cas-Nuclease codiert, eine Cas9-Nuclease codiert, die für die Expression in Hefe codonoptimiert ist; oder
(ii) die Zelle des mikrobischen Organismus eine Bakterienzelle ist und das Polynucleotid, das die Cas-Nuclease codiert, eine Cas9-Nuclease codiert, die für die Expression in Bakterien codonoptimiert ist; oder
(iii) die Cas-Nuclease eine S.-pyogenes-Cas9-Nuclease ist.

**14.** Gentechnisch veränderte Zelle eines mikrobischen Organismus gemäß Anspruch 9, wobei

(i) die erste chromogencodierende Sequenz ein erstes chromogenes funktionelles Protein codiert und/oder die zweite chromogencodierende Sequenz ein zweites chromogenes funktionelles Protein codiert; oder
(ii) die erste codierende Sequenz eine gentechnisch erzeugte Disruption in einer Sequenz, die ein erstes chromogenes Protein codiert, umfasst, welche die Expression des ersten chromogenen Proteins verhindert, und/oder die zweite codierende Sequenz eine gentechnisch erzeugte Disruption in einer Sequenz, die ein zweites chromogenes Protein codiert, umfasst, welche die Expression des zweiten chromogenen Proteins verhindert; oder
(iii) die erste chromogencodierende Sequenz ein erstes chromogenes funktionelles Protein codiert und die zweite codierende Sequenz eine gentechnisch erzeugte Disruption in einer Sequenz, die ein zweites chromogenes Protein codiert, umfasst, welche die Expression des zweiten chromogenen Proteins verhindert; oder
(iv) die erste codierende Sequenz eine gentechnisch erzeugte Disruption in einer Sequenz, die ein erstes chromogenes Protein codiert, umfasst, welche die Expression des ersten chromogenen Proteins verhindert, und die zweite chromogencodierende Sequenz ein zweites chromogenes funktionelles Protein codiert.

**15.** Gentechnisch veränderte Zelle eines mikrobischen Organismus gemäß einem der Ansprüche 1 bis 14, wobei

(i) die Zelle des mikrobischen Organismus eine prokaryontische Zelle ist; oder
(iii) die Zelle des mikrobischen Organismus eine eukaryontische Zelle ist, wobei vorzugsweise die eukaryontische Zelle eine Hefezelle ist.

16. Gentechnisch veränderter mikrobischer Organismus, umfassend die gentechnisch veränderte Zelle des mikrobischen Organismus gemäß einem der Ansprüche 1 bis 15, wobei vorzugsweise der mikrobische Organismus in lyophilisierter Form vorliegt.

17. Kit, umfassend:

die gentechnisch veränderte Zelle des mikrobischen Organismus gemäß einem der Ansprüche 1 bis 15 oder den gentechnisch veränderten mikrobischen Organismus gemäß Anspruch 16; und
ein oder mehrere Reagentien, die ein Kulturmedium, Selektionsmedium, Mediumergänzungen, festes Ausstrichmedium, Platten, Röhrchen, Schleifen oder andere Kunststoffware umfassen,
wobei vorzugsweise der Kit weiterhin eine oder mehrere heterologe Polynucleotidsequenzen, die eine homologe Spender-DNA umfassen, umfasst.

18. Gentechnisch veränderte Zelle des mikrobischen Organismus gemäß einem der Ansprüche 1 bis 15, gentechnisch veränderter mikrobischer Organismus gemäß Anspruch 16 und/oder Kit gemäß Anspruch 17 zur Verwendung in Bildung und Ausbildung.

19. Verfahren zur Veränderung der Genexpression in einer Zelle unter Erzeugung einer Zelle zur Verwendung in Bildung und Ausbildung, wobei das Verfahren umfasst:
Kultivieren der gentechnisch veränderten Zelle des mikrobischen Organismus gemäß einem der Ansprüche 1 bis 15 oder des gentechnisch veränderten mikrobischen Organismus gemäß Anspruch 16 unter Bildung einer Population von gentechnisch veränderten Zellen, wobei das Kultivieren unter Bedingungen durchgeführt wird, die zur Expression der folgenden Gene führen:

(i) der Cas-Nuclease in wenigstens einer gentechnisch veränderten Zelle des mikrobischen Organismus, wobei die Cas-Nuclease ein endogenes Gen, einen genomischen Bereich oder eine phänotypcodierende Sequenz in der gentechnisch veränderten Zelle des mikrobischen Organismus spaltet, wodurch die Gen-Expression in wenigstens einer gentechnisch veränderten Zelle verändert wird, vorzugsweise der Promotor, der funktionell mit dem Polynucleotid, das die Cas-Nuclease codiert, verknüpft ist, ein induzierbarer Promotor ist und wobei das Kultivieren in Gegenwart eines Induktors erfolgt, um die Expression der Cas-Nuclease in wenigstens einer gentechnisch veränderten Zelle des mikrobischen Organismus zu induzieren; oder
(ii) von Lambda-Red in wenigstens einer gentechnisch veränderten Zelle, wobei das Lambda-Red die homologe Rekombination einer Spender-DNA-Sequenz mit einem endogenen Gen, einem genomischen Bereich oder einer phänotypcodierenden Sequenz in der gentechnisch veränderten Zelle katalysiert, wodurch die Gen-Expression in wenigstens einer gentechnisch veränderten Zelle verändert wird, vorzugsweise das Lambda-Red unter der Kontrolle eines induzierbaren Promotors steht, und wobei das Kultivieren in Gegenwart eines Induktors erfolgt, um die Expression von Lambda-Red in wenigstens einer gentechnisch veränderten Zelle zu induzieren.

20. Modulares System zur Bildung und Ausbildung eines Individuums in Laborverfahren und der Verwendung von Laborgeräten für Gen-Expression und Gen-Editing, wobei das modulare System umfasst:

(a) ein Modul zum Exprimieren eines nachweisbaren Phänotyps und/oder zum Exprimieren eines defekten Gens in einer Zelle oder einem Organismus, umfassend die gentechnisch veränderte Zelle des mikrobischen Organismus gemäß einem der Ansprüche 1 bis 15 oder den gentechnisch veränderten mikrobischen Organismus gemäß Anspruch 16 und weiterhin umfassend ein oder mehrere Reagentien zum Kultivieren, Transfizieren und/oder Transformieren der Zelle oder des Organismus;
(b) ein Modul zum Verändern der Expression des nachweisbaren Phänotyps und/oder zum Reparieren des defekten Gens in der Zelle oder dem Organismus, umfassend ein oder mehrere Reagentien zum Exprimieren einer Cas-Nuclease, zum Exprimieren einer gRNA oder zur Verhinderung der Expression einer gRNA in der Zelle oder dem Organismus;
(c) ein Modul zum Analysieren der Zelle oder des Organismus, umfassend ein oder mehrere Reagentien zum Nachweisen der Veränderung der Gen-Expression und/oder der Reparatur des defekten Gens in der Zelle oder dem Organismus; und
(d) ein Anleitungshandbuch, das Anweisungen zur Verwendung jedes der Module umfasst,

wobei vorzugsweise das modulare System weiterhin ein Bewertungsmodul umfasst, das Materialien zur Bewertung der Leistungen oder Fähigkeiten eines Anwenders bei der Benutzung der Module (a), (b) und (c) und des Anleitungshandbuchs (d) umfasst.

**Revendications**

1. Cellule d'organisme microbien manipulée comprenant :

(a) un gène endogène ou une région génomique à cibler qui code pour une protéine fonctionnelle ayant un premier phénotype détectable, et une séquence polynucléotidique hétérologue codant pour une séquence codant pour un phénotype fonctionnellement liée à un promoteur qui (i) code pour une seconde protéine fonctionnelle ayant un second phénotype détectable, ou (ii) comprend une disruption manipulée dans une séquence codant pour la seconde protéine qui empêche l'expression du second phénotype détectable pour une altération génique ou
une ou plusieurs séquences polynucléotidiques hétérologues comprenant :

une séquence codant pour un premier phénotype fonctionnellement liée à un promoteur, dans laquelle la séquence codant pour un premier phénotype (i) code pour une première protéine fonctionnelle ayant un premier phénotype détectable, ou (ii) comprend une disruption manipulée dans une séquence codant pour une protéine qui empêche l'expression du premier phénotype détectable ; et
une séquence codant pour un second phénotype fonctionnellement liée à un promoteur, où la séquence codant pour un second phénotype (i) code pour une seconde protéine fonctionnelle ayant un second phénotype détectable, ou (ii) comprend une disruption manipulée dans une séquence codant pour la seconde protéine qui empêche l'expression du second phénotype détectable ; dans laquelle chacun des premier et second phénotypes détectables est une couleur, une fluorescence, un parfum, une activité enzymatique, une résistance aux antibiotiques, une morphologie ou une létalité détectable ;

(b) une séquence polynucléotidique hétérologue comprenant un promoteur fonctionnellement lié à un polynucléotide codant pour une nucléase Cas et une séquence polynucléotidique hétérologue comprenant un ARN guide (ARNg) qui cible la séquence codant pour un premier phénotype, un ARNg qui cible la séquence codant pour le second phénotype ; et une séquence polynucléotidique hétérologue comprenant un promoteur fonctionnellement lié à un polynucléotide codant pour le système lambda-red.

2. Cellule d'organisme microbien manipulée de la revendication 1, dans laquelle (b) comprend en outre une première et/ou une seconde séquence d'ADN donneur homologue.

3. Cellule d'organisme microbien manipulée de la revendication 1 ou 2, comprenant

(a) un gène endogène ou une région génomique à cibler pour une altération génique, où le gène endogène ou la région génomique à cibler pour une altération génique est un gène ou une région génomique fonctionnel(le) et la séquence d'ADN donneur est une séquence qui perturbe le gène ou la région génomique fonctionnel(le) ou est une séquence qui remplace le gène ou la région génomique fonctionnel(le) par un gène fonctionnel différent ou une région génomique fonctionnelle différente ; ou
(b) le gène endogène ou la région génomique à cibler est un gène ou une région génomique perturbé(e), et où la séquence d'ADN donneur est une séquence qui restaure la fonction du gène ou de la région génomique.

4. Cellule d'organisme microbien manipulée de l'une des revendications 1 à 3, comprenant une ou plusieurs séquences polynucléotidiques hétérologues comprenant une séquence codant pour un phénotype fonctionnellement liée à un promoteur, où la séquence codant pour un phénotype code pour une protéine fonctionnelle ayant un phénotype détectable.

5. Cellule d'organisme microbien manipulée de la revendication 1, comprenant :

une séquence polynucléotidique hétérologue comprenant une séquence codante chromogène qui code pour une protéine chromogène fonctionnelle ;
une séquence polynucléotidique hétérologue comprenant un promoteur fonctionnellement lié à un polynucléotide codant pour une nucléase Cas ; une séquence polynucléotidique hétérologue comprenant un ARNg qui cible la séquence codante chromogène ; et une séquence polynucléotidique hétérologue comprenant une séquence d'ADN donneur homologue, de préférence

(i) la séquence polynucléotidique comprenant l'ARNg qui cible la séquence codante chromogène est fonctionnellement liée à la séquence polynucléotidique comprenant le promoteur fonctionnellement liée au polynucléotide codant pour une nucléase Cas ; ou

(ii) la séquence polynucléotidique comprenant l'ARNg qui cible la séquence codante chromogène est fonctionnellement liée à la séquence polynucléotidique hétérologue comprenant une séquence d'ADN donneur homologue ; ou

(iii) la séquence polynucléotidique comprenant l'ARNg qui cible la séquence codante chromogène et la séquence polynucléotidique hétérologue comprenant une séquence d'ADN donneur homologue sont liées de manière opérationnelle à la séquence polynucléotidique comprenant le promoteur fonctionnellement lié au polynucléotide codant pour une nucléase Cas ; ou

(iv) la séquence polynucléotidique hétérologue comprenant la séquence d'ADN donneur homologue comprend une mutation qui empêche l'expression de la protéine chromogène, ou

(v) la séquence polynucléotidique hétérologue comprenant la séquence d'ADN donneur homologue comprend une mutation qui modifie l'expression de la protéine chromogène d'une première couleur en une seconde couleur.

6. Cellule d'organisme microbien manipulée de la revendication 1, comprenant :

une séquence polynucléotidique hétérologue comprenant une disruption manipulée dans une séquence codant pour une protéine chromogène qui empêche l'expression de la protéine chromogène fonctionnelle ;
une séquence polynucléotidique hétérologue comprenant un promoteur fonctionnellement lié à un polynucléotide codant pour une nucléase Cas ;
une séquence polynucléotidique hétérologue comprenant un ARNg qui cible la séquence polynucléotidique comprenant la disruption manipulée dans la séquence codant pour la protéine chromogène ; et
une séquence polynucléotidique hétérologue comprenant une séquence d'ADN donneur homologue pour réparer la disruption manipulée.

7. Cellule d'organisme microbien manipulée de la revendication 1, comprenant :

une première séquence polynucléotidique hétérologue comprenant une séquence codant pour un premier phénotype fonctionnellement liée à un premier promoteur, où la séquence codant pour un premier phénotype (i) code pour une première protéine fonctionnelle ayant un premier phénotype détectable, ou (ii) comprend une disruption manipulée dans une séquence codant pour une première protéine qui empêche l'expression du premier phénotype détectable ;
une deuxième séquence polynucléotidique hétérologue comprenant une séquence codant pour un second phénotype fonctionnellement liée à un deuxième promoteur, où la séquence codant pour un second phénotype (i) code pour une seconde protéine fonctionnelle ayant un second phénotype détectable, ou (ii) comprend une disruption manipulée dans une séquence codant pour une seconde protéine qui empêche l'expression du second phénotype détectable ;
une troisième séquence polynucléotidique hétérologue comprenant un troisième promoteur fonctionnellement lié à un polynucléotide codant pour une nucléase Cas ;
une quatrième séquence polynucléotidique hétérologue comprenant un premier ARNg qui cible la séquence codant pour un premier phénotype ; et
une cinquième séquence polynucléotidique hétérologue comprenant un second ARNg qui cible la séquence codant pour un second phénotype.

8. Cellule d'organisme microbien manipulée de la revendication 1, dans laquelle le premier ou le second phénotype détectable est une fluorescence détectable, et dans laquelle la séquence codant pour un phénotype (i) code pour une protéine fluorescente fonctionnelle, ou
(ii) comprend une disruption manipulée dans une séquence codant pour une protéine qui empêche l'expression de la protéine fluorescente et, de préférence, la protéine fluorescente est une protéine fluorescente de corail.

9. Cellule d'organisme microbien manipulée de la revendication 7, dans laquelle le premier phénotype détectable et le second phénotype détectable sont une couleur et/ou une fluorescence détectables, et dans laquelle :

la première séquence codante chromogène (i) code pour une première protéine chromogène fonctionnelle, ou
(ii) comprend une disruption manipulée dans une séquence codant pour une première protéine chromogène qui empêche l'expression de la première protéine chromogène ; et
la seconde séquence codante chromogène (i) code pour une seconde protéine chromogène fonctionnelle, ou (ii) comprend une disruption manipulée dans une séquence codant pour une seconde protéine chromogène qui empêche l'expression de la seconde protéine chromogène.

10. Cellule d'organisme microbien manipulée de l'une des revendications 1 à 9, dans laquelle une ou plusieurs des séquences polynucléotidiques comprennent en outre un marqueur auxotrophe, antibiotique ou un autre marqueur sélectionnable.

11. Cellule d'organisme microbien manipulée de la revendication 7, dans laquelle la quatrième séquence polynucléotidique comprend en outre un premier marqueur auxotrophe, antibiotique ou un autre marqueur sélectionnable et la cinquième séquence polynucléotidique comprend en outre un second marqueur auxotrophe, antibiotique ou un autre marqueur sélectionnable, où le premier marqueur auxotrophe, antibiotique ou autre marqueur sélectionnable et le second marqueur auxotrophe, antibiotique ou autre marqueur sélectionnable sont des marqueurs différents.

12. Cellule d'organisme microbien manipulée de l'une des revendications 1 à 11, dans laquelle la séquence polynucléotidique comprenant un ARNg comprend en outre une séquence polynucléotidique donneur homologue.

13. Cellule d'organisme microbien manipulée de l'une des revendications 1 à 12, dans laquelle la nucléase Cas est une nucléase Cas9 ou un variant déstabilisé de Cas9, de préférence le polynucléotide codant pour la nucléase Cas est optimisé pour une expression dans la cellule, de manière la plus préférée

(i) la cellule d'organisme microbien est une cellule de levure et le polynucléotide codant pour la nucléase Cas code pour une nucléase Cas9 dont un codon est optimisé pour une expression dans une levure ; ou
(ii) la cellule d'organisme microbien est une cellule bactérienne et le polynucléotide codant pour la nucléase Cas code pour une nucléase Cas9 dont un codon est optimisé pour une expression dans des bactéries ; ou
(iii) la nucléase Cas est une nucléase Cas9 de *S. pyogenes.*

14. Cellule d'organisme microbien manipulée de la revendication 9, dans laquelle

(i) la première séquence codante chromogène code pour une première protéine chromogène fonctionnelle et/ou la seconde séquence codante chromogène code pour une seconde protéine chromogène fonctionnelle ; ou
(ii) la première séquence codante comprend une disruption manipulée dans une séquence codant pour une première protéine chromogène qui empêche l'expression de la première protéine chromogène, et/ou la seconde séquence codante comprend une disruption manipulée dans une séquence codant pour une seconde protéine chromogène qui empêche l'expression de la seconde protéine chromogène ; ou
(iii) la première séquence codante chromogène code pour une première protéine chromogène fonctionnelle et la seconde séquence codante comprend une disruption manipulée dans une séquence codant pour une seconde protéine chromogène qui empêche l'expression de la seconde protéine chromogène ; ou
(iv) la première séquence codante comprend une disruption manipulée dans une séquence codant pour une première protéine chromogène qui empêche l'expression de la première protéine chromogène et la seconde séquence codante chromogène code pour une seconde protéine chromogène fonctionnelle.

15. Cellule d'organisme microbien manipulée de l'une des revendications 1 à 14, dans laquelle

(i) la cellule d'organisme microbien est une cellule procaryote ; ou
(iii) la cellule d'organisme microbien est une cellule eucaryote, de préférence la cellule eucaryote est une cellule de levure.

16. Organisme microbien manipulé comprenant la cellule d'organisme microbien manipulée de l'une des revendications 1 à 15, et de préférence l'organisme microbien se présente sous une forme lyophilisée.

17. Kit comprenant :

la cellule d'organisme microbien manipulée de l'une des revendications 1 à 15 ou l'organisme microbien manipulé de la revendication 16 ; et
un ou plusieurs réactifs comprenant un milieu de culture, un milieu sélectif, des suppléments de milieux, un milieu d'étalement solide, des plaques, des tubes, des boucles ou d'autres ustensiles en matière plastique,

de préférence, le kit comprend en outre une ou plusieurs séquences polynucléotidiques hétérologues comprenant un ADN donneur homologue.

**18.** Cellule d'organisme microbien manipulée de l'une des revendications 1 à 15, organisme microbien manipulé de la revendication 16, et/ou kit de la revendication 17 qui est/sont destinés à être utilisés dans l'enseignement et la formation.

**19.** Procédé d'altération de l'expression d'un gène dans une cellule pour générer une cellule destinée à être utilisée dans l'enseignement et la formation, le procédé comprenant :
la culture de la cellule d'organisme microbien manipulée de l'une des revendications 1 à 15 ou de l'organisme microbien manipulé de la revendication 16 pour former une population de cellules manipulées, où la culture est effectuée dans des conditions qui entraînent l'expression de

(i) la nucléase Cas dans au moins une cellule d'organisme microbien manipulée, où la nucléase Cas clive un gène endogène, une région génomique ou une séquence codant pour un phénotype dans la cellule d'organisme microbien manipulée, altérant ainsi l'expression génique dans au moins une cellule manipulée, de préférence le promoteur qui est fonctionnellement lié au polynucléotide codant pour la nucléase Cas est un promoteur inductible, et où la culture se déroule en présence d'un inducteur permettant d'induire l'expression de la nucléase Cas dans au moins une cellule d'organisme microbien manipulée ; ou
(ii) le système lambda-red dans au moins une cellule manipulée, où le système lambda-red catalyse la recombinaison homologue d'une séquence d'ADN donneur au niveau d'un gène endogène, d'une région génomique ou d'une séquence codant pour un phénotype dans la cellule manipulée, altérant de ce fait l'expression génique dans au moins une cellule manipulée, de préférence le système lambda-red se trouve sous la commande d'un promoteur inductible, et où la culture se déroule en présence d'un inducteur pour induire l'expression du système lambda-red dans au moins une cellule manipulée.

**20.** Système modulaire d'enseignement et de formation d'un individu aux procédures de laboratoire et à l'utilisation d'équipement de laboratoire pour une expression génique et une édition génique, le système modulaire comprenant :

(a) un module permettant d'exprimer un phénotype détectable et/ou permettant d'exprimer un gène cassé dans une cellule ou un organisme, comprenant la cellule d'organisme microbien manipulée de l'une des revendications 1 à 15 ou l'organisme microbien manipulé selon la revendication 16 et comprenant en outre un ou plusieurs réactifs permettant de cultiver des réactifs, transfecter et/ou transformer la cellule ou l'organisme ;
(b) un module permettant d'altérer l'expression du phénotype détectable et/ou de réparer le gène cassé dans la cellule ou l'organisme, comprenant un ou plusieurs réactifs pour exprimer une nucléase Cas, exprimer un ARNg ou empêcher l'expression d'un ARNg dans la cellule ou l'organisme ;
(c) un module permettant d'analyser la cellule ou l'organisme, comprenant un ou plusieurs réactifs pour détecter l'altération de l'expression génique et/ou la réparation du gène cassé dans la cellule ou l'organisme ; et
(d) un manuel d'instructions, comprenant des instructions d'utilisation de chacun des modules, de préférence, le système modulaire comprend en outre un module d'évaluation, comprenant des matériels pour évaluer les performances ou les compétences d'un utilisateur à utiliser les modules (a), (b), et (c) et le manuel d'instructions (d).

FIG. 1

*FIG. 2*

Yeast Version 2  FIG. 3

Yeast Version 3

**FIG. 4**

Constitutive promoter — yEmRFP

Native promoter — ADE2*

Galactose-inducible promoter — yeast optimized Cas9

gRNA A — URA3

gRNA B — LYS2

No selection or induction → No Cas9, gRNA A, gRNA B → yEmRFP (purple)

Galactose induction only → Cas9, gRNA A, gRNA B → Red color

Galactose plus 5-FOA → Cas9, gRNA B → yEmRFP plus red (light purple)

Galactose plus alpha AA → Cas9, gRNA A → white

*ADE2 (white)
ade2 (red)

Yeast Version 4

**FIG. 5**

Constitutive promoter — Chromogenic protein A

Native promoter — A | DE2*

Galactose-inducible promoter — yeast optimized Cas9

gRNA A
+mutant HR donor
URA3

gRNA B/C
+repair HR donor
LYS2

No selection or induction → No Cas9 gRNA A gRNA B/C → Chromogenic protein A plus red

Galactose induction only → Cas9 gRNA A gRNA B/C → white

Galactose plus 5-FOA → Cas9 gRNA B/C → Chromogenic protein A

Galactose plus alpha AA → Cas9 gRNA A → red

*ADE2 (white)
ade2 (red)

EP 3 728 581 B1

# FIG. 6
## Bacteria version 1

**Already present in bacteria:**

Constitutive or inducible promoter — GFP

**Transformed into bacteria:**

Inducible promoter — E coli optimized Cas9 — gRNA — mutant HR donor (disrupt GFP)

gRNA version 1 ⟶ No GFP expression

EP 3 728 581 B1

# FIG. 7

Bacteria version 2

gRNA version 2 ⟶ BFP or YFP expression

EP 3 728 581 B1

# FIG. 8

## Bacteria version 3

**Already present in bacteria:**

Constitutive or inducible promoter — GFP

**Transformed into bacteria:**

Inducible promoter — E coli optimized Cas9 — gRNA  +  mutant HR donor (disrupt GFP)

gRNA version 3 ⟶ No GFP expression

EP 3 728 581 B1

# FIG. 9

Bacteria version 4

**Already present in bacteria:**

Constitutive or inducible promoter → GFP

**Transformed into bacteria:**

Inducible promoter → E coli optimized Cas9 → gRNA + mutant HR donor (GFP to BFP or YFP)

gRNA version 4 ⟶ BFP or YFP expression

# FIG. 10

Bacteria version 5

**Already present in bacteria:**

Constitutive or inducible promoter — G | FP

**Transformed into bacteria:**

Inducible promoter — E coli optimized Cas9 — gRNA — Wild type HR donor

gRNA version 5 ⟶ Restore GFP expression

EP 3 728 581 B1

# FIG. 11

## Bacteria version 6

**Already present in bacteria:**

Constitutive or inducible promoter

G    FP

**Transformed into bacteria:**

Inducible promoter — E coli optimized Cas9    gRNA    +    ssDNA Wild type HR donor

gRNA version 6 ⟶ Restore GFP expression

EP 3 728 581 B1

## FIG. 12

Bacteria version 7

# FIG. 13

## Bacteria version 8

**Already present in bacteria:**

Chromogenic Protein — Cas9 — Ku/ LigD

**Transformed into bacteria:**

gRNA-CP

1. CRISPR makes double strand break within Chromogenic protein
2. Error prone NHEJ DNA repair mediated by Ku-LigD

**Altered bacterial genome:**

Chr...Protein — Cas9 — Ku/ LigD

Key: Constitutive Promoter   Inducible Promoter   Cassette/ORF   Reading frame disruption

# FIG. 14

## Bacteria version 9

Key: Constitutive Promoter | Inducible Promoter | Cassette/ORF

# FIG. 15A-15B

FIG. 15A                                    HB101 with Cas9, Lambda Red

| Electroporation | CAM colonies | Amp/Kan colonies |
|---|---|---|
| gRNA, donorDNA | >200 | >300 (lawn) |
| Water | 0 | >300 (lawn) |

FIG. 15B

Chromosomal LacZ —— [ LacZ ] —— ~450 bp PCR

donorDNA with
homology arms to LacZ    [ | CAM | ]

Chromosomal LacZ —— [ La | CAM | cZ ] —— ~1300 bp PCR
with donorDNA

EP 3 728 581 B1

FIG. 15C

# FIG. 16

## FIG. 16A

Control Transformation 1
+ Cas9
- gRNA

## FIG. 16B

Control Transformation 2
- Cas9
+ gRNA

## FIG. 16C

CRISPR Transformation
+ Cas9
+ gRNA

FIG. 16D

|  | Number of Colonies | Blue Colonies | Percent Targeted |
|---|---|---|---|
| Control Transformation 1 | ~600 | ~600 | ~0% |
| Control Transformation 2 | ~600 | ~600 | ~0% |
| CRISPR Transformation | ~350 | 43 | ~88% |

EP 3 728 581 B1

# FIG. 17A-17B

## FIG. 17A

WT *E. coli*

Targeted *E. coli*

## FIG. 17B

FIG. 17C

| | # of Colonies Screened | # of Mutant Colonies | Percent Targeted |
|---|---|---|---|
| Control Transformation 2 | 20 | 0 | 0% |
| CRISPR Transformation | 19 | 18 | 95% |

FIG. 17D

*LacZ* locus

WT *E. coli*

Targeted *E. coli*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **SAMBROOK et al.** MOLECULAR CLONING: A LABORATORY MANUAL. Cold Spring Harbor Laboratory Press, 1989
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *, Mol. Cell. Probes,* 1994, vol. 8, 91-98
- **SMITH ; WATERMAN.** *Adv. Appl Math,* 1981, vol. 2, 482
- **NEEDLEMAN ; WUNSCH.** *J. Mol. Biol.,* 1970, vol. 48, 443
- **PEARSON ; LIPMAN.** *Proc. Nat'l. Acad. Sci. USA,* 1988, vol. 85, 2444
- **ALTSCHUL et al.** *Nuc. Acids Res.,* 1977, vol. 25, 3389-402
- **ALTSCHUL et al.** *J. Mol. Biol.,* 1990, vol. 215, 403-10
- **HENIKOFF ; HENIKOFF.** *Proc. Natl. Acad. Sci. USA,* 1989, vol. 89, 10915
- **KARLIN ; ALTSCHUL.** *Proc. Nat'l. Acad. Sci. USA,* 1993, vol. 90, 5873-5787
- **KEPPLER-ROSS et al.** *Genetics,* 2008, vol. 179, 705-710
- **JINEK et al.** *Science,* 2012, vol. 337, 816-821
- **JINEK et al.** *eLife,* 2013, vol. 2, e00471
- **MUROVEC et al.** *Plant Biotechnology Journal,* 2017, vol. 15, 917-926
- **SADHU et al.** *bioRxiv,* 2017, https://doi.org/10.1101/147637
- **CASINI et al.** *Nature Biotechnology,* 2018, vol. 36, 265-271
- **DICARLO et al.** *Nucleic Acids Res,* 2013, vol. 41, 4336-4343
- **SENTURK et al.** *Nat Commun,* 2017, vol. 8, 14370
- **YAN et al.** *ApplEnviron Microbiol,* 2017, vol. 83 (17
- **MUMBERG et al.** *Nucleic Acids Res.,* 1994, vol. 22, 5767-5768
- **CAO et al.** *Nucleic Acids Res,* 2016, vol. 44, e149
- **PYNE et al.** *Applied and Environmental Microbiology,* 2015, vol. 81, 5103-5114
- **DANG et al.** *Genome Biology,* 2015, vol. 16, 280
- **LEE et al.** *Exp Physiol,* 2017
- **ZUKER ; STIEGLER.** *Nucleic Acids Res.,* 1981, vol. 9, 133-148
- **A. R. GRUBER et al.** *Cell,* 2008, vol. 106 (1), 23-24
- **PA CARR ; GM CHURCH.** *Nature Biotechnology,* 2009, vol. 27 (12), 1151-62
- **MALYARCHUK et al.** *DNA Repair,* 2007, vol. 6, 1413-1424
- **CHAYOT et al.** *PNAS,* 2010, vol. 1017, 2141-2146